# EUROPEAN PATENT SPECIFICATION

(11) **EP 3 924 048 B1**
(45) Date of publication and mention of the grant of the patent: **10.06.2026**
(21) Application number: 20707786.8
(22) Date of filing: 14.02.2020
(51) Int. Cl.: A61K 48/00, A61P 9/04, A61P 11/00, C07K 14/47, C07K 14/82, C12N 9/12

(54) **MYC, CYCLIN T1 AND/OR CDK9 FOR USE IN THE TREATMENT OF DEGENERATIVE HEART AND CNS DISORDERS**
MYC UND CYCLIN T1 ZUR VERWENDUNG BEI DER BEHANDLUNG VON DEGENERATIVEN HERZERKRANKUNGEN
MYC ET CYCLINE T1 POUR L'UTILISATION DANS LE TRAITEMENT DE TROUBLES DÉGÉNÉRATIFS CARDIAQUES

(30) Priority: 14.02.2019 GB 201902068
(43) Date of publication of application: 22.12.2021
(73) Proprietor: Cambridge Enterprise Limited, Cambridge CB2 1TN (GB)
(72) Inventor: WILSON, Catherine Helen, Cambridge, Cambridgeshire CB2 1PD (GB); BYWATER, Megan, Herston, Queensland 4006 (AU)
(74) Representative: Marks & Clerk LLP
(86) International application number: PCT/GB2020/050350
(87) International publication number: WO 2020/165603

(56) References cited:
- WO-A1-2004/041838
- WO-A1-2004/041838
- JADLOWSKY JULIE K ET AL: "Dominant negative mutant Cyclin T1 proteins inhibit HIV transcription by specifically degrading Tat", RETROVIROLOGY, BIOMED CENTRAL LTD., LONDON, GB, vol. 5, no. 1, 11 July 2008 (2008-07-11), pages 63, XP021038066, ISSN: 1742-4690
- CALDWELL R L ET AL: "HIV-1 Tat interaction with cyclin T1 represses mannose receptor and the bone morphogenetic protein receptor-2 transcription", ARCHIVES OF BIOCHEMISTRY AND BIOPHYSICS, ACADEMIC PRESS, US, vol. 449, no. 1-2, 15 May 2006 (2006-05-15), pages 27 - 33, XP024943174, ISSN: 0003-9861, [retrieved on 20060515], DOI: 10.1016/J.ABB.2006.02.020
- SHAMANNA R A ET AL: "Induction of p53, p21 and apoptosis by silencing the NF90/NF45 complex in human papilloma virus-transformed cervical carcinoma cells", ONCOGENE, NATURE PUBLISHING GROUP UK, LONDON, vol. 32, no. 43, 3 December 2012 (2012-12-03), pages 5176 - 5185, XP037748140, ISSN: 0950-9232, [retrieved on 20121203], DOI: 10.1038/ONC.2012.533
- MOTOAKI SANO ET AL: "Activation of cardiac Cdk9 represses PGC-1 and confers a predisposition to heart failure", THE EMBO JOURNAL / EUROPEAN MOLECULAR BIOLOGY ORGANIZATION, vol. 23, no. 17, 1 September 2004 (2004-09-01), Oxford, pages 3559 - 3569, XP055684703, ISSN: 0261-4189, DOI: 10.1038/sj.emboj.7600351
- BYWATER MEGAN J. ET AL: "Reactivation of Myc transcription in the mouse heart unlocks its proliferative capacity", NATURE COMMUNICATIONS, vol. 11, no. 1, 14 April 2020 (2020-04-14), XP093098358, Retrieved from the Internet <URL:https://www.nature.com/articles/s41467-020-15552-x> DOI: 10.1038/s41467-020-15552-x
- SATOSHI KANAZAWA ET AL: "c-Myc recruits P-TEFb for transcription, cellular proliferation and apoptosis", ONCOGENE, vol. 22, no. 36, 28 August 2003 (2003-08-28), pages 5707 - 5711, XP055063461, ISSN: 0950-9232, DOI: 10.1038/sj.onc.1206800
- LEE HYOUNG-GON ET AL: "The Neuronal Expression of MYC Causes a Neurodegenerative Phenotype in a Novel Transgenic Mouse", THE AMERICAN JOURNAL OF PATHOLOGY, vol. 174, no. 3, 1 March 2009 (2009-03-01), US, pages 891 - 897, XP093098458, ISSN: 0002-9440, Retrieved from the Internet <URL:https://www.ncbi.nlm.nih.gov/pmc/articles/PMC2665749/pdf/JPATH174000891.pdf> DOI: 10.2353/ajpath.2009.080583
- HYUN-PIL LEE ET AL: "Early induction of c-Myc is associated with neuronal cell death", NEUROSCIENCE LETTERS, ELSEVIER, AMSTERDAM, NL, vol. 505, no. 2, 2 October 2011 (2011-10-02), pages 124 - 127, XP028108457, ISSN: 0304-3940, [retrieved on 20111008], DOI: 10.1016/J.NEULET.2011.10.004
- SANO MOTOAKI ET AL: "Activation and function of cyclin T-Cdk9 (positive transcription elongation factor-b) in cardiac muscle-cell hypertrophy", NATURE MEDICINE, vol. 8, no. 11, 30 November 2002 (2002-11-30), New York, pages 1310 - 1317, XP093098513, ISSN: 1078-8956, DOI: 10.1038/nm778
- R. E. KIERNAN ET AL: "Interaction between Cyclin T1 and SCFSKP2 Targets CDK9 for Ubiquitination and Degradation by the Proteasome", MOLECULAR AND CELLULAR BIOLOGY, vol. 21, no. 23, 1 December 2001 (2001-12-01), US, pages 7956 - 7970, XP055642791, ISSN: 0270-7306, DOI: 10.1128/MCB.21.23.7956-7970.2001
- HUANG CHUN-HAO ET AL: "CDK9-mediated transcription elongation is required for MYC addiction in hepatocellular carcinoma", GENES & DEVELOPMENT, vol. 28, no. 16, 15 August 2014 (2014-08-15), US, pages 1800, XP093098737, ISSN: 0890-9369, DOI: 10.1101/gad.244368.114
- JADLOWSKY JULIE K ET AL: "Dominant negative mutant Cyclin T1 proteins inhibit HIV transcription by specifically degrading Tat", RETROVIROLOGY, BIOMED CENTRAL LTD., LONDON, GB, vol. 5, no. 1, 11 July 2008 (2008-07-11), pages 63, XP021038066, ISSN: 1742-4690
- CALDWELL R L ET AL: "HIV-1 Tat interaction with cyclin T1 represses mannose receptor and the bone morphogenetic protein receptor-2 transcription", ARCHIVES OF BIOCHEMISTRY AND BIOPHYSICS, ACADEMIC PRESS, US, vol. 449, no. 1-2, 15 May 2006 (2006-05-15), pages 27 - 33, XP024943174, ISSN: 0003-9861, [retrieved on 20060515], DOI: 10.1016/J.ABB.2006.02.020
- SHAMANNA R A ET AL: "Induction of p53, p21 and apoptosis by silencing the NF90/NF45 complex in human papilloma virus-transformed cervical carcinoma cells", ONCOGENE, NATURE PUBLISHING GROUP UK, LONDON, vol. 32, no. 43, 3 December 2012 (2012-12-03), pages 5176 - 5185, XP037748140, ISSN: 0950-9232, [retrieved on 20121203], DOI: 10.1038/ONC.2012.533
- MOTOAKI SANO ET AL: "Activation of cardiac Cdk9 represses PGC-1 and confers a predisposition to heart failure", THE EMBO JOURNAL / EUROPEAN MOLECULAR BIOLOGY ORGANIZATION, vol. 23, no. 17, 1 September 2004 (2004-09-01), Oxford, pages 3559 - 3569, XP055684703, ISSN: 0261-4189, DOI: 10.1038/sj.emboj.7600351
- BYWATER MEGAN J. ET AL: "Reactivation of Myc transcription in the mouse heart unlocks its proliferative capacity", NATURE COMMUNICATIONS, vol. 11, no. 1, 14 April 2020 (2020-04-14), XP093098358, Retrieved from the Internet <URL:https://www.nature.com/articles/s41467-020-15552-x> DOI: 10.1038/s41467-020-15552-x
- SATOSHI KANAZAWA ET AL: "c-Myc recruits P-TEFb for transcription, cellular proliferation and apoptosis", ONCOGENE, vol. 22, no. 36, 28 August 2003 (2003-08-28), pages 5707 - 5711, XP055063461, ISSN: 0950-9232, DOI: 10.1038/sj.onc.1206800
- LEE HYOUNG-GON ET AL: "The Neuronal Expression of MYC Causes a Neurodegenerative Phenotype in a Novel Transgenic Mouse", THE AMERICAN JOURNAL OF PATHOLOGY, vol. 174, no. 3, 1 March 2009 (2009-03-01), US, pages 891 - 897, XP093098458, ISSN: 0002-9440, Retrieved from the Internet <URL:https://www.ncbi.nlm.nih.gov/pmc/articles/PMC2665749/pdf/JPATH174000891.pdf> DOI: 10.2353/ajpath.2009.080583
- HYUN-PIL LEE ET AL: "Early induction of c-Myc is associated with neuronal cell death", NEUROSCIENCE LETTERS, ELSEVIER, AMSTERDAM, NL, vol. 505, no. 2, 2 October 2011 (2011-10-02), pages 124 - 127, XP028108457, ISSN: 0304-3940, [retrieved on 20111008], DOI: 10.1016/J.NEULET.2011.10.004
- SANO MOTOAKI ET AL: "Activation and function of cyclin T-Cdk9 (positive transcription elongation factor-b) in cardiac muscle-cell hypertrophy", NATURE MEDICINE, vol. 8, no. 11, 30 November 2002 (2002-11-30), New York, pages 1310 - 1317, XP093098513, ISSN: 1078-8956, DOI: 10.1038/nm778
- R. E. KIERNAN ET AL: "Interaction between Cyclin T1 and SCFSKP2 Targets CDK9 for Ubiquitination and Degradation by the Proteasome", MOLECULAR AND CELLULAR BIOLOGY, vol. 21, no. 23, 1 December 2001 (2001-12-01), US, pages 7956 - 7970, XP055642791, ISSN: 0270-7306, DOI: 10.1128/MCB.21.23.7956-7970.2001
- HUANG CHUN-HAO ET AL: "CDK9-mediated transcription elongation is required for MYC addiction in hepatocellular carcinoma", GENES & DEVELOPMENT, vol. 28, no. 16, 15 August 2014 (2014-08-15), US, pages 1800, XP093098737, ISSN: 0890-9369, DOI: 10.1101/gad.244368.114
- M BELLO ROUFAI ET AL: "Heart-specific inhibition of protooncogene c-myc attenuates cold-induced cardiac hypertrophy", GENE THERAPY, vol. 14, no. 19, 1 October 2007 (2007-10-01), GB, pages 1406 - 1416, XP055684708, ISSN: 0969-7128, DOI: 10.1038/sj.gt.3302995
- MURPHY DANIEL J. ET AL: "Distinct Thresholds Govern Myc's Biological Output In Vivo", CANCER CELL, vol. 14, no. 6, 1 December 2008 (2008-12-01), US, pages 447 - 457, XP093263140, ISSN: 1535-6108, Retrieved from the Internet <URL:https://pdf.sciencedirectassets.com/272618/1-s2.0-S1535610808X00121/1-s2.0-S153561080800367X/main.pdf?hash=94d06116aa32e1dc514737876070ae37a4abaa01e51620b98a4f738069296151&host=68042c943591013ac2b2430a89b270f6af2c76d8dfd086a07176afe7c76c2c61&pii=S153561080800367X&tid=spdf-8e6097cd-9c1f-4168-b325-eed> DOI: 10.1016/j.ccr.2008.10.018
- HIRST SHANNON K ET AL: "Dierential activity of conditional MYC and its variant MYC-S in human mortal broblasts", ONCOGENE, vol. 19, 1 September 2000 (2000-09-01), pages 5189 - 5197, XP093263145
- N IKEGAKI ET AL: "The human L-myc gene is expressed as two forms of protein in small cell lung carcinoma cell lines: detection by monoclonal antibodies specific to two myc homology box sequences", THE EMBO JOURNAL, 1 June 1989 (1989-06-01), ENGLAND, pages 1793 - 1799, XP055447530, Retrieved from the Internet <URL:https://www.ncbi.nlm.nih.gov/pmc/articles/PMC401025/pdf/emboj00130-0159.pdf> DOI: 10.1002/j.1460-2075.1989.tb03573.x
- KACZÓWKA PRZEMYSLAW ET AL: "The role of N-Myc gene amplification in neuroblastoma childhood tumour - single-centre experience", WSPÓ&LSTROK;CZESNA ONKOLOGIA, vol. 22, no. 4, 1 January 2018 (2018-01-01), pages 223 - 228, XP093263285, ISSN: 1428-2526, Retrieved from the Internet <URL:https://pmc.ncbi.nlm.nih.gov/articles/PMC6377415/pdf/WO-22-81402.pdf> DOI: 10.5114/wo.2018.81402
- BIRRER MICHAEL J. ET AL: "A Transfected L-myc Gene Can Substitute for c-myc in Blocking Murine Erythroleukemia Differentiation", MOLECULAR AND CELLULAR BIOLOGY, vol. 9, no. 6, 1 June 1989 (1989-06-01), USA, pages 2734 - 2737, XP093326986, ISSN: 1098-5549, DOI: 10.1128/mcb.9.6.2734-2737.1989
- BOUCHARD CAROLINE ET AL: "Regulation of cyclin D2 gene expression by the Myc/Max/Mad network: Myc-dependent TRRAP recruitment and histone acetylation at the cyclin D2 promoter", GENES & DEVELOPMENT, vol. 15, no. 16, 15 August 2001 (2001-08-15), US, pages 2042 - 2047, XP093326994, ISSN: 0890-9369, DOI: 10.1101/gad.907901
- MCMAHON STEVEN B ET AL: "The Novel ATM-Related Protein TRRAP Is an Essential Cofactor for the c-Myc and E2F Oncoproteins", CELL, vol. 94, 7 August 1998 (1998-08-07), pages 363 - 374, XP093326999
- MALYNN BARBARA A. ET AL: "N- myc can functionally replace c- myc in murine development, cellular growth, and differentiation", GENES & DEVELOPMENT, vol. 14, no. 11, 1 June 2000 (2000-06-01), US, pages 1390 - 1399, XP093326997, ISSN: 0890-9369, DOI: 10.1101/gad.14.11.1390
- CHEN HUI ET AL: "Targeting oncogenic Myc as a strategy for cancer treatment", SIGNAL TRANSDUCTION AND TARGETED THERAPY, vol. 3, no. 1, 23 February 2018 (2018-02-23), XP055963890, DOI: 10.1038/s41392-018-0008-7
- PETERLIN B. MATIJA ET AL: "Controlling the Elongation Phase of Transcription with P-TEFb", MOLECULAR CELL, vol. 23, no. 3, 1 August 2006 (2006-08-01), AMSTERDAM, NL, pages 297 - 305, XP093327002, ISSN: 1097-2765, DOI: 10.1016/j.molcel.2006.06.014
- HIROFUJI AINA ET AL: "Mycn Reactivates the Cell Cycle in Adult Cardiomyocytes and Promotes Cardioprotection in Myocardial Infarction", BIORXIV, 26 July 2025 (2025-07-26), pages 1 - 31, XP093327004, Retrieved from the Internet <URL:https://www.biorxiv.org/content/biorxiv/early/2025/07/26/2025.07.23.666231.full.pdf> DOI: 10.1101/2025.07.23.666231
- PREETI AHUJA ET AL: "Myc controls transcriptional regulation of cardiac metabolism and mitochondrial biogenesis in response to pathological stress in mice", JOURNAL OF CLINICAL INVESTIGATION, vol. 120, no. 5, 3 May 2010 (2010-05-03), GB, pages FP,1494 - 1505, XP055684035, ISSN: 0021-9738, DOI: 10.1172/JCI38331
- AHUJA PREETI ET AL: "Supp. Material: Myc controls transcriptional regulation of cardiac metabolism and mitochondrial biogenesis in response to pathological stress in mice", JOURNAL OF CLINICAL INVESTIGATION, vol. 120, no. 5, 3 May 2010 (2010-05-03), pages S1 - S7, XP055684039, DOI: 10.1172/JCI38331
- KYRAN O MITCHELL ET AL: "Overexpression of c-Myc Inhibits p21 WAF1/CIP1 Expression and Induces S-Phase Entry in 12-O-Tetradecanoylphorbol-13- acetate (TPA)-sensitive Human Cancer Cells 1", CELL GROWTH & DIFFERENTIATION, vol. 10, 1 April 1999 (1999-04-01), pages 223 - 230, XP055684581
- HYEWON YOUN ET AL: "Modified mRNA as an alternative to plasmid DNA (pDNA) for transcript replacement and vaccination therapy", EXPERT OPINION ON BIOLOGICAL THERAPY, vol. 15, no. 9, 2 September 2015 (2015-09-02), ASHLEY, LONDON; GB, pages 1337 - 1348, XP055406240, ISSN: 1471-2598, DOI: 10.1517/14712598.2015.1057563
- ARA HACOBIAN ET AL: "Pushing the Right Buttons: Improving Efficacy of Therapeutic DNA Vectors", TISSUE ENGINEERING PART B-REVIEWS, vol. 24, no. 3, 1 June 2018 (2018-06-01), US, pages 226 - 239, XP055684622, ISSN: 1937-3368, DOI: 10.1089/ten.teb.2017.0353

## Description

### FIELD OF THE INVENTION

The invention relates to a nucleic acid molecule comprising a nucleic acid sequence encoding a myc transcription factor selected from c-myc, I-myc or n-myc and cyclin T1 for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

### BACKGROUND OF THE INVENTION

When an organ is damaged in order to maintain the integrity of the physiological and morphological state, the tissue must repair. The regenerative potential of an organ relates to the ability of the cells within that tissue to restore original tissue architecture post damage. Some organs have low regenerative potential and when damaged replace tissue with connective tissue that causes subsequent scarring. Certain organs, tissues and cells of the body are more capable of cellular proliferation (and hence regeneration) than others (Krafts, 2010). Broadly, organs can be separated into three categories based on their regenerative potential (Kotton and Morrisey, 2014). First, organs such as the intestine, skin and hematopoietic system that show high levels of cellular turnover and require a dedicated undifferentiated stem cell population to replace dead or sloughed-off cells. These tissues have enormous proliferative and self-renewing capacity. Second, quiescent tissues such as the liver, lung and pancreas respond with a proliferative burst after injury to replace lost cells (Kotton and Morrisey, 2014). Third, non-dividing tissues such as the central nervous system (CNS) and heart regenerate poorly or not at all after injury. These tissues are for the most part, incapable of self-repair and regeneration. Much research is devoted to developing technologies and techniques to aid or promote healing when organs are damaged.

There are ~23 million heart failure patients worldwide, a condition for which there is currently no cure and current treatments only slow disease progression. The initiating cause of most instances of heart failure is a loss of cardiomyocytes, which are never replenished due to the low intrinsic regenerative capacity of the adult heart (Porrello et al., 2011; Porrello and Olson, 2014). Likewise, nervous system injuries affect over 90,000 people every year (Stabenfeldt et al., 2006). Traumatic injury to the CNS causes cell death and, for the most part, the CNS is incapable of regeneration of these lost cells. Control of neuron regeneration is both a major unmet medical need and an unsolved problem in neurobiology (Mahar and Cavalli, 2018). The failure of damaged adult CNS axons to regrow results in permanent disabilities for individuals with spinal cord injury or stroke (Mahar and Cavalli, 2018) which has enormous socio-economic impact. Furthermore, many diseases for example, multiple sclerosis, could be alleviated by regeneration of the CNS.

Regeneration of cells is essentially mediated via genetic regulation and the identification of factors that are capable of re-activating proliferation in adult tissue has become a promising avenue of research. While a variety of different factors can increase proliferation, the effects on regeneration have been modest (Leach et al., 2017; Mohamed et al., 2018; Nakada et al., 2017). Therefore, further understanding the cell intrinsic mechanisms of regeneration of these non-dividing cells is imperative if innate regenerative capacity is to be harnessed.

Myc is a basic helix-loop-helix-leucine zipper (bHLH-LZ) transcription factor that binds preferentially to specific sequences in the genome, termed E-boxes (Blackwood and Eisenman, 1991), via association with its bHLH-LZ heterodimerisation partner Max (Amati et al., 1993, 1992; Blackwood et al., 1992). Myc functions principally as a transcriptional activator by potentiating transcription initiation via association with various cofactors such as TRRAP and associated histone acetyl-transferases (Bouchard et al., 2001; McMahon et al., 1998) and facilitating productive transcriptional elongation by promoting RNA PolII loading and via its association with positive transcription elongation factor (P-TEFb) (De Pretis et al., 2017; Eberhardy and Farnham, 2002; Kanazawa et al., 2003; Rahl et al., 2010). P-TEFb is comprised of Cdk9 and Cyclin T1 which are stringently regulated by various transcriptional and post transcriptional mechanisms (Jonkers and Lis, 2015; Peterlin and Price, 2006; Zhou et al., 2012; Zhou and Yik, 2006), and dynamically controlled by an association with inactivation complex comprised of 7SK snRNA, Larp7, MEPCE and HEXIM (Barboric et al., 2005; He et al., 2008; Yik et al., 2003). P-TEFb phosphorylates Serine 2 of the C-terminal Domain (CTD) of paused RNA Polll leading to productive elongation (Jonkers and Lis, 2015; Peterlin and Price, 2006; Zhou et al., 2012; Zhou and Yik, 2006).

Myc is a highly pleiotropic transcription factor which coordinates multiple transcriptional programmes involved in cell replication and differentiation, metabolism and apoptosis (Amati et al., 1993; Dang, 2013; Eilers et al., 1991; Evan et al., 1992; Roussel et al., 1991). With the development of animal models allowing for switchable ectopic Myc expression *in vivo,* it has become evident that Myc also governs diverse cell extrinsic processes required for tissue regeneration - such as angiogenesis, modulation of the local inflammatory and immune responses, invasion and migration - but in a manner that is tightly tailored to the tissue in which Myc is activated (Kortlever et al., 2017; Shchors et al., 2006; Sodir et al., 2011). Since deregulated and elevated Myc expression is a pervasive and causal attribute of most, perhaps all, tumours, understanding how tissue-specific responses to Myc are determined at a molecular level is imperative.

Comprehensive analysis of Myc transcriptional output in individual cell types indicates that Myc regulates the expression of thousands of genes, perhaps as much as a third of the transcriptome. Such studies show great diversity across experimental platforms and hint that components of the transcriptional repertoire of Myc are highly context specific. In particular, genome-wide analysis of Myc occupancy indicates the presence of Myc on virtually all promoters with open chromatin, suggesting that tissue-specific variations in Myc activity result from specific, pre-existing resident cellular programmes. However, this large and diverse number of potential Myc target genes, and the lack of comparative analysis of transcriptional responses to Myc in different tissues, have together confounded reliable identification of common and tissue-specific Myc-dependent transcriptional programmes. Moreover, recruitment of Myc to a given gene does not always correlate with its level of transcription and binding efficiency and transcriptional outputs are influenced significantly by different levels of Myc expression. Nonetheless, it remains clear that Myc is a selective transcription factor that regulates a defined set of core genes across multiple tissues, and others that are tissue-specific.

Ahuja et al. (2010) describes that Myc directly regulates glucose metabolism and mitochondrial biogenesis in cardiac myocytes and is an important regulator of energy metabolism in the heart in response to pathologic stress.

In Sano et al. (2004) the authors, report that αMHC-cyclin **T1** mice appear normal at baseline yet suffer fulminant apoptotic cardiomyopathy when challenged by mechanical stress or signaling by the G-protein Gq. At pathophysiological levels, Cdk9 activity suppresses many genes for mitochondrial proteins including master regulators of mitochondrial function (peroxisome proliferator-activated receptor gamma coactivator 1 (PGC-1), nuclear respiratory factor-1). In culture, cyclin T1/Cdk9 suppresses PGC-1, decreases mitochondrial membrane potential, and sensitizes cardiomyocytes to apoptosis, effects rescued by exogenous PGC-1. Cyclin T1/Cdk9 inhibits PGC-1 promoter activity and preinitiation complex assembly. Thus, the authors concluded that chronic activation of Cdk9 causes not only cardiomyocyte enlargement but also defective mitochondrial function, via diminished PGC-1 transcription, and a resulting susceptibility to apoptotic cardiomyopathy.

Kiernan et al. (2001) reports that CDK9 is ubiquitinated and degraded by the proteasome whereas cyclin T1 is stable. SCF(SKP2) was recruited to CDK9/cyclin T1 via cyclin T1 in an interaction requiring its PEST domain. CDK9 accumulated in p45(SKP2-/-) cells, and its expression during the cell cycle was periodic. The transcriptional activity of CDK9/cyclin T1 on the class II major histocompatibility complex promoter could be regulated by CDK9 degradation in vivo. The authors propose a novel mechanism whereby recruitment of SCF(SKP2) is mediated by cyclin T1 while ubiquitination occurs exclusively on CDK9.

Huang et al. (2014), reported that pharmacological or shRNA-mediated CDK9 inhibition led to robust anti-tumour effects that correlated with MYC expression levels and depended on the role that both CDK9 and MYC exert in transcription elongation. The authors identified CDK9 inhibition as a therapeutic strategy for MYC-overexpressing liver tumours and highlight the relevance of transcription elongation in the addiction of cancer cells to MYC.

As discussed above, there is a need to understand the intrinsic cellular mechanisms of regeneration or lack of in non-dividing cells, and moreover, a need to be able to influence these mechanisms such that the regenerative potential of these cells can be reestablished. The present invention addresses this need.

### SUMMARY OF THE INVENTION

Using a reversibly switchable mouse model in which supraphysiological levels of Myc are expressed comparably across different tissues we have determined that there are three general classes of response to activation of oncogenic levels of Myc: tissues that proliferated in response to the activation of ectopic Myc; tissues that did not; and tissues that exhibited innately high endogenous Myc and proliferative indices. The correlation between proliferative response to ectopic Myc in these tissues and the innate regenerative potential of each tissue is striking and suggests an underlying mechanistic connection. We have further determined that tissue regenerative capacity is tightly linked to the capacity of that tissue to respond to Myc and that tissue Myc responsiveness is governed principally by availability of key components of the core transcriptional machinery, namely p-TEFb, which Myc co-opts to drive its regenerative biological output. As such, we have determined that increasing the expression and/or activity of at least one, preferably both of Myc and p-TEFb, can increase or rather re-establish regenerative proliferation in adult tissues without proliferative potential. Where we refer to p-TEFb herein is meant cyclin T1.

In one aspect of the invention there is provided a nucleic acid molecule comprising a nucleic acid sequence encoding a myc transcription factor selected from c-myc, I-myc and n-myc and a cyclin T1 protein for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

In one embodiment, the nucleic acid is a ribonucleotide, preferably mRNA. In a further preferred embodiment, the mRNA molecule comprises at least one modification selected from a cap modification, a tail modification, a nucleoside modification or an untranslated region (UTR) modification.

In one embodiment, the mRNA molecule encodes a myc transcription factor as defined in SEQ ID NO: 11 or a functional variant thereof, wherein the functional variant retains the transcription factor activity of the full non-variant sequence and has at least 75% overall sequence identity to SEQ ID NO: 11, and a cyclin T1 protein as defined in SEQ ID NO: 5 or a functional variant thereof wherein the functional variant retains the biological function of the full non-variant sequence and has at least 75% overall sequence identity to SEQ ID NO: 5.

In an alternative embodiment, the nucleic acid molecule is a nucleic acid construct or vector.

In one embodiment, the nucleic acid sequence encodes an inducible myc transcription factor as defined in SEQ ID NO: 1 or 10 or a functional variant thereof wherein the functional variant retains the transcription factor activity of the full non-variant sequence and has at least 75% overall sequence identity to SEQ ID NO: 1 or 10, operably linked to a first regulatory sequence; and a nucleic acid sequence encoding a cyclin T1 protein as defined in SEQ ID NO: 5 or a functional variant thereof wherein the functional variant retains the biological function of the full non-variant sequence and has at least 75% overall sequence identity to SEQ ID NO: 5, operably linked to the first regulatory sequence or a second regulatory sequence.

In one embodiment, the regulatory sequence is a promoter. In one example, the promoter is the CAG promoter.

In one embodiment, the vector is a viral vector. In one preferred embodiment the viral vector is an adeno-associated viral vector.

In another aspect of the invention there is provided a composition comprising a nucleic acid molecule as described above and a pharmaceutically acceptable carrier for use as a medicament.

In another aspect of the invention there is provided a composition comprising a first modified mRNA molecule and at least a second modified mRNA molecule, wherein the first modified mRNA molecule encodes a myc transcription factor selected from c-myc, I-myc or n-myc and the second modified mRNA molecule encodes a cyclin T1 protein, wherein the modification is selected from at least one of a cap modification, a tail modification, a nucleoside modification and a untranslated region (UTR) modification, and wherein the first modified mRNA molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

In another aspect of the invention there is provided a composition comprising a first vector and at least a second vector, wherein the first vector comprises a nucleic acid sequence encoding an inducible myc transcription factor selected from c-myc, n-myc or I-myc operably linked to a regulatory sequence and the second vector comprises a nucleic acid sequence encoding a cyclin T1 protein operably linked to a regulatory sequence for use as a medicament, and wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

In a further aspect of the invention there is provided a nucleic acid molecule as described herein or a composition as described herein for use in the treatment of a condition characterised by the loss of cells, wherein the condition is selected from myocardial infarction or reduced ejection fraction of the heart.

In another aspect of the invention there is provided a method of increasing at least one of cell proliferation, mitosis and cytokinesis in a cell *in vitro,* the method comprising introducing to the cell a nucleic acid molecule as described herein or a composition as described herein, wherein the cell is a heart cell.

In a further aspect of the invention there is provided a method of increasing organ size *in vitro,* the method comprising introducing to the organ a nucleic acid molecule as described herein or a composition as described herein, wherein the organ is a heart.

In another aspect of the invention, there is provided a host cell comprising the nucleic acid molecule as described herein or a composition as described herein, wherein the host cell is a heart cell.

In a final aspect of the invention there is provided a nanoparticle comprising a nucleic acid molecule as described herein.

### DESCRIPTION OF THE FIGURES

The invention is further described in the following non-limiting figures:

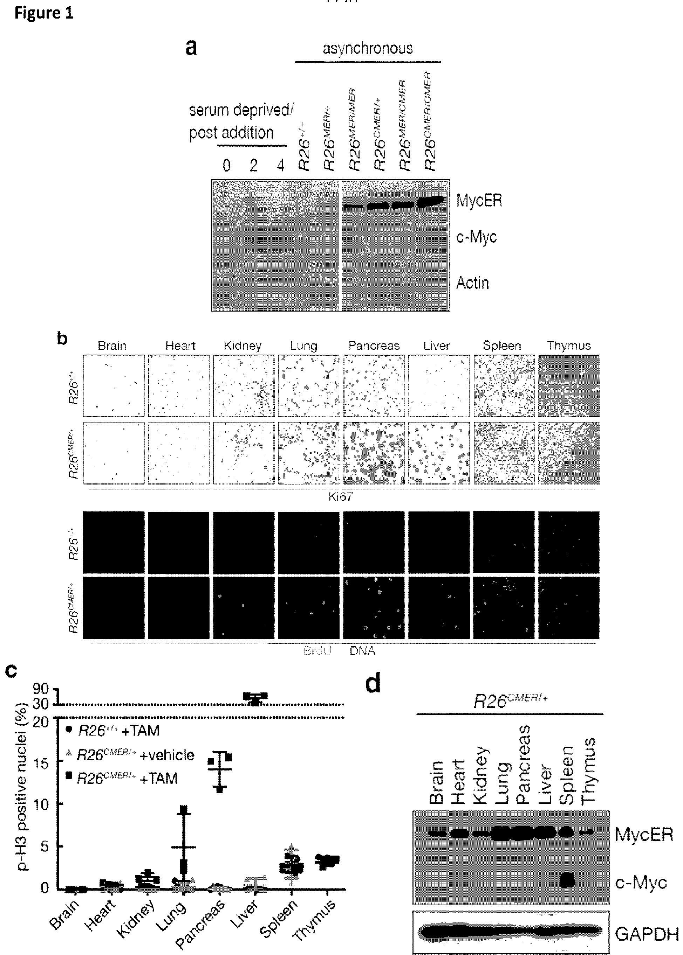

**Figure 1** shows the proliferative response to supraphysiological Myc is very variable across different tissues.
   (a) Immunoblot analysis of MycERT2 and endogenous c-Myc protein levels in wild-type (R26+/+) murine embryonic fibroblasts (MEFs) maintained in serum-deprived media, and at the indicated type points (in hours) post addition of serum, all compared with asynchronous R26+/+, R26MER/+, R26MER/MER, R26CMER/+, R26MER/CMER, R26CMER/CMER MEFs. Expression of Actin is included as a loading control.
   (b) Immunohistochemical and immunofluorescence staining of Ki67 and BrdU in the brain, heart, kidney, lung, pancreas, liver, spleen and thymus isolated from wild-type (R26+/+) and R26CMER/+ mice 24 hours post administration of tamoxifen, Representative images based on analysis of 5 independent mice.
   (c) Quantification of p-H3-positive nuclei percentage from brain, heart, kidney, lung, pancreas, liver (hepatocytes), spleen (red pulp) and thymus isolated from oil treated *R26*^{*CMER*/+} (n≥3) mice or wild-type (R26+/+, n≥3) and R26CMER/+ (n=3) mice 24 hours post administration of tamoxifen. Mean of 5 images per mouse; error bars, s.e.m.
   (d) Immunoblot analysis of MycERT2 and endogenous c-Myc expression in the brain, heart, kidney, lung, pancreas, liver, spleen and thymus isolated from R26CMER/+ mice. Sample loading was normalized for equal protein content, as determined by a bicinchoninic acid assay (BCA). Expression of GAPDH is included as a confirmation of efficient protein isolation. Representative results based on analysis of 4 independent mice.

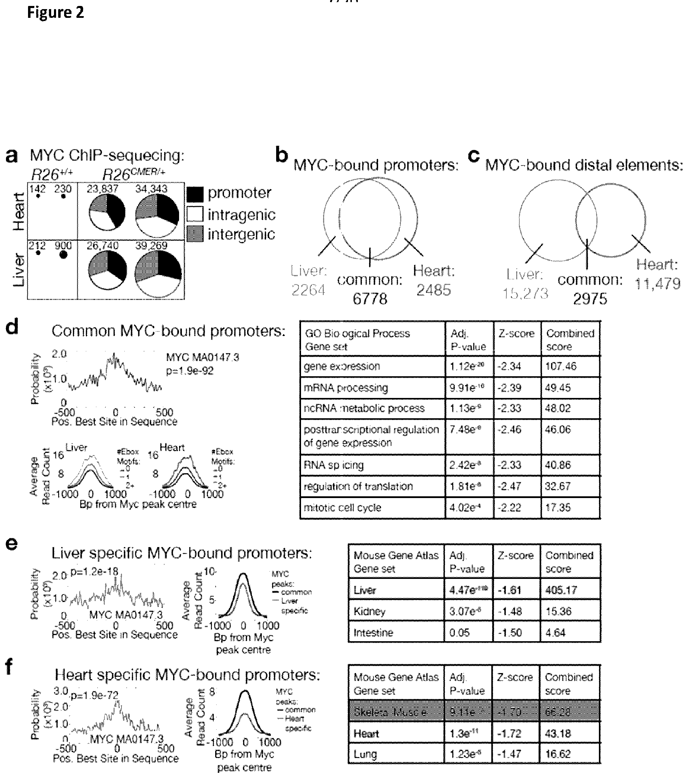

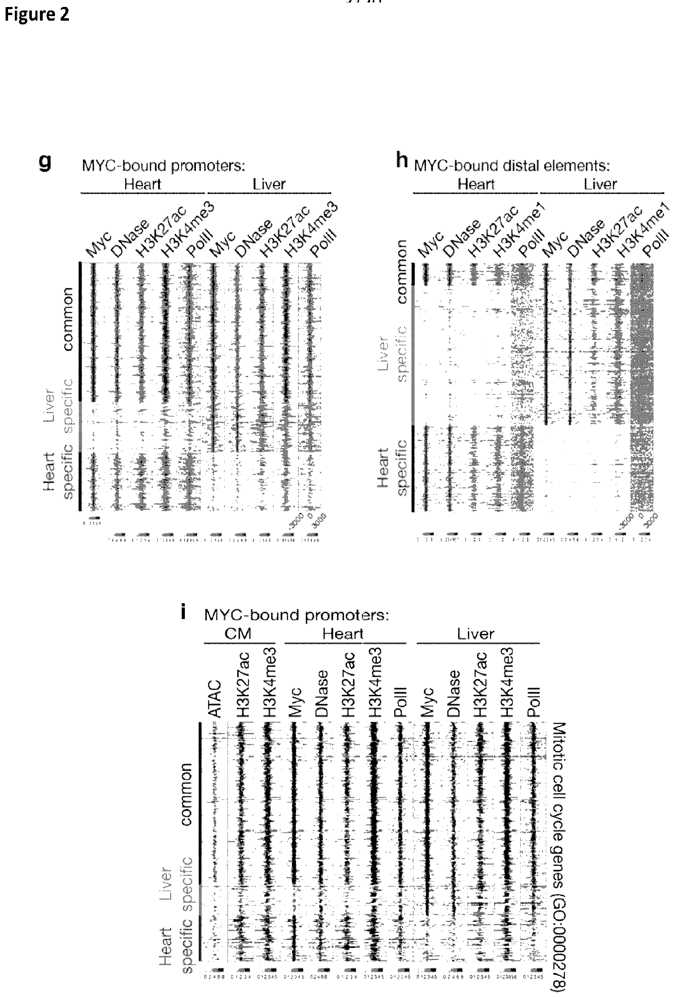

**Figure 2** shows Myc binding is dictated by chromatin access and differs between tissues. However, common Myc binding sites in the heart and liver are at genes involved in mitotic cell cycle and gene expression.
   (a) The number of peaks called for c-Myc ChIP sequencing performed on chromatin isolated from hearts and livers harvested from wild-type (R26+/+) and R26CMER/+ mice 4 hours post administration of 4-OHT, and their location in relation to coding regions of the genome (promoter, intragenic, intergenic). Replicates are derived from independent mice.
   (b) Venn diagram of the overlap of peaks called within promoter regions (- 2 kb to +1 kb from the nearest TSS), identified by Myc ChIP sequencing on chromatin isolated from heart and livers harvested from R26CMER/+ mice (n = 2) 4 hours post administration of 4-OHT.
   (c) Venn diagram of the overlap of peaks called within distal elements, identified by Myc ChIP sequencing performed on the heart and livers isolated from R26CMER/+ mice (n = 2) 4 hours post administration of 4-OHT.
   (d) Common Myc-bound promoters in both the liver and heart. Motif probability curves show the probability of an E-box consensus sequence occurring at a given position relative to the Myc ChIP peak at each common promoter site, as determined by CentriMo (top left). Average read count of Myc peaks at common promoters containing 0, 1 or >2 E-box motifs within 1,000 bp from the peak centre in Liver and Heart chromatin (bottom left). Selected significant GO Biological Process Gene sets that overlap with common Myc-bound promoter elements (right).
   (e) Liver-specific Myc-bound promoters bound by Myc only in the liver. Motif probability curves show the probability of an E-box consensus sequence occurring at a given position relative to the Myc ChIP peak at each liver-specific promoter site, as determined by CentriMo (left). Average read count of Myc peaks shown at common (black) and liver-specific (green) promoter sites (centre). Selected significant Mouse Gene Atlas Gene sets are shown that overlap with liver-specific Myc-bound promoter elements (right).
   (f) Heart specific Myc-bound promoters bound by Myc only in the heart. Motif probability curves show the probability of an E-box consensus sequence occurring at a given position 5 relative to the Myc ChIP peak at each heart-specific promoter site, as determined by CentriMo (left). Average read count of Myc peaks shown at common (black) and heartspecific (red) promoter sites (centre). Significant Mouse Gene Atlas Gene sets are shown that overlap with heart-specific Myc-bound promoter elements (right).
   (g) Heat map of peaks called by DNAse treated, acetylated H3K27 (H3K27ac), tri10 methylated H3K4 (H3K4me3) and RNA Polymerase II (Polll) ChIP sequencing at Mycbound promoter elements that are common between both the liver and heart (commonblack) or specific for an individual tissue (liver specific-green, heart specific-red). Myc ChIP sequencing was performed on the heart and livers isolated from R26CMER/+ mice 4 hours post administration of 4-OHT, overlap of n=2. Polll ChIP sequencing performed on the heart and livers isolated from wild-type (R26+/+ 15) mice, overlap of n=2. ChIP sequencing data for DNase treated, H3K27ac and H3K4me3 were taken from the ENCODE Project (accession numbers; GSM1014166, GSM1000093, GSM769017, GSM1014195, GSM1000140, GSM769014).
   (h) As in (g) but for distal elements.
   (i) Heat map of peaks called for ATAC seq, acetylated H3K27 (H3K27ac) and trimethylated H3K4 (H3K4me3) ChIP sequencing on purified cardiomyocytes (CM) compared to peaks called by DNAse treated, acetylated H3K27 (H3K27ac), trimethylated H3K4 (H3K4me3) and RNA Polymerase II (Polll) ChIP sequencing on whole heart (Heart) and liver (Liver) at Mitotic Cell Cycle genes (GO Biological process gene set 0000278) with Myc-bound promoter elements that are common between both the liver and heart (common-black) or specific for an individual tissue (liver specific-green, heart specific-red). Myc ChIP sequencing was performed on the heart and livers isolated from *R26*^{*CMER*/+} mice 4 hours post administration of 4-OHT, overlap of n=2. Polll ChIP sequencing performed on the heart and livers isolated from wild-type (*R26*^{+/+}) mice, overlap of n=2. ChIP sequencing data for DNase treated, H3K27ac and H3K4me3 were taken from the ENCODE Project (accession numbers; GSM1014166, GSM1000093, GSM769017, GSM1014195, GSM1000140, GSM769014), cardiomyocyte ATAC sequencing accession: GSE95763 (39), cardiomyocyte H3K27ac and H3K4me3 ChIP sequencing accession: SRP033385 (90).

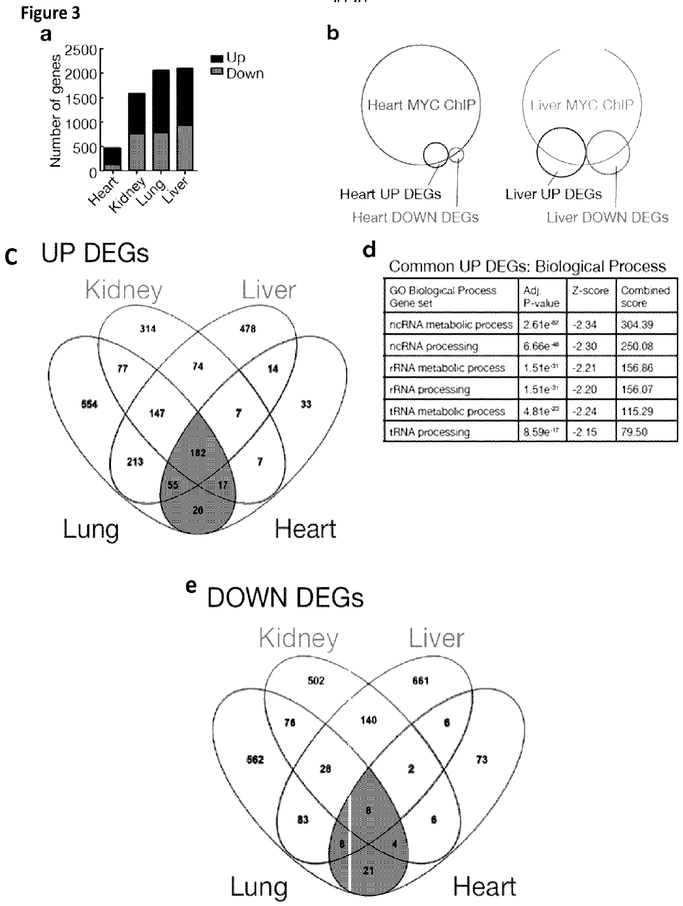

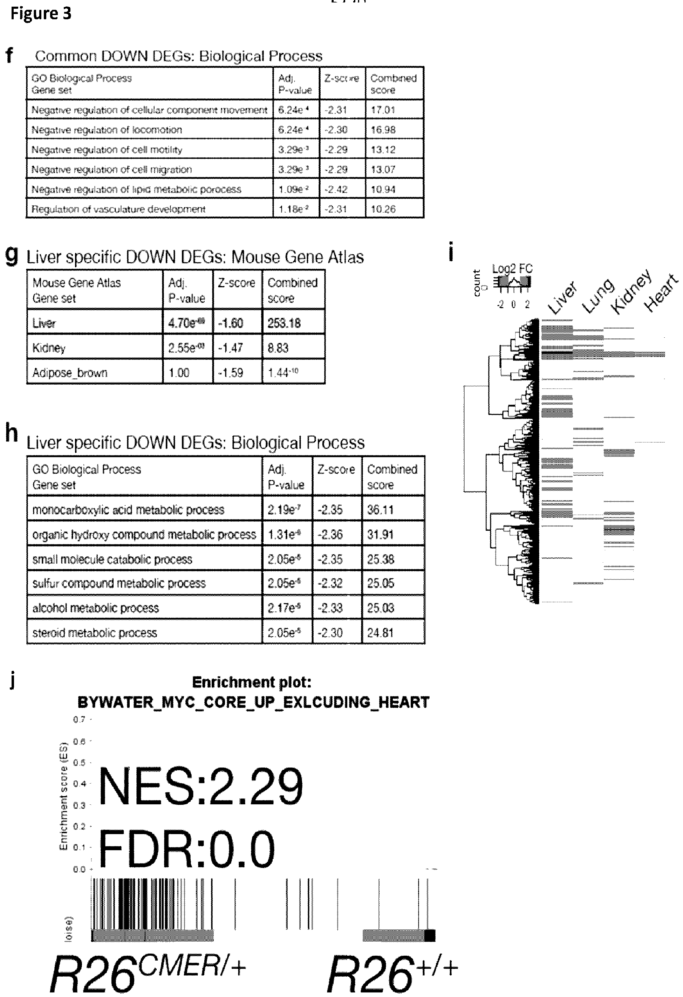

**Figure 3** shows Myc-directed transcriptional targets differ between tissues.
   (a) The number of differentially expressed genes in the heart, kidney, lung and liver of R26CMER/+ (n≥3) compared to wild-type (R26+/+, n≥3) mice 4 hours post administration of 4-OHT, as determined by RNA sequencing.
   (b) Venn diagram depicting overlap of peaks within promoter regions identified by c-Myc ChIP sequencing performed on chromatin from heart (Heart MYC ChIP, red) or liver (Liver MYC ChIP, green) isolated from R26CMER/+ mice together with genes differentially expressed in response to supraphysiological Myc in these same tissues (UP DEGs-black, DOWN DEGs-grey), as determined by RNA sequencing on tissues isolated from from R26CMER/+ versus wild-type (R26+/+) mice. All analysis performed at 4 hours post administration of 4-OHT. Areas are proportional to the numbers of promoters or differentially expressed genes within each cohort.
   (c) Venn diagram depicting numbers of genes showing increased expression in response to supraphysiological Myc (UP DEGs) in the kidney (yellow), liver (green), heart (red) and lung (blue) of R26CMER/+ (n≥3) mice relative to wild-type (R26+/+, n≥3), as determined by RNA sequencing (FDR <0.05 and abs(log2FC)>0.5) at 4 hours post administration of 4-OHT.
   (d) 6 most significant GO Biological Process Gene sets that overlap with listed genes, showing a differential increase in expression (UP DEGs) in two or more tissues (kidney, liver, heart, lung) of R26CMER/+ (n≥3) mice relative to wild-type (R26+/+, n≥3), as determined by RNA sequencing at 4 hours post administration of 4-OHT.
   (e) Venn diagram depicting numbers of genes showing decreased expression in response to supraphysiological Myc (DOWN DEGs) in the kidney (yellow), liver (green), heart (red) and lung (blue) of R26CMER/+ (n≥3) mice relative to wild-type (R26+/+, n≥3), as determined by RNA sequencing at 4 hours post administration of 4-OHT.
   (f) 6 most significant GO Biological Process Gene sets that overlap with listed genes that show a differential decrease in expression (DOWN DEGs) in two or more tissues (kidney, liver, heart, lung) of R26CMER/+ (n≥3) mice relative to wild-type (R26+/+, n≥3), as determined by RNA sequencing at 4 hours post administration of 4-OHT.
   (g) 3 most significant Mouse Gene Atlas Gene sets that overlap with listed genes that show a differential decrease in expression (DOWN DEGs) only in the liver (liver-specific) of R26CMER/+ (n≥3) mice relative to wild-type (R26+/+, n≥3), as determined by RNA sequencing at 4 hours post administration of 4-OHT.
   (h) 6 most significant GO Biological Process Gene sets that overlap with listed genes that show a differential decrease in expression (DOWN DEGs) only in the liver (liver-specific, absent in kidney, heart and lung) of R26CMER/+ (n≥3) mice relative to wild-type (R26+/+, n≥3), as determined by RNA sequencing at 4 hours post administration of 4-OHT.
   (i) Heatmap showing the union of DEGs called in each tissue; liver, lung, kidney and heart. Shown are mRNA expression fold changes (Log2) upon MycERT2 activation relative to wild-type as determined by RNA sequencing of R26CMER/+ (n≥3) mice relative to wild-type (R26+/+, n≥3) at 4 hours post administration of 4-OHT.
   (j) Hearts isolated from *R26*^{*CMER*/+} mice show enrichment of common Myc targets (exclusive to the liver, lung and kidney) in comparison to wild-type (*R26*^{+/+}), as determined by RNA sequencing at 4 hours post administration of 4-OHT.

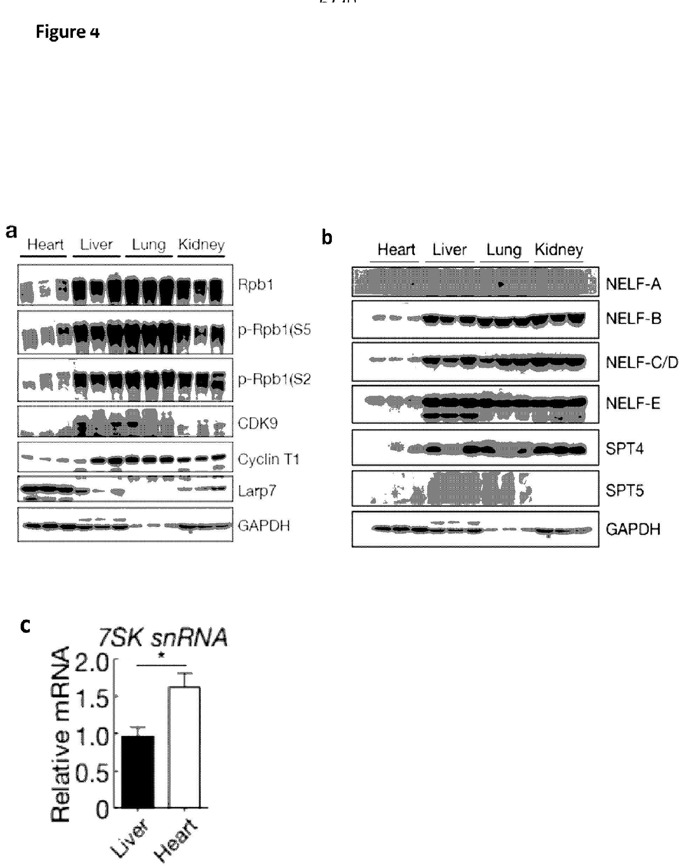

**Figure 4** shows the heart has low levels of endogenous P-TEFb
   (a) Immunoblot analysis of the C-terminal domain of RNA Polymerase II - total (Rpb1) and phosphorylated (p-Rpb1(S5) and p-Rpb1(S2)), CDK9, Cyclin T1 and Larp7 expression in the heart, liver, lung and kidney isolated from wild-type (R26+/+) mice. Sample loading was normalized for equal protein content. Expression of GAPDH is included as a confirmation of efficiency of protein isolation and comparable loading between individual tissue samples. Replicate samples are derived from independent mice. The composite figure is generated from the same samples loaded across multiple blots and a representative image for GAPDH is shown.
   (b) Immunoblot analysis of NELF-A, NELF-B, NELF-C/D, NELF-E, SPT4 and SPT5 expression in the heart, liver, lung and kidney isolated from wild-type (R26+/+ ) mice. Sample loading was normalized for equal protein content. Expression of GAPDH is included as a confirmation of efficiency of protein isolation and comparable loading between individual tissue samples. Replicate samples are derived from independent mice. The composite figure is generated from the same samples loaded across multiple blots and a representative image for GAPDH is shown.
   (c) Quantitative RT-PCR analysis of 7SK snRNA in liver and heart isolated from wild-type mice (n=5). Expression is normalized to Actin and Gapdh and relative to liver. Error bars show s.e.m. Unpaired t-test; liver vs heart P= 0.019*. Replicate samples are derived from independent mice.

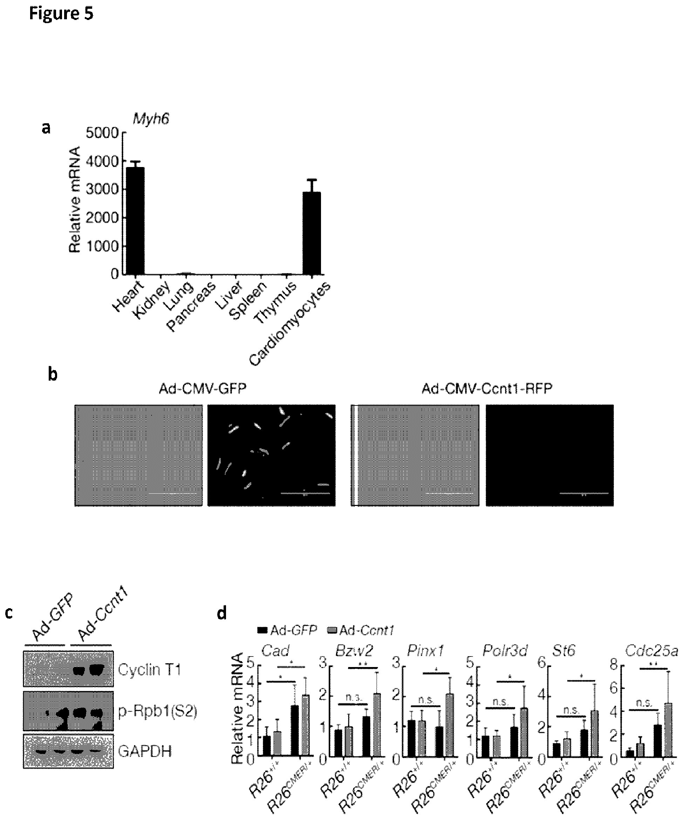

**Figure 5** shows that overexpression of Ccnt1 *in vitro* increases efficiency of Myc driven transcription in cardiomyocytes.
   (a) Quantitative RT-PCR analysis of Myh6 in the heart (n=4), kidney (n=4), lung (n=4), pancreas (n=4), liver (n=4), spleen (n=4) and thymus (n=3) of wild-type mice, and cardiomyoctyes (n=3) isolated from wild-type mice. Expression is normalized to Actin and Gapdh and relative to liver. Error bars show s.e.m. Replicate samples are derived from independent mice.
   (b) Phase contrast images of adult cardiomyocytes in culture 48 hours post infection and accompanying fluorescent images to determine transfection efficiency on the basis of fluorescent protein marker expression, either GFP (Ad-CMV-GFP) (left) or RFP (Ad-AMV-Ccnt1-RFP) (right). Scale bar = 400 µm.
   (c) Immunoblot analysis of Cyclin T1 and phosphorylated C-terminal domain of RNA Polymerase II (p-Rpb1(S2)) in R26CMER/+ primary cardiomyocytes infected with an adenovirus encoding either GFP (Ad-GFP) or Ccnt1 (Ad-Ccnt1). Replicate samples are derived from independent primary cardiomyocyte isolations.
   (d) Quantitative RT-PCR analysis of Cad, Bzw2, Pinx1, Polr3d, St6 and Cdc25a in wild type (R26+/+, n≥4) and R26CMER/+ (n≥4) primary cardiomyocytes infected with an adenovirus encoding either GFP (Ad-GFP) or Ccnt1 (Ad-Ccnt1), 4 hours post addition of 100nM 4-OHT. Expression is normalized to Gapdh and Actin and relative to an individual wild-type (R26+/+) Ad-GFP control. Error bars show s.d. Two Way ANOVA with Tukey's multiple comparisons test; Ad-GFP R26+/+ vs R26CMER/+: P=0.05* (Cad), Ad-Ccnt1 R26+/+ vs R26CMER/+: P=0.05* (Cad, Pinx1, Polr3d, St6) P=0.01** (Bzw2, Cdc25a). Replicate samples are derived from independent primary cardiomyocyte isolations.

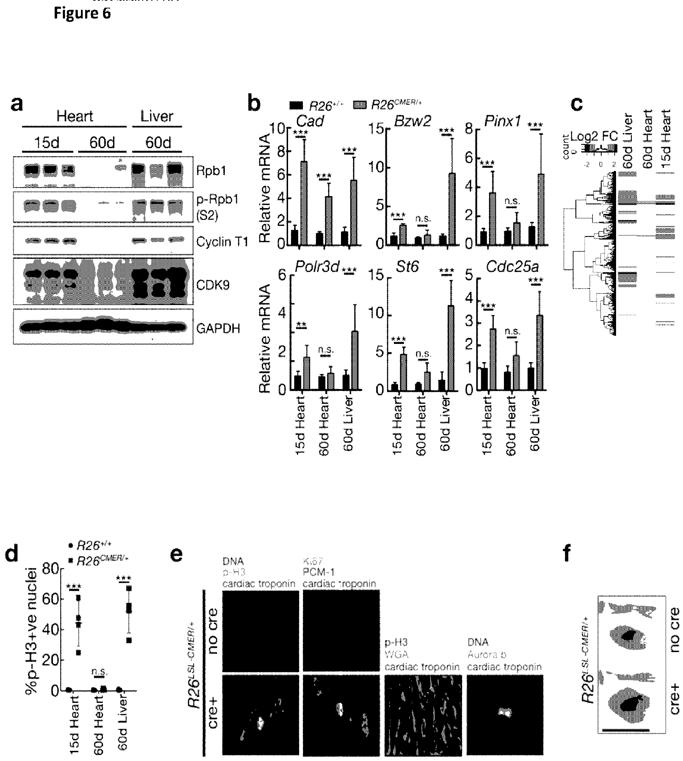

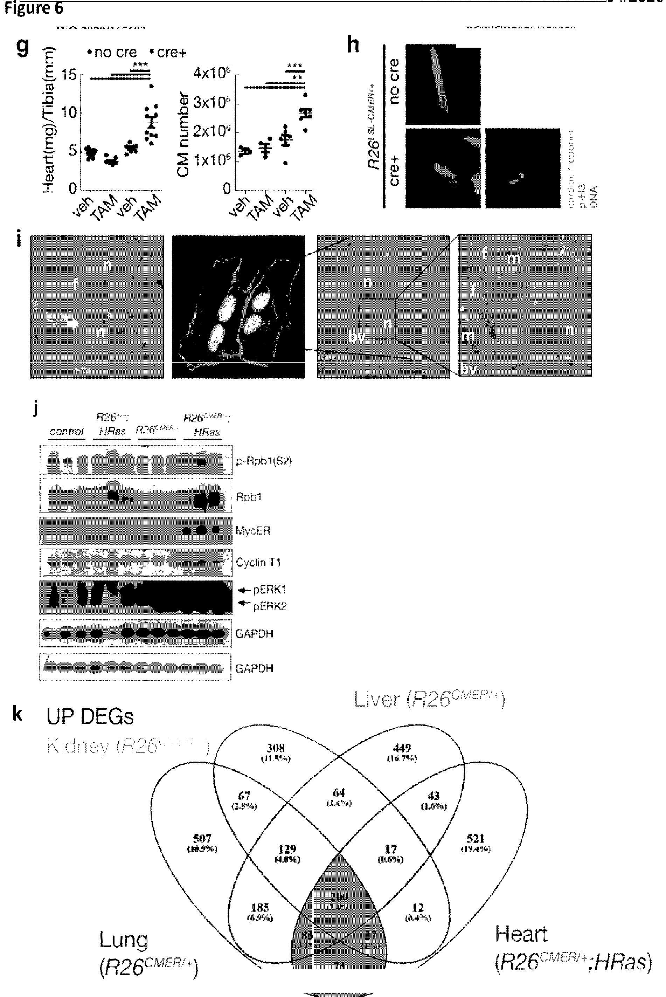

**Figure 6** shows that the juvenile heart has high levels of P-TEFb and is permissive for Myc-driven transcriptional activation
   (a) Immunoblot analysis of the C-terminal domain of RNA Polymerase II, total (Rpb1) and phosphorylated (p-Rpb1(S2)), Cyclin **T1** and CDK9 protein expression in wild-type heart and liver isolated from 15 day-old (15d) and 60 day-old (60d) mice. Expression of GAPDH is included as a loading control. Replicate samples are derived from independent mice.
   (b) Quantitative RT-PCR analysis of Cad, Bzw2, Pinx1, Polr3d, St6 and Cdc25a expression in wild-type (R26+/+, n≥6) and R26CMER/+ (n≥6) heart isolated from 15 day-old (15d) mice versus heart and liver isolated from 60 day-old (60d) mice 4 hours post administration of 4- OHT. Expression is normalized to Actin and Gapdh and relative to the respective wildtype (R26+/+). Error bars show s.d. Two Way ANOVA with Tukey's multiple comparisons test; 15 day R26+/+ 4-OHT vs R26CMER/+ 4-OHT: P=0.001*** (Cad, Bzw2, Pinx1, Polr3d, St6 and Cdc25a), 15 day R26+/+ 4-OHT vs R26CMER/+ 4-OHT: P=0.001** (Polr3d), adult heart R26+/+ 4-OHT vs R26CMER/+ 4-OHT: P=0.001*** (Cad), adult liver R26+/+ 4-OHT vs R26CMER/+ 4-OHT: P=0.001*** (Cad, Bzw2, Pinx1, Polr3d, St6 and Cdc25a). Replicate samples are derived from independent mice.
   (c) Heatmap showing the union of DEGs called in each tissue; adult liver, adult heart and P15 heart. mRNA expression fold changes (Log2) upon MycER activation are shown relative to wild type, as determined by RNA sequencing of R26CMER/+ (n≥3) tissues relative to wild-type (R26+/+, n≥3) at 4 hours post administration of 4-OHT.
   (d) Quantification of p-H3 positive nuclei percentage in heart (positive cardiomyocyte nuclei only) and liver (positive hepatocyte nuclei only) isolated from 15 day-old (15d) and 60 day-old (60d) wild-type (R26+/+, n≥7) and R26CMER/+ (n≥4) mice 24 hours post administration of tamoxifen. Means are taken from 5 images per mouse; Error bars show s.d. Two Way ANOVA with Tukey's multiple comparisons test; R26+/+ 4-OHT vs R26CMER/+ 4-OHT: P=0.001*** (15 day heart and adult liver).
   (e) Immunofluorescent staining of cardiac troponin, p-H3, Ki67, PCM1, Wheat Germ Agglutinin and Aurora B positive mid-body in the heart of 15 day-old control (R26^{*LSL*-*CMER*/+}; no cre) and *Myh6-Cre; R26*^{*LSL-CMER*/+} (*R26*^{*LSL-CMER*/+} *;* cre+) mice 48 hours post administration of tamoxifen. Representative images based on analysis of 5 independent mice.
   (f) Image of the whole heart and a tibia isolated from a 15 day-old control (*R26*^{*LSL- CMER*/+}; no cre) and *Myh6-Cre; R26*^{*LSL-CMER*/+} (*R26*^{*LSL-CMER*/+} *;* cre+) mice 48 hours post administration of tamoxifen. Scale bar represents 10mm. Representative images based on analysis on > 4 independent mice.
   (g) The weight (mg) of hearts (left) and quantification of the number of cardiomyocytes (right) isolated from 15 day-old control (*R26*^{*LSL-CMER*/+}; no cre, n=4) and *Myh6-Cre;R26*^{*LSL-CMER*/+} *(R26*^{*LSL-CMER*/+} ; cre+, n=7) mice 48 hours post administration of tamoxifen or oil (veh), expressed as fold change over the length (mm) of a tibia isolated from the same mouse. Error bars show s.e.m. Unpaired t-test; no cre vs cre+ P= 0.0003*** (weight), Mann Whitney test; no cre vs cre+ P= 0.0061** (cardiomyoctyte number). Replicate samples are derived from independent mice.
   (h) Immunofluorescent staining of cardiac troponin T (green), p-H3 (red) and DNA (blue) on cardiomyocytes isolated from fixed and dissociated hearts of 15 day-old control (*R26*^{*LSL-CMER*/+}; no cre) and *Myh6-Cre; R26*^{*LSL-CMER*/+} (*R26*^{*LSL-CMER*/+;}*cre*+) mice.
   (i) Three dimensional render (second from left) generated from 500 image z-stack (50 µm) of transmission electron micrographs from a *Myh6-Cre;R26*^{*LSL-CMER*/+} heart 48 hours post administration of tamoxifen. Green shows outline of cells, blue shows nuclei, pink line shows site of cross section through cells, outlined in green (second from right), and expanded (right). Left image shows mitotic nuclei, arrow. Structures are indicated; f-myofibrils, n- nucleus, m- mitochondria, bv- blood vessel.
   (j) Immunoblot analysis of the C-terminal domain of RNA Polymerase II, total (Rpb1) and phosphorylated (p-Rpb1(S2)), MycERT2, Cyclin T1 and phosphorylated ERK1 (T202/T204) and ERK2 (T185/T187) protein expression in hearts isolated from control (R26+/+; TetO-HRas, R26+/+; Myh6-tTA or R26+/+), TetO-HRas; Myh6-tTA; R26+/+ (R26+/+;HRas), R26CMER/+ and TetO-HRas; Myh6-tTA; R26 CMER/+ (R26 CMER/+;HRas) mice 4 weeks post withdrawal of doxycycline and 4 hours post administration of 4-OHT. Replicate samples are derived from independent mice.
   (k) Venn diagram of the number of genes showing an increase in expression in response to supraphysiological Myc (UP DEGs) in the kidney (yellow), liver (green) and lung (blue) of R26CMER/+ (R26 CMER/+, n≥3) in comparison to wild-type (R26+/+, n≥3) and heart (red) from TetO-HRas; Myh6-tTA; R26 CMER/+ (R26 CMER/+;HRas) mice (n=3) in comparison to R26+/+;TetO-HRas; Myh6-tTA mice (n=3) four weeks post withdrawal of doxycycline, as determined by RNA sequencing (FDR<0.05 and abs(log2FC)>0.5) at 4 hours post administration of 4-OHT.

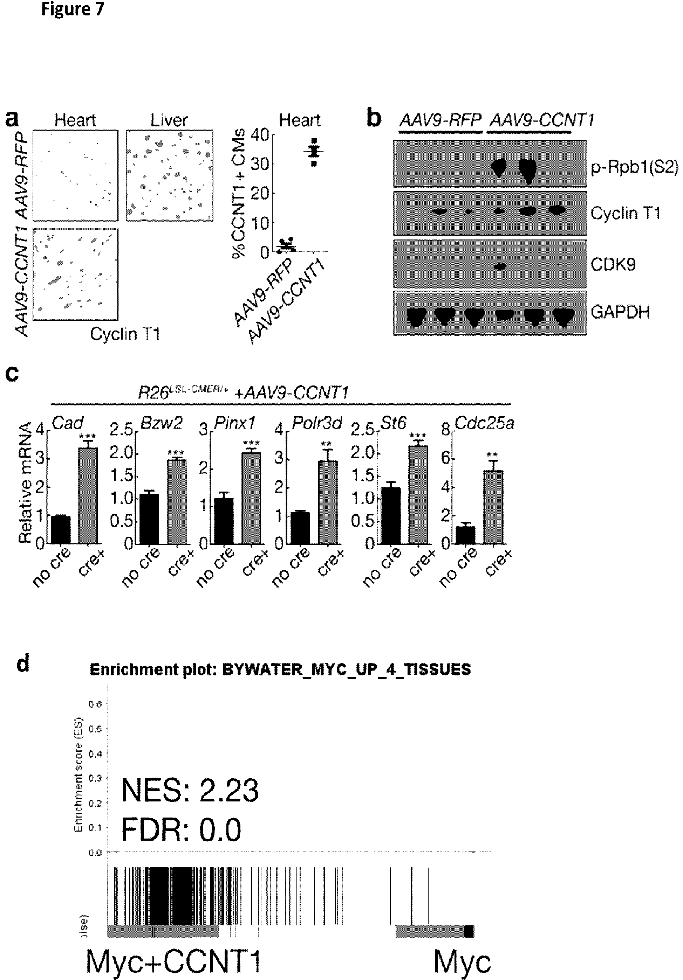

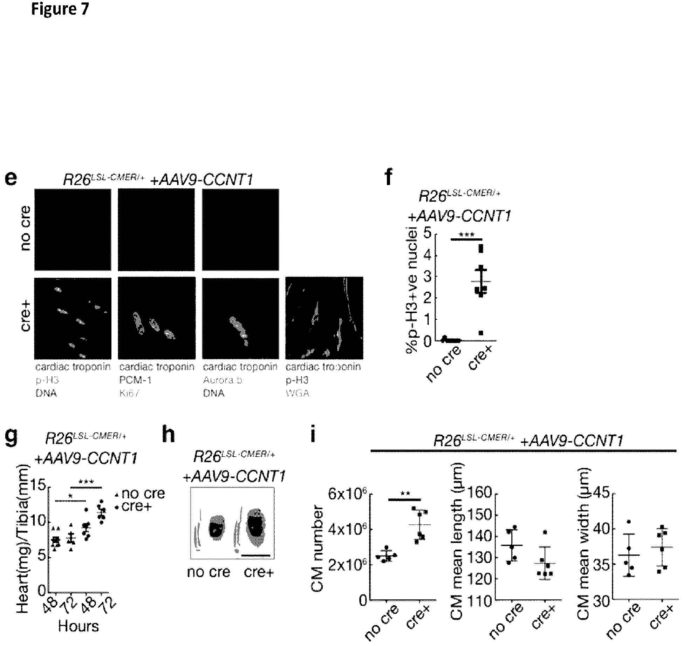

**Figure 7** shows that overexpression of Ccnt1 in the adult heart leads to Myc driven transcription and proliferation of cardiomyocytes
   (a) Immunohistochemistry (left) and quantification (right) of Ccnt1 in the liver isolated from adult *R26*^{*CMER*/+} or heart isolated from *Myh6-Cre; R26*^{*LSL-CMER*/+} mice systemically infected with adeno associated virus (serotype 9, 1 x 1011 vg/mouse) encoding either *RFP (AAV9-RFP,* n=5) or *Ccnt1* (*AAV9-Ccnt1*, n=4) 4 weeks post infection. Representative images based on analysis of 5 independent mice.
   (b) Immunoblot analysis of the C-terminal domain of phosphorylated RNA Polymerase II p-Rpb1(S2)), CDK9 and Cyclin T1 expression in the heart isolated from adult *Myh6-Cre; R26*^{*LSL-CMER*/+} mice systemically infected with adeno associated virus encoding either *RFP (AAV9-RFP)* or *Ccnt1 (AAV9-Ccnt1)* 4 weeks post infection. Sample loading was normalized for equal protein content. Expression of GAPDH is included as a confirmation of efficiency of protein isolation and comparable loading between individual tissue samples. Replicate samples are derived from independent mice.
   (c) Quantitative RT-PCR analysis of *Cad, Bzw2, Pinx1, Polr3d, St6* and Cdc25a expression in control (*R26*^{*LSL-CMER*/+}; no cre, n=5) and *Myh6-Cre; R26*^{*LSL-CMER*/+} *(R26*^{*LSL- CMER*/+};*cre*+, n=7) adult mouse heart isolated 4 weeks post systemic infection with an adeno associated virus encoding *Ccnt1 (AAV9-Ccnt1)* and 4 hours post administration of 4-OHT. Expression is normalized to *Actin* and *Gapdh* and relative to the respective wildtype (*R26*^{*LSL-CMER*/+}). Error bars show s.e.m. Unpaired t-test; no cre vs cre+: P<0.0005***(Cad, *Bzw2, Pinx1* and *St6),* P=0.0013** (Cdc25a), P=0.0035** *(Polr3d).* Replicate samples are derived from independent mice.
   (d) Enrichment of common Myc targets (liver, lung, kidney and heart) in cardiomyocytes isolated from the heart of AAV-CCNT1 infected *Myh6-cre; R26*^{*LSL-CMER*/+} (Myc+CCNT1) mice in comparison to uninfected *Myh6-cre; R26*^{*LSL-CMER*/+} mice (Myc) at 4 hours post administration of 4-OHT, as determined by RNA sequencing. Including normalised enrichment score (NES) and FDR q-value (FDR).
   (e) Immunofluorescent staining of cardiac troponin, p-H3,Ki67, PCM1, Wheat Germ Agglutinin (WGA) and Aurora B in control (*R26*^{*LSL-CMER*/+}; no cre) and *Myh6-Cre; R26*^{*LSL- CMER*/+}(*R26*^{*LSL-CMER*/+;}cre+) adult mouse heart isolated 4 weeks post systemic infection with an adeno associated virus encoding *Ccnt1 (AAV9-Ccnt1)* and 48 hours post administration of tamoxifen. Representative images based on analysis of 5 independent mice.
   (f) Quantification of p-H3 positive nuclei percentage in control (*R26*^{*LSL-CMER*/+}; no cre, n=9) and *Myh6-Cre; R26*^{*LSL-CMER*/+} (*R26*^{*LSL-CMER*/+};cre+, n=7) adult mouse heart isolated 4 weeks post systemic infection with an adeno associated virus encoding *Ccnt1* (*AAV9-Ccnt1)* and 48 hours post administration of tamoxifen. Means are taken from 5 images per mouse; Error bars show s.e.m. Unpaired t-test; no cre vs cre+ P<0.0001.
   (g) The weight (mg) of hearts isolated from control (*R26*^{*LSL-CMER*/+}; no cre, n>5) and *Myh6-Cre; R26*^{*LSL-CMER*/+} (*R26*^{*LSL-CMER*/+};cre+, n>6) adult mouse heart 4 weeks post systemic infection with an adeno associated virus encoding *Ccnt1 (AAV9-Ccnt1)* and 48 and 72 hours post administration of tamoxifen, expressed as fold change over the length (mm) of a tibia isolated from the same mouse. Error bars show s.e.m. Two Way ANOVA with Tukey's multiple comparisons test; no cre vs cre+: P=0.05* (48 hours), P=0.001*** (72 hours). Replicate samples are derived from independent mice.
   (h) Image of the whole heart and a tibia from control (*R26*^{*LSL-CMER*/+}; no cre) and *Myh6-Cre; R26*^{*LSL-CMER*/+} (*R26*^{*LSL-CMER*/+};cre+) adult mouse heart isolated 4 weeks post systemic infection with an adeno associated virus encoding *Ccnt1 (AAV9-Ccnt1)* and 72 hours post administration of tamoxifen. Scale bar represents 10mm. Representative images based on analysis on ≥5 independent mice.
   (i) Quantification of the number and size of cardiomyocytes from control (*R26*^{*LSL- CMER*/+}; no cre, n=5) and *Myh6-Cre; R26*^{*LSL-CMER*/+} (*R26*^{*LSL-CMER*/+};cre+, n=6) adult mouse heart isolated 4 weeks post systemic infection with an adeno associated virus encoding *Ccnt1* (*AAV9-Ccnt1*) and 72 hours post administration of tamoxifen. Error bars show s.d. Mann Whitney test; no cre vs cre+ P= 0.0043**. Replicate samples are derived from independent mice.

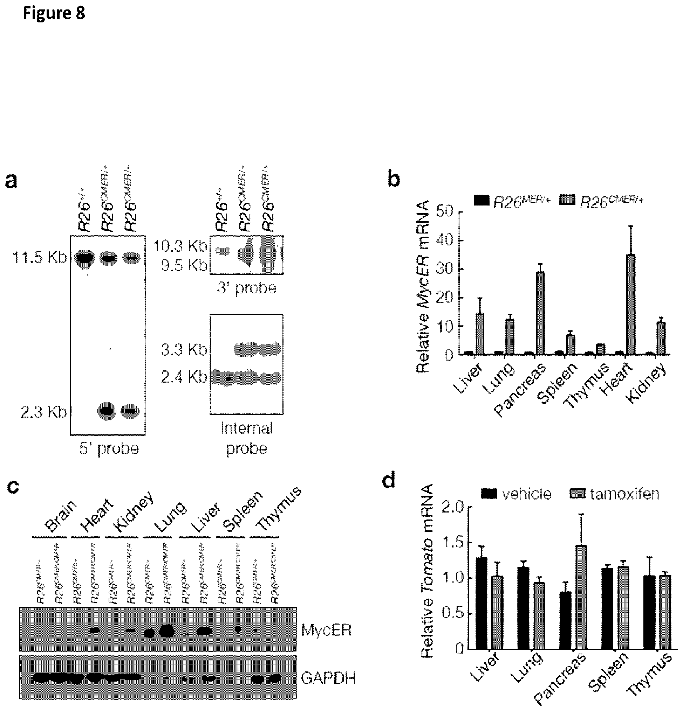

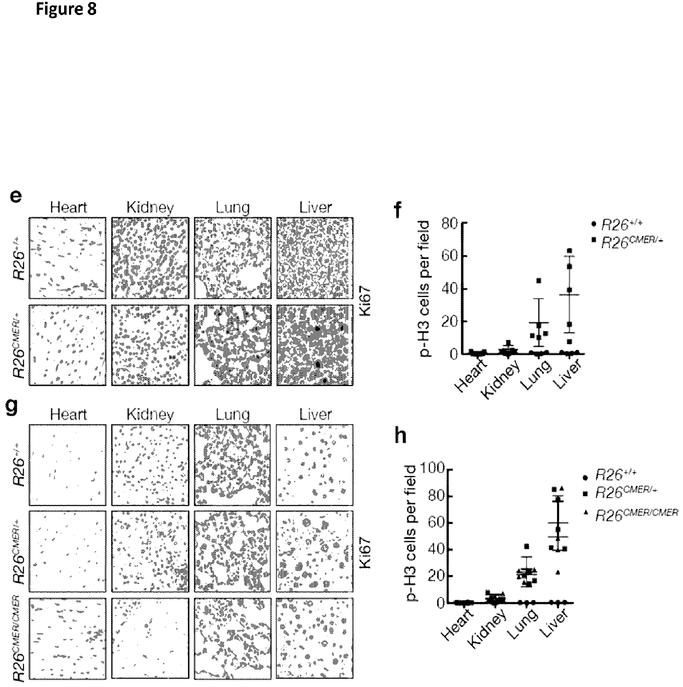

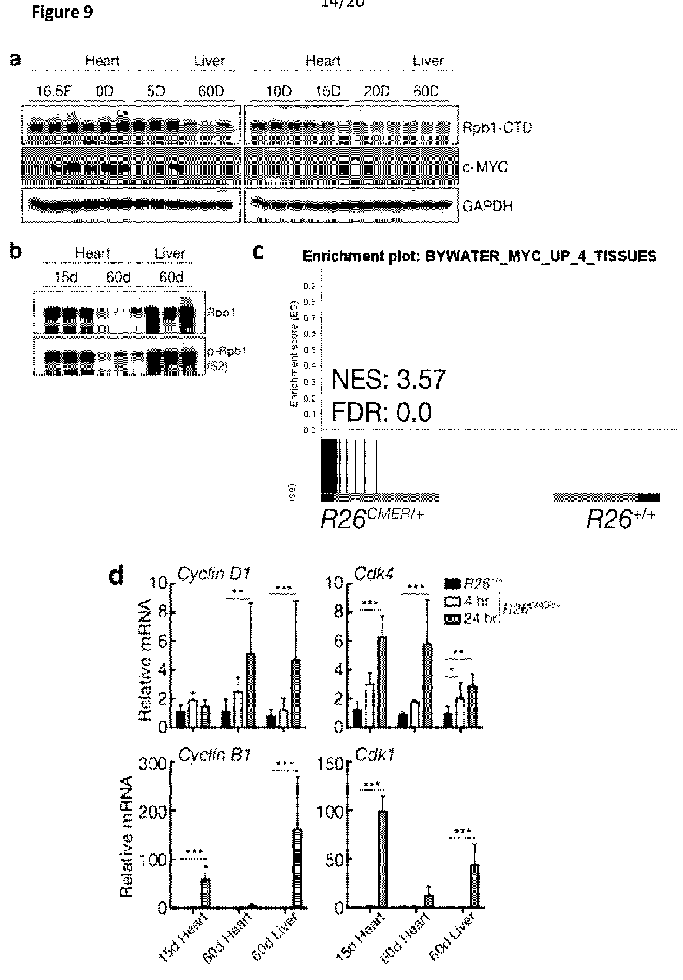

**Figure 8** shows (a) Southern blot analysis of digested DNA from R26+/+ wild-type and R26CMER/+ mice, analysed with 5' (EcoRI), 3' (EcoRI/Sall) and internal (BamHI) probes. Wild-type fragments (11.5 kb, 5'; 10.3 kb, 3' and 2.4 kb, internal) are present in wild-type and R26CMER/+ mice whereas the targeted fragments (2.3 kb, 5'; 9.5 kb, 3' and 3.3 kb, internal) are present in only R26CMER/+ mice.
   (b) Quantitative RT-PCR analysis of MycER in R26MER/+ (n=4) and R26CMER/+ (n=4) liver, lung, pancreas, spleen, thymus, heart and kidney. Expression is normalized to Actin and Gapdh and relative to the respective R26MER/+. Error bars show s.e.m. Replicate samples are derived from independent mice.
   (c) Immunoblot analysis of MycERT2 expression in the brain, heart, kidney, lung, pancreas, liver, spleen and thymus isolated from R26CMER/+ and R26CMER/CMER mice. Sample loading was normalized for equal protein content, as determined by a bicinchoninic acid assay (BCA). Expression of GAPDH is included as a confirmation of efficient protein isolation and equal loading within individual tissue samples.
   (d) Quantitative RT-PCR analysis of Tomato in R26CMER/mTmG liver, lung, pancreas, spleen, thymus, heart and kidney isolated 4 hours post administration of oil (vehicle, n≥3) or tamoxifen (n≥3). Expression is normalized to Actin and Gapdh and relative to the respective vehicle control. Error bars show s.e.m. Replicate samples are derived from independent mice.
   (e) Immunohistochemistry of Ki67 in the heart, kidney, lung and liver isolated from wild-type (R26+/+) and R26CMER/+ mice 72 hours post administration of tamoxifen. Representative images based on analysis of 4 independent mice.
   (f) Quantification of p-H3 positive nuclei per field of view of the heart, kidney, lung and liver (positive hepatocyte nuclei only) isolated from wild-type (R26+/+, n=4) and R26CMER/+ (n=5) mice 72 hours post administration of tamoxifen. Means are taken from 5 images per mouse; error bars show s.d.
   (g) Immunohistochemical staining of Ki67 in the heart, kidney, lung and liver isolated from wild-type (R26+/+), R26CMER/+ and R26CMER/CMER mice 24 hours post administration of tamoxifen Representative images based on analysis of at least 3 independent mice.
   (h) Quantification of p-H3 positive nuclei per field of view of the heart, kidney, lung and liver (positive hepatocyte nuclei only) isolated from wild-type (R26+/+, n=3), R26CMER/+ (n=5) and R26CMER/CMER (n=4) mice 24 hours post administration of tamoxifen. Means are taken from 5 images per mouse; error bars show s.d.

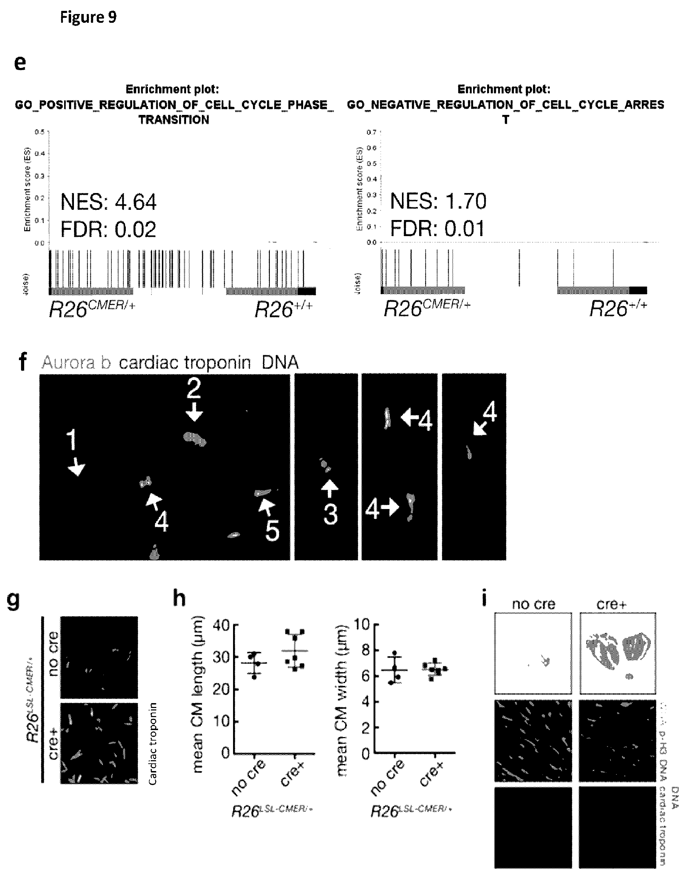

**Figure 9** shows (a) Immunoblot analysis of the C-terminal domain of RNA Polymerase II, total (Rpb1) and c-Myc protein expression in wild-type heart isolated from embryos at 16.5 days post fertilisation (E16.5), neonates at birth (OD), 5 day (5D), 10 day (10D) 15 day (15D) and 20 day (20D) old mice and wild-type liver isolated from 60 day (60D) old mice. Expression of GAPDH is included as a loading control. Replicate samples are derived from independent mice.
   (b) Longer exposure of Immunoblot shown in Fig. 9a, showing the C-terminal domain of RNA Polymerase II and total (Rpb1) and phosphorylated (p-Rpb1(S2)) expression in wild-type heart and liver isolated from 15 day-old (15d) and 60 day-old (60d) mice.
   (c) Enrichment of common Myc targets (liver, lung, kidney and heart) in heart isolated from 15 day R26CMER/+ mice in comparison to controls (R26+/+) at 4 hours post administration of 4-OHT, as determined by RNA sequencing. Including normalised enrichment score (NES) and FDR q-value (FDR).
   (d) Quantitative RT-PCR analysis of Cyclin D1, Cdk4, Cyclin B1 and Cdk1 in wild-type (R26+/+, n≥5) and R26CMER/+heart isolated from 15 day-old (15d Heart, n≥4) mice versus heart and liver isolated from 60 day-old (60d Heart, n≥5)(60d Liver, n≥4) mice 4 (light grey) or 24 (dark grey) hours post i.p. of 4-OHT. Expression is normalized to Actin and Gapdh and relative to the respective wildtype (R26+/+). Error bars show s.d. One Way ANOVA with Tukey's multiple comparisons test; 15d heart R26+/+vs 24 hour R26CMER/+: P=0.001*** (Cdk4, Cyclin B1 and Cdk1), 60d heart R26+/+vs 24 hour R26CMER/+: P=0.01** (Cyclin D1) P=0.001*** (Cdk4), 60d liver R26+/+vs 4 hour R26CMER/+: P=0.05* (Cdk4), 60d liver R26+/+vs 24 hour R26CMER/+: P=0.01** (Cdk4) P=0.001*** (Cyclin D1, Cyclin B1, Cdk1). Replicate samples are derived from independent mice.
   (e) Enrichment of pro-cell cycle progression gene sets in heart isolated from 15 day R26CMER/+ mice in comparison to controls (R26+/+) at 4 hours post administration of 4- OHT, as determined by RNA sequencing. Including normalised enrichment score (NES) and FDR q-value (FDR).
      Immunofluorescent staining of cardiac troponin and Aurora B in the heart of 15 day-old
   (f) Myh6-Cre; R26LSL-CMER/+ mice 48 hours post administration of tamoxifen. Arrows show differences in Aurora B localization throughout the cell cycle. 1- G2, nucleus. 2-Metaphase, metaphase chromosomes. 3- Anaphase, mid-zone. 4-Cytokinesis, centrally located mid-body. 5- Failing cytokinesis- laterally displaced mid-body.
   (g) Immunofluorescent staining of cardiac troponin T (green) positive cardiomyocytes isolated from fixed and dissociated hearts of 15 day-old control (*R26LSL-CMER*/*+;* no cre) and *Myh6-Cre; R26LSL-CMER*/*+(R26LSL-CMER*/*+;cre*+) mice.
   (h) Quantification of the length and width of cardiomyocytes isolated from the hearts of 15 day-old control (R26LSL-CMER/+; no cre, n=4) and Myh6-Cre; R26LSL-CMER/+(R26LSL-CMER/+;cre+, n=6) mice 48 hours post administration of tamoxifen. Mean determined from the measurement of >13 individual cardiomyocytes per mouse. Error bars show s.e.m. Mann Whitney test; no cre vs cre+ P= not significant. Replicate samples are derived from independent mice.
   (i) Top-H&E staining, middle-WGA and p-H3 immunofluorescent staining, bottom-cardiac troponin immunofluorescent staining of hearts from 15 day-old control (R26LSL-CMER/+; no cre) and Myh6-Cre; R26LSL-CMER/+(R26LSL-CMER/+;cre+) mice 96 hours post administration of tamoxifen.

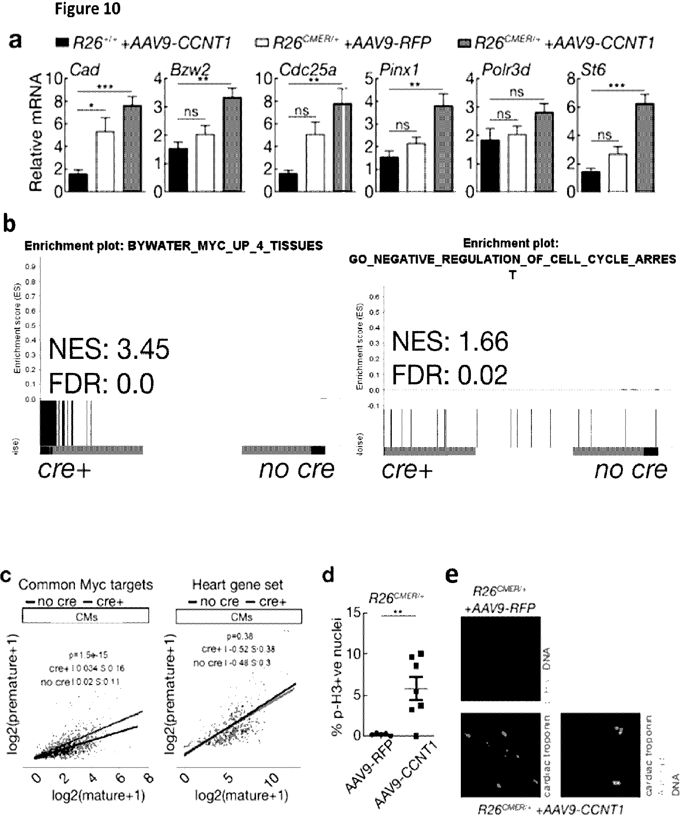

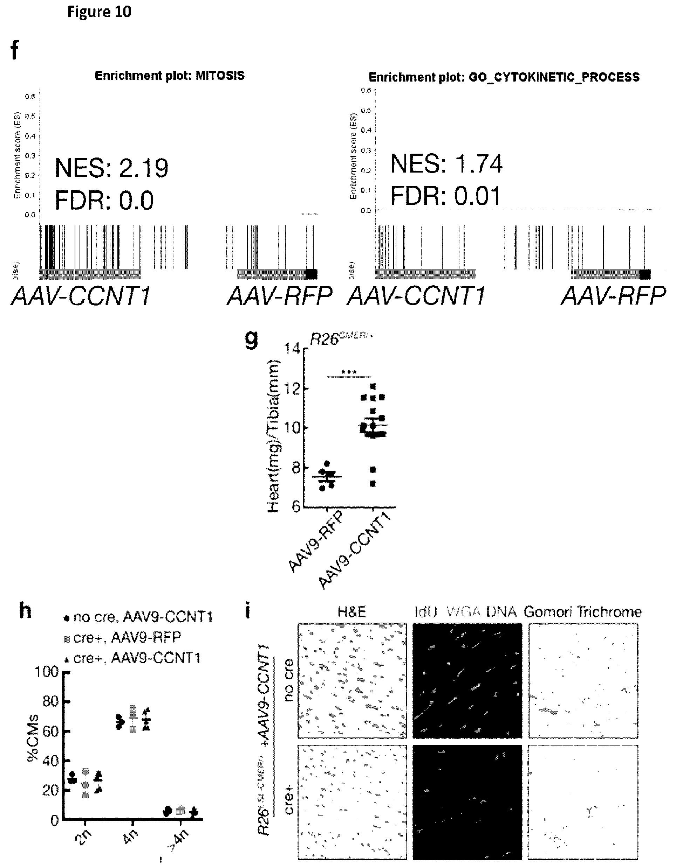

**Figure 10** shows (a) Quantitative RT-PCR analysis of Cad, Bzw2, Pinx1, Polr3d, St6 and Cdc25a expression in adult wildtype (R26+/+, n=6) and R26CMER/+mouse heart isolated 4 weeks post systemic infection with an adeno associated virus encoding either RFP (AAV9-RFP, n=4) or Ccnt1 (AAV9-Ccnt1, n=6) and 4 hours post administration of 4-OHT. Expression is normalized to Actin and Gapdh and relative to the respective wildtype. Error bars show s.e.m. Two Way ANOVA with Tukey's multiple comparisons test; R26+/+ AAV9-Ccnt1 vs R26CMER/+ AAV9-RFP: P=0.05* (Cad). R26+/+ AAV9-Ccnt1 vs R26CMER/+ AAV9-Ccnt1: P=0.01** (Bzw2, Cdc25a and Pinx1). R26+/+ AAV9-Ccnt1 vs R26CMER/+ AAV9-Ccnt1: P=0.001*** (Cad and St6).Replicate samples are derived from independent mice.
   (b) Top- enrichment of common Myc targets (liver, lung, kidney and heart), bottom-enrichment of a pro-cell cycle progression gene set in heart isolated from adult Myh6-Cre; R26LSL-CMER/+(cre+) in comparison to control (R26LSL-CMER/+, no cre), both treated with AAV-CCNT1 4 weeks before administration of 4-OHT (4hr), as determined by RNA sequencing. Including normalised enrichment score (NES) and FDR q-value (FDR).
   (c) Relative amounts of premature and mature mRNA expressed from common Myc target genes (left) and heart-specific genes (right) was determined from RNA sequencing performed on cardiomyocytes purified from adult Myh6-Cre; R26LSL-CMER/+ (cre+) and control (R26LSL-CMER/+, no cre) mice, both treated with AAV-CCNT1 4 weeks before administration of 4-OHT (4hr), as determined by RNA sequencing. Intercept (I) and slope (S) included.
   (d) Quantification of p-H3 positive nuclei per field of view from adult R26CMER/+mouse hearts isolated 4 weeks post systemic infection with an adeno associated virus encoding either RFP (AAV9-RFP, n=5) or Ccnt1 (AAV9-Ccnt1, n=7) and 48 hours post administration of tamoxifen. Means are taken from 5 images per mouse; Error bars show s.e.m. Unpaired t-test; AAV9-RFP vs AAV9-Ccnt1, P=0.0110.
   (e) Immunofluorescent staining of cardiac troponin (red), p-H3 (green, left) and Aurora B positive mitotic nuclei (green, right) from adult R26CMER/+mouse hearts isolated 4 weeks post systemic infection with an adeno associated virus encoding either RFP (AAV9-RFP) or Ccnt1 (AAV9-Ccnt1) and 48 hours post administration of tamoxifen. Representative images based on analysis of 5 independent mice.
   (f) Enrichment of mitosis and cytokinesis gene sets in heart isolated from adult Myh6-Cre;R26LSL-CMER/+ mice 4 weeks post systemic infection with AAV-CCNT1 verses AAV-RFP and hours post administration of tamoxifen, as determined by RNA sequencing. Including normalised enrichment score (NES) and FDR q-value (FDR).
   (g) The weight (mg) of hearts isolated from adult R26CMER/+mouse hearts isolated 4 weeks post systemic infection with an adeno associated virus encoding either RFP (AAV9-RFP, n=5) or Ccnt1 (AAV9-Ccnt1, n=15) and 48 hours post administration of tamoxifen, expressed as fold change over the length (mm) of a tibia isolated from the same mouse. Error bars show s.e.m. Unpaired t-test; AAV9-RFP vs AAV9-Ccnt1, P=0.0006. Replicate samples are derived from independent mice.
   (h) Nuclei quantification performed on images of cardiomyocytes isolated from control (R26LSL-CMER/+ ; no cre) and Myh6-Cre; R26LSL-CMER/+ (R26LSL-CMER/+ ; cre+) adult mouse hearts isolated 4 weeks post systemic infection with an adeno associated virus encoding Ccnt1 (AAV9-Ccnt1) or RFP (AAV9-RFP) and 72 hours post administration of tamoxifen. Error bars show s.d. Mann Whitney test; no cre vs cre+ P= 0.0043**. Replicate samples are derived from independent mice.
   (i) Left- H&E staining, middle- IdU (BrdU) and WGA immunofluorescent staining and, right- Gomori Trichrome staining from control (R26LSL-CMER/+ ; no cre) and Myh6-Cre; R26LSL-CMER/+ (R26LSL-CMER/+ ; cre+) adult mouse heart. Mice were systemically infected with an adeno associated virus encoding Ccnt1 (AAV9-Ccnt1) at 4 weeks of age, given a single injection of tamoxifen to transiently activate MycER at 8 weeks of age and collected at 16 weeks of age.

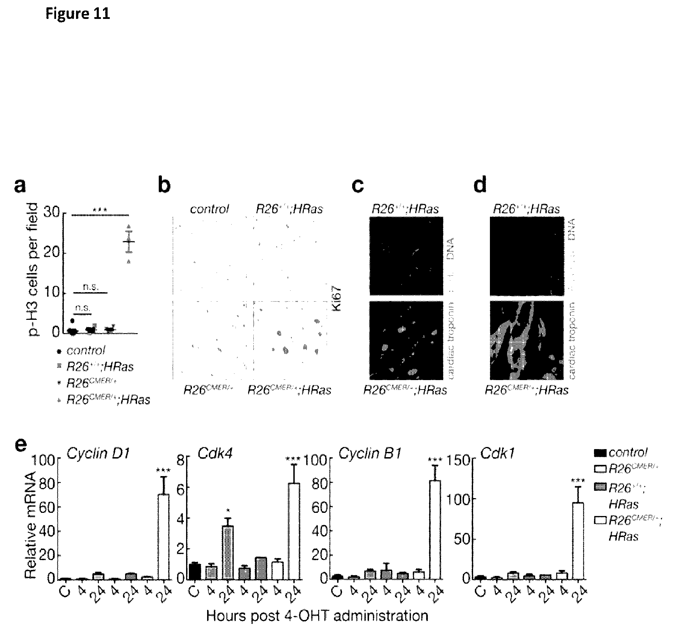

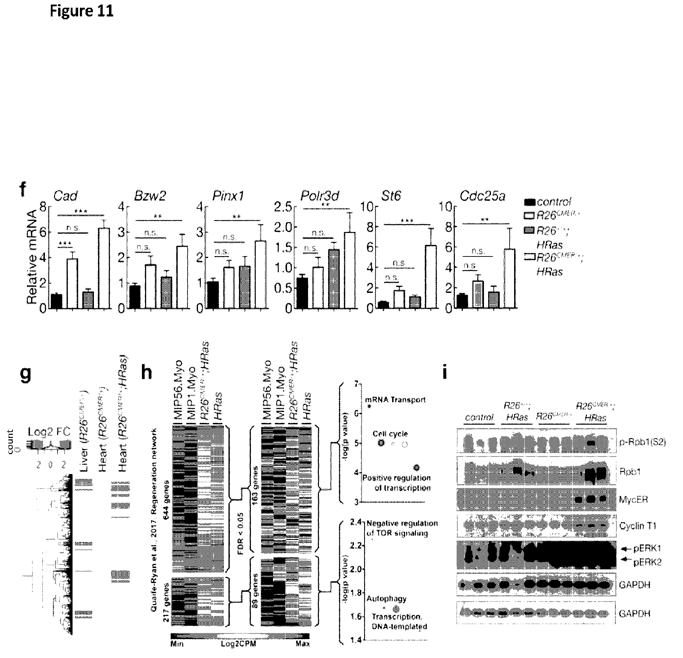

**Figure 11** shows indirectly increasing PTEF-b activity leads to Myc driven transcription and proliferation in cardiomyocytes
   (a) Quantification of p-H3 positive nuclei per field of view in hearts isolated from control (R26+/+; TetO-HRas, R26+/+; Myh6-tTA or R26+/+, black, n=8), TetO-HRas; Myh6-tTA; R26+/+ (R26+/+;HRas, red, n=5), R26CMER/+ (blue, n=4) and TetO-HRas; Myh6-tTA; R26 CMER/+ (R26 CMER/+;HRas, purple, n=3) mice 4 weeks post withdrawal of doxycycline and 24 hours post administration of tamoxifen. Representative images based on analysis of 5 images per mouse; error bars show s.e.m. One Way ANOVA with Tukey's multiple comparisons test; control vs TetO-HRas; Myh6-tTA; R26 CMER/+: P=0.001***.
   (b) Immunohistochemistry of Ki67 in hearts isolated from control (R26+/+; TetO-HRas, R26+/+; Myh6-tTA or R26+/+), TetO-HRas; Myh6-tTA; R26+/+ (R26+/+;HRas), R26CMER/+ and TetO-HRas; Myh6-tTA; R26 CMER/+ (R26 CMER/+;HRas) mice 4 weeks post withdrawal of doxycycline and 24 hours post administration of tamoxifen. Representative images based on analysis of at least 3 independent mice.
   (c) Immunofluorescent staining of cardiac troponin (red) and p-H3 positive mitotic nuclei (green) in the heart of TetO-HRas; Myh6-tTA; R26 CMER/+ /+ (R26 CMER/+;HRas) mice and TetO-HRas; Myh6-tTA; R26+/+ control (R26+/+;HRas) 4 weeks post withdrawal of doxycycline and 24 hours post administration of tamoxifen. Representative images based on analysis of 5 independent mice.
   (d) Immunofluorescent staining of cardiac troponin (red) and Aurora B (green) in the heart of TetO-HRas; Myh6-tTA; R26 CMER/+ (R26 CMER/+;HRas) mice and TetO-HRas; Myh6-tTA; R26+/+ control (R26+/+;HRas) 4 weeks post withdrawal of doxycycline and 24 hours post administration of tamoxifen. Representative images based on analysis of 5 independent mice.
   (e) Quantitative RT-PCR analysis of Cyclin D1, Cdk4, Cyclin B1 and Cdk1 in control (C; R26+/+; TetO-HRas, R26+/+; Myh6-tTA or R26+/+, n≥24), TetO-HRas; Myh6-tTA; R26+/+(R26+/+;HRas, n≥5), R26CMER/+ (n≥14) and TetO-HRas; Myh6-tTA; R26 CMER/+ (R26 CMER/+;HRas, n=8) mice 4 weeks post withdrawal of doxycycline and 4 hours or 24 hours post administration of 4-OHT. Expression is normalized to Actin and Gapdh and relative to the respective control. Error bars show s.e.m. One Way ANOVA with Tukey's multiple comparisons test; control vs 24 hour R26CMER/+: P=0.05* (Cdk4), control vs 24 hour TetO-HRas; Myh6-tTA; R26 CMER/+: P***=0.001 (Cyclin D1, Cdk4, Cyclin B1, Cdk1). Replicate samples are derived from independent mice.
   (f) Quantitative RT-PCR analysis of Cad, Bzw2, Pinx1, Polr3d, St6 and Cdc25a in control (R26+/+; TetO-HRas, R26+/+; Myh6-tTA or R26+/+, n≥14), TetO-HRas; Myh6-tTA; R26+/+(R26+/+;HRas, n=4), R26CMER/+ (n=6) and TetO-HRas; Myh6-tTA; R26 CMER/+ (R26 CMER/+;HRas, n=5) mice 4 weeks post withdrawal of doxycycline and 4 hours post administration of 4-OHT. Expression is normalized to Actin and Gapdh and relative to the respective control. Error bars show s.e.m. Two Way ANOVA with Tukey's multiple comparisons test; control vs R26CMER/+: P=0.001*** (Cad), control vs TetO-HRas; Myh6-tTA; R26 CMER/+: P**=0.01 (Bzw2, Pinx1, Polr3d, Cdc25a) P***=0.001 (St6). Replicate samples are derived from independent mice.
   (g) Heat map showing the union of DEGs called in each tissue; adult liver and adult heart. Shown are mRNA expression fold changes (Log2) upon MycERT2 activation relative to wild-type, as determined by RNA sequencing of R26CMER/+ (n≥3) mice in comparison to wild-type (R26+/+, n≥3) and TetO-HRas; Myh6-tTA; R26 CMER/+ mice (R26 CMER/+;HRas, n=3) in comparison to TetO-HRas; Myh6-tTA; R26+/+ mice (R26+/+;HRas, n=3) at 4 hours post administration of 4-OHT.
   (h) Heat map depicts gene expression of the "regeneration network" (described in Quaife-Ryan et al., 2017) from RNA-seq of TetO-HRas; Myh6-tTA; R26+/+ (R26+/+;HRas, n=3) and TetO-HRas; Myh6-tTA; R26 CMER/+ P60 adult hearts (R26 CMER/+;HRas, n=3). MIP1Myo and MIP56.Myo denote neonatal (P1) or adult (P56) cardiomyocytes isolated from myocardial infarction-operated hearts (n=4). Regeneration network genes were filtered for genes differentially expressed (DEGs) between TetO-HRas; Myh6-tTA; R26+/+ (R26+/+;HRas) and TetO-HRas; Myh6-tTA; R26 CMER/+ (R26 CMER/+;HRas) hearts (FDR < 0.05).
   (i) Immunoblot analysis of the C-terminal domain of RNA Polymerase II, total (Rpb1) and phosphorylated (p-Rpb1(S2)), MycERT2, Cyclin T1 and phosphorylated ERK1 (T202/T204) and ERK2 (T185/T187) protein expression in hearts isolated from control (R26+/+; TetO-HRas, R26+/+; Myh6-tTA or R26+/+), TetO-HRas; Myh6-tTA; R26+/+ (R26+/+;HRas), R26CMER/+ and TetO-HRas; Myh6-tTA; R26 CMER/+ (R26 CMER/+;HRas) mice 4 weeks post withdrawal of doxycycline and 4 hours post administration of 4-OHT. Replicate samples are derived from independent mice.

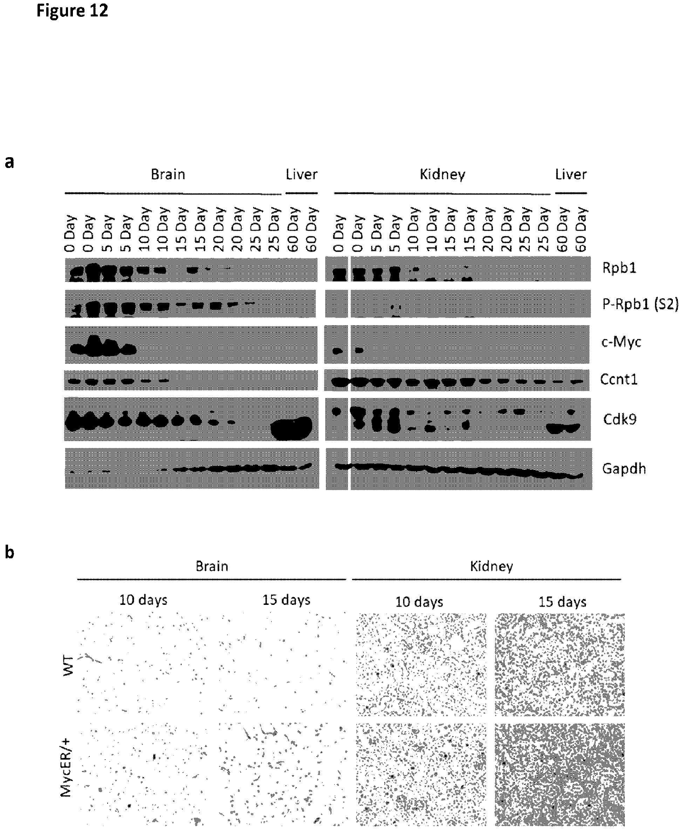

**Figure 12** shows (a) Immunoblot analysis of the C-terminal domain of RNA Polymerase II - total (Rpb1) and phosphorylated (p-Rpb1(S2)), CDK9, Cyclin T1 and endogenous c-Myc in wild-type brain and kidney over time, compared to adult liver. Sample loading was normalized for equal protein content. Expression of GAPDH is included as a confirmation of efficiency of protein isolation and comparable loading between individual tissue samples. Replicate samples are derived from independent mice. The composite figure is generated from the same samples loaded across multiple blots and a representative image for GAPDH is shown. (b) Immunohistochemical staining of p-H3, in the brains and kidneys of 10 and 15-day-old R26CMER/+ and wild-type mice, 24hrs post Myc activation by tamoxifen.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The practice of the present invention will employ, unless otherwise indicated, conventional techniques of microbiology, tissue culture, molecular biology, chemistry, biochemistry, recombinant DNA technology, and bioinformatics which are within the skill of the art. Such techniques are explained fully in the literature.

As used herein, the words "nucleic acid", "nucleic acid sequence", "nucleotide", "nucleic acid molecule" or "polynucleotide" are intended to include DNA molecules (e.g., cDNA or genomic DNA), RNA molecules (e.g., mRNA), natural occurring, mutated, synthetic DNA or RNA molecules, and analogs of the DNA or RNA generated using nucleotide analogs. It can be single-stranded or double-stranded. Such nucleic acids or polynucleotides include, but are not limited to, coding sequences of structural genes, anti-sense sequences, and non-coding regulatory sequences that do not encode mRNAs or protein products. These terms also encompass a gene. The term "gene" or "gene sequence" is used broadly to refer to a DNA nucleic acid associated with a biological function. Thus, genes may include introns and exons as in the genomic sequence, or may comprise only a coding sequence as in cDNAs, and/or may include cDNAs in combination with regulatory sequences.

The terms "polypeptide" and "protein" are used interchangeably herein and refer to amino acids in a polymeric form of any length, linked together by peptide bonds.

In one aspect of the invention there is provided a nucleic acid molecule comprising a nucleic acid sequence encoding a myc transcription factor selected from c-myc, I-myc or n-myc and cyclin T1 protein for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

Cyclin T1 is encoded by the ccnt1 gene and such terms are used interchangeably throughout the application.

In one embodiment, the nucleic molecule is a ribonucleotide (RNA) molecule. Accordingly, in one embodiment of the invention there is provided a ribonucleotide, preferably an mRNA encoding a myc transcription factor selected from c-myc, I-myc or n-myc and cyclin T1 protein, wherein the ribonucleotide encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25; and wherein the mRNA comprises at least one modification selected from a cap modification, a tail modification, a nucleoside modification and an untranslated region (UTR) modification.

In one embodiment, myc is selected from c-myc, I-myc or n-myc. In a most preferred embodiment, myc is c-myc.

Not encompassed by the claims, the (unmodified) RNA sequence of *c-myc* may comprise or consist of a sequence as defined in SEQ ID NO: 15 or a functional variant thereof. In an alternative embodiment, myc is an inducible form of myc as defined below. Not encompassed by the claims, where the myc is I-myc or n-myc, the DNA sequence that encodes the unmodified mRNA sequence of the I-myc or n-myc may comprise or consist of a sequence as defined in SEQ ID NO: 22 or SEQ ID NO: 24 respectively, or a functional variant thereof.

The amino acid sequences of I-myc and n-myc are given as SEQ ID NO: 23 and 25 respectively.

In one embodiment, not encompassed by the claims, the cyclin T1 RNA sequence comprises or consists of SEQ ID NO: 13.

In a preferred embodiment, the cap modification comprises the introduction of an anti-reverse-cap analogue (ARCA) comprising a modified cap structure containing a 5'-5' triphosphate bridge, wherein this modification increases the efficiency of mRNA translation. More preferably, the ARCA comprises a high number of modified cap structures and elongated 5'-5' phosphate bridges, wherein this increases the efficiency of mRNA translation as well as the stability of the mRNA.

In a further preferred embodiment, the tail modification comprises a poly(A) tail comprising at least 20 poly(A)s, wherein this increases the efficiency of mRNA translation and stability of the mRNA.

In another preferred embodiment, the nucleoside modification is the introduction of a modified nucleoside selected from one or more of the following: 2'-O methyl nucleoside, pseudouridine, 2-thiouridine, 5-methylcytidine, 6-methyladenosine and inosine, wherein the modification reduces immune activation, preferably TLR-mediated immune activation.

In a further preferred embodiment, the UTR modification comprises replacing unstable non-coding sequences (for example, AU-rich sequences in the UTR) with non-coding sequences of mRNAs that are known to be stable (for example, 3'UTR of the human globin gene), wherein the modification improves stability of the mRNA.

Not encompassed by the claims, in another embodiment, the modification is codon optimisation.

Methods for synthesising modified RNA molecules are known in the art - for example, as described in Kondrat et al. 2017.

Not encompassed by the claims, in a particularly preferred embodiment, the modification comprises a cap modification and at least one modified nucleoside, preferably the modification of uracil to pseudouridine and/or the modification of cytidine to 5-methylcytidine. Accordingly, in one embodiment, the RNA sequence of myc may comprise a sequence as defined in SEQ ID NO: 37 or a functional variant thereof; the RNA sequence of cyclin-T1 may comprise a sequence as defined in SEQ ID NO: 16.

In an alternative embodiment, the nucleic acid molecule is a nucleic acid construct or vector (such terms may be used interchangeably). Accordingly, in one embodiment there is provided a vector comprising a nucleic acid molecule comprising a nucleic acid sequence encoding a myc transcription factor selected from c-myc, I-myc or n-myc and a cyclin T1 protein for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

In one embodiment, the vector comprises a nucleic acid sequence encoding an inducible form of myc and a nucleic acid sequence encoding a cyclin T1 protein, wherein the nucleic acid sequence encodes an inducible myc transcription factor as defined in SEQ ID NO: 1 or 10 or a functional variant thereof wherein the functional variant retains the transcription factor activity of the full non-variant sequence and has at least 75% overall sequence identity to SEQ ID NO: 1 or 10, operably linked to a first regulatory sequence; and a nucleic acid sequence encoding a cyclin T1 protein as defined in SEQ ID NO: 5 or a functional variant thereof wherein the functional variant retains the biological function of the full non-variant sequence and has at least 75% overall sequence identity to SEQ ID NO: 5, operably linked to the first regulatory sequence or a second regulatory sequence.

By "inducible" is meant that the expression and/or activity of the myc transcription factor is responsive to the presence or absence of an external stimuli. That is, expression and/or activity of the myc transcription factor can be turned on or off by the presence or absence of an external stimuli. Preferably the external stimuli acts as a reversible switch, meaning that expression and/or activity can be turned on and then off again by the presence of the external stimuli.

Not encompassed by the claims, in one embodiment, the activity of myc may be regulated using a myc fusion protein. For example, myc may be fused to a steroid or thyroid binding domain, such as for example, an estrogen receptor (ER), glucocorticoid receptor (GR), progesterone receptor (PR), thyroid receptor (TR), mineralocorticoid receptor (MR) or androgen receptor (AR) ligand binding domain. More preferably, the steroid or thyroid binding domain may be modified. For example, the ER binding domain may be modified to bind tamoxifen or the PR binding domain may be modified to bind RU486.

Not encompassed by the claims, in a preferred example, myc is fused to a modified hormone-binding domain of the estrogen receptor - called ER^{T2} as described in Murphy et al. 2008. In this system binding of 4-hydroxytamoxifen (4-OHT) to the ER binding domain leads to a conformational change and activation of the myc fusion protein.

In one embodiment, the inducible myc transcription factor is defined in SEQ ID NO: 1 or 10 or a functional variant thereof, wherein the variant sequence retains the transcription factor activity of the full non-variant sequence and has at least 75% overall sequence identity to SEQ ID NO: 1 or 10.

Not encompassed by the claims, in a preferred embodiment, the nucleic acid sequence encoding the inducible myc transcription factor comprises SEQ ID NO: 2 or 9 or a functional variant thereof.

The term "operably linked" as used throughout refers to a functional linkage between the promoter sequence and the gene of interest, such that the promoter sequence is able to initiate transcription of the gene of interest.

Not encompassed by the claims, in one embodiment, the regulatory sequence is a promoter, preferably a constitutive or inducible promoter. An inducible promoter may be used with an inducible or non-inducible form of myc, but preferably an inducible promoter is used instead of an inducible form of myc.

Not encompassed by the claims, one example of an inducible promoter is the Tet-On or Tet-Off promoter system. Accordingly, in one embodiment, the promoter is a Tet-inducible promoter. In a further embodiment, the vector may additionally comprise a tetracycline-controlled transactivator (tTA) or a reverse tetracycline-controlled transactivator (rtTA). Preferably, the tTA or rtTA are operably linked to a regulatory sequence such as a promoter. Suitable promoters would be known to the skilled person. Alternatively, the tTA or rtTA may be provided as a separate vector.

According to all aspects of the invention, the term "regulatory sequence" is used interchangeably herein with "promoter" and all terms are to be taken in a broad context to refer to regulatory nucleic acid sequences capable of effecting expression of the sequences to which they are ligated. The term "regulatory sequence" also encompasses a synthetic fusion molecule or derivative that confers, activates or enhances expression of a nucleic acid molecule in a cell, tissue or organ.

The term "promoter" typically refers to a nucleic acid control sequence located upstream from the transcriptional start of a gene and which is involved in the binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue- specific manner. Also included within the term is a transcriptional regulatory sequence of a classical prokaryotic gene, in which case it may include a -35 box sequence and/or -10 box transcriptional regulatory sequences.

A "constitutive promoter" refers to a promoter that is transcriptionally active during most, but not necessarily all, phases of growth and development and under most environmental conditions, in at least one cell, tissue or organ. Examples of constitutive promoters include the CAG promoter, the Rosa26 promoter, the ubiquitin promoter, the actin promoter, the tubulin promoter, the GAPDH promoter, as well as the CMV, PGK, SV40, and EF1A promoters.

For the identification of functionally equivalent promoters, the promoter strength and/or expression pattern of a candidate promoter may be analysed for example by operably linking the promoter to a reporter gene and assaying the expression level and pattern of the reporter gene in various tissue. Suitable well-known reporter genes are known to the skilled person and include for example beta-glucuronidase or beta-galactosidase.

In one embodiment, the regulatory sequence of the vector is a promoter, preferably the CAG promoter, as defined in SEQ ID NO: 3 or a functional variant thereof.

Not encompassed by the claims, in one embodiment, to allow two proteins to be expressed as individual proteins from a single mRNA molecule, ribosomal skipping sequences may be added to the 5' and/or 3' end of the individual proteins (for example, myc and the cyclin T1). During translation, when the ribosome encounters a ribosomal skipping sequence it is prevented from creating the peptide bond with the last proline in the ribosomal skipping sequence. As a result, translation is stopped, the nascent polypeptide is released and translation is re-initiated to produce a second polypeptide.

This results in the addition of a C-terminal ribosomal skipping sequence (or the majority of such a sequence) to the first polypeptide chain, and an N-terminal proline to the next polypeptide. Not encompassed by the claims, accordingly, in a further embodiment, the nucleic acid construct comprises at least one ribosomal skipping sequence. In one example, the ribosomal skipping sequence is a 2A-like peptide, such as P2A (comprising ATNFSLLKQAGDVEENPGP, SEQ ID NO: 26), E2A (comprising QCTNYALLKLAGDV ESNPGP, SEQ ID NO: 27) or F2A (comprising VKQTLNFDLLKLAGDVESNPGP, SEQ ID NO: 28). In an alternative embodiment, the nucleic acid construct may comprise at least one 2A-like peptide which provides a proteolytic cleavage site, for example T2A (comprising EGRGSLLTCGDVEENPGP, SEQ ID NO: 29).

Not encompassed by the claims, alternatively, tRNA sequences may be used to allow multiple RNAs to be produced from a single engineered polycistronic gene consisting of tandemly arrayed tRNA-RNA units. After the polycistronic gene is transcribed by endogenous transcriptional machinery, the endonucleases RNAse P and RNAse Z (or RNAse E in bacterium) recognise and specifically cleave the tRNAs at specific sites at the 3' and 5' ends, releasing mature RNAs and tRNAs. Advantageously, tRNAs and their processing system are virtually conserved in all living organisms and therefore this method can be used in all known species. In particular, this technology can allow for the production of multiple excised mature gRNAs which can direct Cas9 to multiple targets, wherein the polycistronic gene contains tandemly arrayed tRNA-gRNA units, where each gRNA contains a target-specific spacer and a conserved gRNA scaffold (see Xie *et al*, 2015). Accordingly, in a further embodiment, the nucleic acid construct comprises at least one tRNA sequence.

Not encompassed by the claims, in one embodiment, at least one of myc and cyclin T1 may be flanked at the 5' or 3' ends by a ribosomal skipping sequence and/or a proteolytic cleavage site. The construct may further comprise linker sequences between one of myc and cyclin T1. In one example, the linker may be a GS linker.

In an alternative embodiment, the nucleic acid sequence encoding cyclin T1 and/or cyclin-dependent kinase 9 (CDK9) is operably linked to a second regulatory sequence. A regulatory sequence is described above. In one example, the regulatory sequence is the troponin T promoter, preferably as defined in SEQ ID NO: 4 or a functional variant thereof.

Not encompassed by the claims, in a further embodiment of the invention, the nucleic acid construct may further comprise at least one UTR, preferably a 5' and 3' UTR. In a further embodiment, at least one of myc and cyclin T1 may be flanked at their 5' and 3' ends with a UTR. In one embodiment, the sequence of the 5' UTR preferably comprises the consensus sequence: GCCRCC, where R is a purine, preferably A. In one example, the sequence of the 5'UTR comprises or consist of SEQ ID NO: 38 or a variant thereof. A variant is defined above.

In a preferred embodiment, the cyclin T1 nucleic acid encodes a protein as defined in SEQ ID NO: 5. Not encompassed by the claims, the cyclin T1 nucleic acid comprises or consists of a sequence as defined in SEQ ID NO: 6 or a functional variant thereof.

Not encompassed by the claims, in a further embodiment, the vector comprises at least one terminator sequence, which marks the end of the operon causing transcription to stop. A suitable terminator sequence would be well known to the skilled person.

Not encompassed by the claims, the cyclin T nucleic acid comprises or consists of a sequence as defined in SEQ ID NO: 8 or a functional variant thereof.

In one embodiment, the vector is a viral vector. More preferably, the viral vector is selected from adenoviruses, adeno-associated viruses (AAV), alphaviruses, flaviviruses, herpes simplex viruses (HSV), measles viruses, rhabdoviruses, retroviruses, lentiviruses, Newcastle disease virus (NDV), poxviruses, picornaviruses and hybrids thereof. In one embodiment, the vector is an adeno-associated virus (AAV). In a further preferred embodiment, the AAV may be selected from serotype 1, 2, 3, 4, 5, 6, 7, 8, 9, 10 or 11. The skilled person would understand that serotypes differ in their tropism and as such the selection of the target tissue will depend on the target tissue to be infected. In one example, where the target tissue is the heart, the AAV may be selected from serotypes 1, 8 and 9, and most preferably is AAV9.

Not encompassed by the claims, in an alternative embodiment, the vector is a non-viral vector, such as a plasmid. In this embodiment, a non-viral vector may be delivered to a target cell or tissue using transfection. Examples of suitable transfection techniques would be known to the skilled person and include chemical and physical transfection. In one embodiment, chemical transfection includes the use of calcium phosphate, lipid or protein complexes. In one example, the non-viral vector may be combined with a lipid solution to result in the formation of a liposome or lipoplex. In another embodiment, physical transfection means include electroporation, microinjection or the use of ballistic particles. In a further example, bacteria can be used to deliver a non-viral vector to a target cell or tissue. This is known as bactofection.

Not encompassed by the claims, in a further aspect of the invention, there is provided a RNA molecule obtained or obtainable by transcription of the nucleic acid construct described above. In one embodiment, where the nucleic acid construct comprises sequences encoding myc and cyclin T1, the RNA molecule obtained or obtainable by transcription of the nucleic acid construct is a bicistronic RNA.

In another aspect of the invention, there is provided a method of increasing at least one of proliferation, mitosis, cytokinesis in a cell *in vitro,* the method comprising introducing at least one nucleic acid molecule or composition as described herein to a cell, wherein the cell is a heart cell.

As used herein an "increase" in proliferation may refer to an increase in proliferation of at least 5%, 10%, 20%, 25%, 30%, 35%, 40%, 45%, 50%, 55%, 60%, 65%, 70%, 75%, 80%, 85%, 90%, 95%, 100% or more compared to the level in a control. As used herein a control may be considered a cell that does not comprise or express a vector of the invention. In one embodiment, an increase in cell proliferation may be assessed by an increase in cell number.

In another aspect of the invention there is provided a method of increasing organ size *in vitro,* the method comprising introducing at least one nucleic acid molecule or a composition as described herein to at least one cell in the organ, wherein the organ is a heart.

As used herein, an "increase" in organ size may refer to an increase in heart size of at least 5%, 10%, 20%, 25%, 30%, 35%, 40%, 45%, 50%, 55%, 60%, 65%, 70%, 75%, 80%, 85%, 90%, 95%, 100% or more compared to the level in a control. As used herein a control may be considered a heart cell or heart organ that does not comprise or express a vector or mRNA molecule of the invention.

The "host cell" or "cell" as used herein is a heart cell. Not encompassed by the the claims, accordingly, in one example, the cell is a cardiomyocyte.

Not encompassed by the claims, in another aspect of the invention there is provided a transgenic organism where the transgenic organism expresses a vector or mRNA molecule of the invention.

Not encompassed by the claims, in a further aspect of the invention, there is provided an organism obtained or obtainable by the methods described herein.

In another aspect of the invention there is provided a composition comprising a first vector and at least a second vector, wherein the first vector comprises a nucleic acid sequence encoding an inducible myc transcription factor selected from c-myc, n-myc or I-myc operably linked to a regulatory sequence and the second vector comprises a nucleic acid sequence encoding a cyclin T1 operably linked to a regulatory sequence for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

In a further aspect of the invention there is provided a composition comprising a first modified mRNA molecule encoding a myc transcription factor selected from c-myc, I-myc or n-myc and a second modified mRNA molecule encoding a cyclin T1, wherein the modification is selected from at least one of a cap modification, a tail modification, a nucleoside modification and a untranslated region (UTR) modification for use a medicament, and wherein the first modified mRNA molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

Optionally the composition may further comprise a pharmaceutically acceptable carrier. Such pharmaceutically acceptable carriers may comprise excipients and other components which facilitate processing of the active compounds into preparations suitable for pharmaceutical administration.

In a further aspect there is provided a nucleic acid molecule or composition as described herein for use in the treatment of a condition characterised by the loss of cells, wherein the condition is selected from myocardial infarction or reduced ejection fraction of the heart.

Where the tissue concerned is the heart, regeneration of cardiomyocyte tissues is most relevant for heart attacks and reduced ejection fraction as the addition of cardiomyocytes has the potential to improve heart functionality. In reduced ejection fraction the heart tissue becomes weaker or thinner thus reducing its ability to pump blood around the body. In a heart attack (myocardial infarction) cardiomyocytes die as a result of oxygen loss to heart tissues (e.g. due to an artery blockage) and thus reduced heart function (or death) can result.

In an alternative embodiment, introduction and expression of the vector described herein leads to ectopic expression of myc and/or cyclin T1. As used herein "ectopic" expression of myc may refer to an increase in the basal levels of myc and/or cyclin T1 expression by at least 200% compared to a control.

Not encompassed in the claims, in a preferred embodiment where the myc transcription factor is fused to an ER binding domain or a modified ER binding domain as described above, the steroid may be 4-OHT. Alternatively, the steroid is tamoxifen.

Not encompassed in the claims, in a preferred embodiment, the steroid or thyroid is administered anything from one day to one or more weeks after administration of a vector of the invention. This time scale depends on the route of delivery of the steroid or thyroid, and suitable timescales would be known to the skilled person. In one embodiment, the steroid or thyroid is administered one, two, three, four, five, six or seven days after administration of the vector of the invention. Preferably in this embodiment the steroid or thyroid is administered in a vector. In an alternative embodiment, the steroid or thyroid is administered one, two, three, four, five, six or seven, eight, nine or ten weeks after administration of the vector of the invention. Preferably in this embodiment, the steroid or thyroid is administered orally.

Not encompassed in the claims, where the composition is used the first vector or first mRNA molecule may be introduced before, after or concurrently with the second vector or second mRNA molecule.

The term "variant" or "functional variant" as used throughout with reference to any of SEQ ID NOs refers to a variant nucleotide, ribonucleotide or protein sequence that retains the biological function of the full non-variant sequence. For example, a myc transcription factor retains the transcription factor activity the full non-variant myc sequence. A functional variant also comprises a variant that has sequence alterations that do not affect function, for example in non-conserved residues. Also encompassed is a variant that is substantially identical, i.e. has only some sequence variations, for example in non-conserved residues, compared to the wild type sequences as shown herein and is biologically active. Alterations in a nucleic acid sequence or ribonucleic acid sequence that result in the production of a different amino acid at a given site that does not affect the functional properties of the encoded polypeptide are well known in the art. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products.

As used in any aspect of the invention described throughout a "variant" or a "functional variant" has at least 75%, overall sequence identity to the non-variant nucleic acid, ribonucleic acid or amino acid sequence.

By "introducing" (of the vector or mRNA molecule of the invention) is meant administration of the vector or mRNA molecule.

Administration of the vector or the mRNA molecule may be accomplished by physical disturbance or generation of mRNA endocytosis by cationic carriers. Methods of physical disturbance include electroporation, gene guns, ultrasound or high-pressure injection. Methods of generation of mRNA endocytosis by cationic carriers such as self-assembled lipo-plexes or poly-plexes made up of cationic lipids or polymers including lipolexes, polyplexes, polycations and dendrimers. Suitable polycations include DEAE (diethylaminoethyl) -dextran, DOTMA/DOPE (1,2-dioeyl-3-trimethylammoniumchloride/1,2-dioleoyl-3-phodphoethanolamine), poly L-lysine, and PEI (polyethylene imine), and most preferably, DOTAP (1,2-dioleoyl-3-trimethylammonium propane).

Administration of the vectors or the mRNA molecule may be accomplished orally or parenterally. Similarly, administration of the external stimuli, such as steroid or thyroid of the invention may also be accomplished orally or parenterally. Methods of parenteral delivery include topical, intra-arterial, intramuscular, intracardiac, subcutaneous, intramedullary, intrathecal, intraventricular, intravenous, intraperitoneal, mucosal or intranasal administration. Most preferably however the vector or mRNA molecule(s) is administered by local or systemic injection. Local injection encompasses electroporation, gene gun, ultrasound and high pressure; whilst systemic injection encompasses vein injection, portal injection and artery injection. Where the target tissue is the heart, the vector or mRNA molecule(s) may be administered directly into the heart concurrently with a surgical procedure for a stent or coronary artery bypass, for example. Where the target tissue is the retina, direct administration to the retina is preferred (for example, retinal injection).

Not encompassed by the claims, , when the mRNA molecule is administered by systemic injection it is administered with cationic lipid or polymer complexes (preferably PEGylated lipid or polymer complexes) to protect the mRNA molecule against nuclease activity and thus improve stability of mRNA.

In one embodiment, the mRNA molecule may be administered in the form of a nanoparticle, more preferably a liposome-protamine-RNA or LPR. An LPR comprises modified anionic mRNA which is mixed with polycation protamine to generate an mRNA/protamine complex which is then mixed with a liposome (comprising cationic lipid DOTAP and cholesterol) to form an LPR complex which is preferably PEGylated (Wang et al, 2013; Molecular Therapy, 21(2) 358-367). The resulting LPR is small in size (<100nm) allowing for easy internalisation. Accordingly, in a further aspect of the invention there is provided a nanoparticle, preferably a LPR comprising a mRNA encoding at least one of an inducible myc transcription factor, a cyclin T1 protein as described above. In a preferred embodiment the nanoparticle is administered by local or systemic injection.

Pharmaceutical compositions for oral administration can be formulated using pharmaceutically acceptable carriers known in the art in dosages suitable for oral administration. Such carriers enable the compositions to be formulated as tablets, pills, dragees, capsules, liquids, gels, syrups, slurries, suspensions, and the like suitable for ingestion by the subject.

Pharmaceutical formulations for parenteral administration include aqueous solutions of active compounds. For injection, the pharmaceutical compositions of the invention may be formulated in aqueous solutions, preferably in physiologically compatible buffers such as Hanks's solution, Ringer's solution, or physiologically buffered saline. Aqueous suspension injections can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension can also contain suitable stabilisers or agents which increase the solubility of the compounds to allow for the preparation of highly concentrated solutions. Pharmaceutical compositions may also include adjuvants to enhance or modulate antigenicity.

For topical or nasal administration, penetrants appropriate to the particular barrier to be permeated may be used in the formulation.

As used herein, a control may be an individual, patient or cell that has not been treated with at least one nucleic acid molecule or composition of the invention.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

The invention is now described in the following non-limiting example.

### EXAMPLE I

Myc is a basic helix-loop-helix-leucine zipper (bHLH-LZ) transcription factor that binds preferentially to specific sequences in the genome, termed E-boxes (Blackwood and Eisenman, 1991), via association with its bHLH-LZ heterodimerisation partner Max (Amati *et al.,* 1993, 1992; Blackwood *et al.,* 1992). Myc functions principally as a transcriptional activator by potentiating transcription initiation via association with various cofactors such as TRRAP (Bouchard *et al.,* 2001; McMahon *et al.,* 1998) and facilitating productive transcriptional elongation by promoting RNA Polll loading and via its association with positive transcription elongation factor (P-TEFb) (De Pretis *et al.,* 2017; Eberhardy and Farnham, 2002; Kanazawa *et al.,* 2003; Rahl *et al.,* 2010). P-TEFb is comprised of Cdk9 and Cyclin T1 which are stringently regulated by various transcriptional and post transcriptional mechanisms (Jonkers and Lis, 2015; Peterlin and Price, 2006; Zhou *et al.,* 2012; Zhou and Yik, 2006), and dynamically controlled by an association with inactivation complex comprised of 7SK snRNA, Larp7, MEPCE and HEXIM (Barboric *et al.,* 2005; He *et al.,* 2008; Yik *et al.,* 2003). P-TEFb phosphorylates Serine 2 of the C-terminal Domain (CTD) of paused RNA Polll leading to productive elongation (Jonkers and Lis, 2015; Peterlin and Price, 2006; Zhou *et al.,* 2012; Zhou and Yik, 2006).

Max is also the heterodimerisation partner of the Mxd family of transcription factors that act principally as transcriptional repressors through their interactions with HDASC2, SMC3 and Sin3A (Diolaiti *et al.,* 2015). Since Mxd/Max heterodimers bind sites overlapping with Myc/Max and act as transcriptional repressors, it has been suggested that competition for Max by Myc versus Mxd proteins dictates transcriptional outputs at bound targets (Ayer *et al.,* 1993; Kretzner *et al.,* 1992).

Myc is a highly pleiotropic transcription factor which coordinates multiple transcriptional programmes involved in cell replication and differentiation, metabolism and apoptosis (Amati *et al.,* 1993; Dang, 2013; Eilers *et al.,* 1991; Evan *et al.,* 1992; Roussel *et al.,* 1991). With the development of animal models allowing for switchable ectopic Myc expression *in vivo,* it has become evident that Myc also governs diverse cell extrinsic processes required for tissue regeneration - such as angiogenesis, modulation of the local inflammatory and immune responses, invasion and migration - but in a manner that is tightly tailored to the tissue in which Myc is activated (Kortlever *et al.,* 2017; Shchors *et al.,* 2006; Sodir *et al.,* 2011). Since deregulated and elevated Myc expression is a pervasive and causal attribute of most, perhaps all, tumours, understanding how tissue-specific responses to Myc are determined at a molecular level is imperative.

To determine how tissue-specificity controls the output of Myc activity, we generated a versatile, reversibly switchable mouse model in which supraphysiological levels of Myc are expressed comparably across different tissues. Using this model, we assessed Myc target gene binding and expression in distinct tissues at just 4 hours post activation of Myc. This short time scale limits any confounding normalization issues that might arise from global RNA amplification as well as avoids indirect gene expression changes coming from secondary transcriptional activation. This unique *in vivo* model allows us to identify essential differences between tissues in which Myc drives proliferation and tissues that exhibit no such proliferative response.

### RESULTS

### The capacity of Myc to drive proliferation is tissue-restricted

To compare the responses of different tissues to the acute activation of a similar level of Myc, we generated a mouse strain in which supraphysiological levels of the switchable Myc protein c-MycER^{T2} are ubiquitously expressed from a common promoter at comparable levels across tissues. In this knock-in mouse strain, the *R26^{LSL-c-MycER}* locus (*R26^{MER}*) (Murphy *et al.,* 2008) is modified to include a *CAG* (chicken beta actin/CMV) enhancer to augment MycER^{T2} expression (Figure 8a). Such *R26^{CAG-LSL-c-MycERT2}* mice were crossed into the *Tg(Zp3-cre)93Knw* strain in which Cre is active in the growing oocyte, effectively excising the LSL stop cassette from all adult tissues in the resultant *R26^{CMER}* mice (De Vries *et al.,* 2000). Compared with the physiological level of Myc expressed in *R26*^{*MER*/+} cells (heterozygous for the *R26^{MER}* allele)(Murphy *et al.,* 2008), *R26*^{*CMER*/+} cells express around 8-fold higher levels of *MycER^{T2}* RNA (Figure 8b) in each tissue tested. *R26^{CAG-LSL-MER}* and *R26^{LSL-MER}* mice were interbred to generate an allelic series of ascending levels of MycER^{T2} expression (*R26*^{+/+}, *R26* ^{*LSL-MER*/+}, *R26*^{*LSL-MER*/*LSL-MER*}, *R26*^{*CAG-LSL-MER*/+}, *R26*^{*CAG-LSL-MER*/*LSL-MER*} and *R26*^{*CAG-LSL-MER*/*LSL-MER*}). To measure directly relative levels of MycER^{T2} in this allelic series, mouse embryo fibroblasts (MEFs) were isolated from each strain and the *LSL* stop cassette excised by infection *in vitro* with a Cre expressing Adenovirus to engage constitutive MycER^{T2} expression. Western blot analysis of cell lysates confirmed an ascending allelic series of MycER^{T2} expression with homozygous *R26^{MER}* and *R26^{CMER}* cells expressing twice the level of their respective heterozygous counterparts (Figure 1a). This allelic expression series was precisely mirrored in tissues from heterozygous and homozygous *R26^{CMER}* mice (Figure 8c).

To rule out the possibility that high levels of Myc might modulate the Rosa26 promoter - and hence elicit artefactual feedback effects - we crossed *R26^{CMER}* mice to mice carrying a *R26^{mTmG}* (*Gt(ROSA)*2*6Sor^{tm4(ACTB-tdTomato,-EGFP)Luo}*) reporter allele (Muzumdar *et al.,* 2007). Systemic activation of MycER^{T2} in *R26*^{*CA*//*mTmG*} mice had no impact on expression of either *Tomato* or *MycER^{T2}* transcripts in any tested tissues (Figure 8d). Hence, elevated *MycER^{T2}* activity does not modulate activity of the Rosa26 promoter.

We next determined whether acute activation of MycER^{T2} elicits a proliferative response in tissues of *R26*^{*CMER*/+} mice. MycER^{T2} was activated for twenty-four hours by systemic administration of tamoxifen (Wilson *et al.,* 2014) and proliferation assessed by immunohistochemical staining of Ki67, BrdU and the mitotic marker phospho-histone H3 (p-H3). We observed a consistent pattern of proliferative responses to supraphysiological Myc in tissues (Figure 1b and c) that fell into three general classes: 1. Adult tissues such as liver, lung and pancreas with normally low levels of endogenous Myc (Figure 1d) but capable of significant regeneration after injury. Such tissues showed a marked induction of proliferation upon Myc activation. 2. Adult tissues, such as kidney, heart and brain, with normally low levels of endogenous Myc and a limited capacity to regenerate, demonstrated only a negligible rise in proliferation. 3. Tissues with constitutively high proliferative rates and substantial constitutive levels of endogenous Myc (Figure 1d), such as thymus and spleen, in which activation of ectopic Myc elicited no significant additional proliferation above the already high basal level. Neither sustaining MycER^{T2} activation for three days nor increasing the expression of MycER^{T2} from 1 to 2 copies of *R26^{CMER}* changed the proliferative responses to Myc in any tissue (Figure 8e to h). Hence, different tissues harbour different inherent sensitivities to Myc-driven proliferation. Of note, quantification of the broad proliferative marker Ki67 revealed a small but significant increase in some cells within the heart in response to global Myc activation. However, these changes were confined to non-cardiomyocytes.

### Target genes and tissue specificity of Myc binding is dictated at the chromatin level

There are several plausible explanations for the failure of acute Myc activation to induce a proliferative response in tissues such as heart and brain. First, non-responsive tissues might express high levels of one or more of the Mxd proteins, which could directly antagonise Myc's transcriptional function by sequestering Max and/or competing for its binding to shared target genes. To investigate this possibility, we determined the levels of *Mxd* expression in various tissues. Although expression of *Mxd* family members varied across tissues, we saw no correlation between the extent of Myc-induced proliferation and the levels of *Mxd* transcripts. Next, we determined whether Myc is still able to bind its target genes using ChIP-sequencing analysis of Myc binding sites 4 hours after activation in exemplar responsive (liver) and non-responsive (heart) tissue. We observed a dramatic increase in gene occupancy upon MycER^{T2} activation in both tissues, from a few hundred sites in wild-type control, reflecting the low levels of endogenous Myc expression in these tissues to ~30,000 after Myc activation (Figure 2a). Hence, the failure of the heart to proliferate in response to Myc activation cannot be attributed to the inability of ectopic Myc to access its target genes. Around a quarter of Myc target binding sites were common to both tissues, with a significant overlap principally at promoter elements (Figure 2b and c). Motif analysis demonstrated significant enrichment for the E-box consensus sequences at these regions (Figure 2d) and gene ontology indicated that these overlapping promoter binding sites located to 'classic' core Myc-dependent genes implicated in control of gene expression, mRNA processing and mitotic cell cycle (Figure 2d). In addition to these common targets, however, Myc also bound discrete sets of promoter elements that differed between the two tissues (Figure 2b). These tissue-specific sites were also enriched for E-box consensus sequences but demonstrated a generally lower affinity for Myc in comparison to the common core elements (Figure 2e and f). Interestingly, these tissue-specific promoter elements were associated with genes whose expression defines each tissue (i.e. hepatocytes versus cardiomyocytes respectively - Figure 2e and f), consistent with the idea that Myc binds already active/open promoters. Myc ChIP-seq profiles, RNA Polymerase II (Polll) ChIP-seq in normal liver and heart, ATAC-seq in purified cardiomyocytes, publicly available data for chromatin marks of active transcription (H3K27 acetylation H3K4 tri-methylation and H3K4 mono-methylation) and DNAse-seq in heart and liver all concurred that Myc binding closely overlaps with transcriptionally active promoter and distal elements in each tissue. This holds true across both common and tissue-specific Myc-bound sites. For example, promoters bound in the heart but not liver are characterised by absence of active chromatin marks and Polll binding in the liver. Conversely, promoters bound by Myc only in liver lack active chromatin marks or associated Polll in heart (Figure 2g and h). To confirm Myc binding to genes implicated in the transcriptional control of cell cycle in the heart, liver and purified cardiomyocytes, we performed sub analysis of Myc binding and open chromatin marks in mitotic cell cycle gene set (GO: 0000278). Over 60% of these sites were marked as open and Myc bound (Figure 2i). These data confirm that Myc binds "open" chromatin at both promoters and distal sites (enhancers) and that the disposition of chromatin accessibility is preconfigured in a tissue-specific way. Furthermore, the data indicate that adult terminally differentiated heart retains an open chromatin architecture at sites required for Myc to drive transcriptional control of proliferation and that the regenerative capacity of the heart is lost due to transcriptional control rather than an epigenetic block.

### Myc binding to target genes does not necessarily correlate with their efficient transcription

Since Myc efficiently binds target gene DNA in both heart and liver, we investigated other potential mechanism(s) that might limit proliferative output in the non-responsive heart. We first determined the overall transcriptional output in various tissues (heart, kidney, liver, lung) of *R26*^{*CMER*/+} and *R26*^{+/+} control mice following acute Myc activation for 4 hours. Differentially expressed genes (DEGs) were called in each tissue based on the fold-change (FC) in mRNA levels between *R26*^{*CMER*/+} and *R26*^{+/+} control mice (Log2FC ≥ 0.5, q ≤ 0.05). In agreement with previous observations (Kress *et al.,* 2015; Sabò *et al.,* 2014; Walz *et al.,* 2014; Zeller *et al.,* 2006), the total number of DEGs in each tissue (Figure 3a) was much lower than the number of Myc-bound promoters, albeit with significant inclusive overlap (Figure 3b). Comparison of DEGs in the liver, lung, kidney and heart indicated considerable commonality amongst up-regulated genes (Figure 3c) implicated in core Myc-regulated processes such as ncRNA, rRNA and tRNA, metabolic processes (Figure 3d). By contrast, Myc repressed genes showed very little overlap across tissues, although gene ontology analysis did suggest that genes overlapping in 2 or more organs favoured involvement in negative regulation of cell movement, locomotion and migration (Figure 3e and f). Tissue-specific genes down-regulated by Myc showed significant overlap with genes normally expressed within that tissue, (Figure 3g) and their functions were generally associated with differentiated tissue-specific processes (Figure 3h).

Remarkably, DEG numbers also correlated well with each tissue's proliferative response to MycER^{T2} (Figure 3a, Figures 1b and c): tissues with the lowest overall transcriptional changes displayed the least proliferative responses. However, while activation of ectopic Myc induced significant transcriptional changes to a greater number of target genes in liver than in heart, quantitative analysis of the RNA showed that most of the mRNAs that were induced in liver also exhibited modest, but non-significant, responses in the heart (Figure 3i). Indeed, gene-set enrichment analysis in the heart confirmed a significant trend in the increased expression of common Myc targets, even when defined as targets commonly upregulated in response to Myc in all tissues excluding the heart (Figure 3j). Hence, the transcriptional response to activation of ectopic Myc in the heart is not absent but highly attenuated and below that required to initiate cell proliferation. This refractoriness of the heart transcriptome to Myc remained evident even after prolonged (24 hours) Myc activation or in response to very high Myc levels (homozygous *R26^{CMER}* mice).

### Productive Myc transcriptional output is limited by availability of transcriptional cofactors

The most plausible explanation for our data is that the attenuated transcriptional and biological response to Myc in heart is due to either active transcriptional inhibition and/or insufficiency of requisite transcriptional co-factors or machinery. Consistent with this, both the total and phosphorylated levels of the C terminal domain of RNA Polll (Rpb1) were much lower in non-responsive heart and kidney than in Myc-responsive tissues such as the liver and lung (Figure 4a). Since recruitment of P-TEFb is necessary for transcriptional elongation, we next assessed the expression levels of the components of P-TEFb - CDK9 and CyclinT1. Both CDK9 and CyclinT1 proteins were present at considerably lower levels in the heart and kidney compared with lung or liver, along with components of the pause factors NELF and DSIF (Figure 4a and b). Conversely, Larp7, a negative regulator of P-TEFb, was relatively more abundant in the heart and kidney, as was the 7SK short non-coding RNA that serves as the scaffold for the P-TEFb inactivation complex (Figure 4a and c). This intimated that the inability of Myc to elicit transcriptional changes in the heart arises from a tissue-specific deficiency in core transcriptional cofactors, such as P-TEFb and RNA Polll itself, which are required for efficient transcriptional initiation and elongation. To determine *in vitro* if P-TEFb was limiting Myc driven transcription we purified adult cardiomyocytes from *R26*^{*CMER*/+} mice and overexpressed *Ccnt1* with an Adeno virus carrying Ccnt1.Ectopic over-expression of Cyclin T1 in adult *R26*^{*CMER*/+} cardiomyocytes *in vitro* efficiently abrogated their normal refractoriness to Myc and permitted efficient expression of Myc target genes which were previously shown to be unresponsive in the adult heart (*Bzw2, Pinx1, Polr3d, St6* and Cdc25a) (Figure 5a to d). Together, these data support the notion that transcriptional activity of Myc is limited by P-TEFb availability.

Given the finding that Larp7 and the 7SK short non-coding RNA were relatively more abundant in the heart and kidney, this suggests that either or both of these may be alternative targets for therapy. For example, we hypothesise that a Larp7 knockdown (for example, via RNAi or CRISPR) could replace cyclin T1 overexpression. Hence, an aspect of the present invention further provides a method of therapy comprising reducing expression of Larp7 in a subject in need thereof. Suitable CRISPR guide sequences for targeting Larp7 include ATCCGGAGGAAAAAACCTC (SEQ ID NO: 30), AGTCTACTGGAGATCCAAA (SEQ ID NO: 31), AAGAAAGGCCGAATGAAAA (SEQ ID NO: 32), CTTACCTGAAGTCAGAACA (SEQ ID NO: 33), ACTTTTGATCGGGGAGCTA (SEQ ID NO: 34). These may be used in a CRISPR drop-out screen, for example as described in Tzelepis et al, 2016, Cell Reports 17, 1193-1205. The Larp7 amino acid and nucleotide sequences are given as SEQ ID NO: 35 and SEQ ID NO: 36, respectively.

### Facilitating Myc-dependent transcriptional activation in terminally differentiated cardiomyocytes in vivo restores their proliferative capacity

Expression of CDK9, CyclinT1 and RNA PolII all progressively decrease during post-natal cardiac maturation (Sano *et al*., 2002). We therefore first determined the extent of Myc-dependent transcriptional responsiveness in juvenile heart tissue. Unlike the adult heart, 15-day old juvenile hearts express levels of P-TEFb, RNA Polll comparable to those in adult liver (Figure 6a and Figure 9a and b). Acute (4 hours) MycER^{T2} activation in *R26*^{*CMER*/+} juvenile heart induced expression of a panel of common Myc transcriptional target genes (*Bzw2, Pinx1, Polr3d, St6* and *Cdc25a*) normally up-regulated by Myc in the liver but not in adult heart (Figure 5b). RNA sequencing indicated a proficient global transcriptional response in juvenile heart (1601 DEGs up-regulated and 1133 down-regulated; Figure 5c), with significant enrichment for common Myc targets (Figure 9c), defined from the overlap of genes upregulated in response to the activation of Myc in all four tissues. Following 24 hours of tamoxifen treatment, juvenile *R26*^{*CMER*/+} mice heart exhibited a marked proliferative response indicated by substantial induction of both G1 (*Cdk4*) and G2 (*Cdk1*/*Ccnb1*) cell cycle genes (Figure 9d), enrichment of pro-cell cycle progression gene sets (Figure 9e) and significant levels of mitosis (Figure 9g) specifically in troponin T-positive cardiomyocytes. The extent of Myc induced cardiomyocyte proliferation was comparable to that observed in tamoxifen-activated *R26*^{*CMER*/+} adult liver (Figure 6d). To confirm that Myc was acting to induce proliferation directly in cardiomyocytes themselves, we restricted expression of MycER^{T2} to cardiomyocytes using the using *Myh6Cre* allele (*Tg(Myh6-cre)2182Mds*/*J* (Agah *et al.,* 1997) in which Cre recombinase is driven from the *Myh6* promoter that directs expression of the Myosin heavy chain α isoform solely in cardiomyocytes. *Myh6Cre; R26*^{*LSL-CMER*/+} mice were generated and at 15 days of age given a single bolus of tamoxifen to activate MycER^{T2}. Within 48 hrs, *Myh6Cre; R26*^{*LSL-CMER*/+} hearts displayed p-H3 and Ki67 positivity specifically in cardiac troponin T and PCM1 positive cardiomyocytes, displayed Aurora B Kinase positivity at centrally located mid-bodies (Figure 6e and Figure 9f) and had doubled in size compared with *R26*^{*LSL-CMER*/+} controls (Figure 6f and g). Overt cell division was evident by a 1.8 fold increase in cardiomyocyte number without any discernible change in cardiomyocyte size (Figure 6g and h and Figure 9h).

To further confirm cytokinesis, transmission electron micrographs (TEM) from *Myh6Cre; R26*^{*LSL-CMER*/+} mice 48 hours post tamoxifen treatment were produced, 3D rendering allowed the identification of cardiomyocytes in mitosis and cytokinesis (Figure 6i). Over the longer term, transient elevated MycER^{T2} activity in 15 day old *Myh6Cre*; *R26*^{*LSL-CMER*/+} mice caused a large increase in heart size, increased p-H3 immunoreactivity, a large proportion of cardiomyocytes displaying disassembled sarcomeres and mice did not survive beyond 4 days post MycER^{T2} activation (Figure 9i).

To directly test if increasing P-TEFb activity within cardiomyocytes of the adult heart *in vivo* is permissive for efficient Myc transcriptional activation and proliferation we infected wild type, *R26*^{*CMER*/+}, *Myh6Cre; R26*^{*LSL-CMER*/+} and *R26*^{*LSL-CMER*/+} mice with an adeno-associated virus directing ectopic expression of Cyclin T1 or RFP specifically in cardiomyocytes under the troponin T promoter. After 4 weeks we confirmed viral Cyclin T1 overexpression in 34% of cardiomyocytes by immunohistochemical analysis (Figure 7a). As described previously and confirmed here, elevated expression of Ccnt1 in cardiomyocytes leads to an increase in CDK9 and phosphorylated RNA Polll (S2) (Figure 7b). Acute MycER activation in the presence of overexpressed Cyclin T1 induced the transcription of a panel of common Myc target genes normally up-regulated by Myc in the liver but not in the adult heart (Figure 7c and Figure 10a). RNA sequencing indicated a proficient global transcriptional response to the activation of Myc in the presence of ectopic Cyclin T1 in the adult heart or purified cardiomyocytes, with positive enrichment for common Myc targets and pro-cell cycle progression gene sets (Figure 10b). Positive enrichment of common Myc targets was also observed in cardiomyocytes purified from Cyclin T1 infected *Myh6Cre; R26*^{*LSL-CMER*/+} hearts verses uninfected *Myh6Cre; R26*^{*LSL-CMER*/+} hearts 4 hours post activation of Myc (Figure 7d). An increase in the rate of transcription of common Myc targets in cardiomyocytes purified from Cyclin T1 infected *Myh6Cre; R26*^{*LSL-CMER*/+} hearts in comparison to Cyclin T1 infected control *R26*^{*LSL-CMER*/+} hearts was inferred by a significant increase in the ratio of premature/mature mRNA (Figure 10c). In contrast, this ratio was unchanged for genes known to be highly expressed in the heart (Figure 10c), indicating that activation of Myc in the presence ectopic Cyclin T1 does not cause a global amplification of the pre-existing transcriptional repertoire of the adult heart.

A short pulse of MycER^{T2} activation in combination with Cyclin T1 overexpression resulted in a marked proliferative response in hearts collected at 48 hours post a single dose of tamoxifen, cardiomyocytes displaying markers of cell cycle, mitosis (Figure 7e and f, Figure 10d and e), cytokinesis (Figure 6e and Figure 10e), expression of mitosis and cytokinesis gene sets (Figure 10f), increased heart size (Figure 7g, h and Figure 10g). Furthermore, 72 hours post MycER^{T2} activation in combination with Cyclin T1 overexpression led to a further increase in heart size accompanied by an increase in cardiomyocyte number without any change in cardiomyocyte size (Figure 7g to i) or nucleation (Figure 10h). A single transient burst of MycER^{T2} activity in Cyclin T1 expressing *Myh6Cre; R26*^{*LSL-CMER*/+} adult cardiomyocytes was compatible with long-term survival, with no histological abnormalities detected at 28 days after MycER^{T2}activation (Figure 10i). Collectively, these data illustrate that Myc transcriptional activity can be facilitated in adult cardiomyoctes by co-activation of the transcriptional co-factor P-TEFb thus enabling adult heart tissue to re-enter the cell cycle.

Furthermore, constitutively elevated levels of P-TEFb have been noted in the hearts of *TetO-HRas; Myh6-tTA (Tg(tetO-HRAS)65Lc*/*Nci); Myh6tTA (Tg(Myh6-tTA)6Smbf*/*Jm)* mice, which constitutively express the oncogenic HRas*^{G12V}* protein in myocardium from weaning age. To test the prediction that such augmented basal levels of p-TEFb render heart tissue Myc responsive, these mice were crossed into the *R26*^{*CMER*/+} switchable Myc background to generate *TetO-HRas; Myh6-tTA; R26*^{*CMER*/+} mice. Doxycycline was withdrawn from *TetO-HRas; Myh6-tTA; R₂₆*^{*CMER*/+} animals at weaning to induce expression of HRas*^{G12V}* and at adulthood animals exhibited the reported elevated levels of CyclinT1, RNA Polll and phospho-RNA Polll (S2) (Figure 11i). Activation of MycER^{T2} (for 24 hours) in these animals induced multiple cell cycle genes (*Cdk4, Ccnd1, Cdk1, Ccnb1*) (Figure 11e) not induced by Myc in control *TetO-HRas; Myh6-tTA; R26*^{+/+} hearts and a marked proliferative response, mitosis (17.8% of cardiomyocytes phospho-histone H3 positive) and cytokinesis (Figure 11a to d). We also observed Myc-dependent modulation of a broad panel of Myc target genes that were not affected in control adult heart (Figure 11f). RNA sequencing confirmed increased transcription (976 DEGs up-regulated and 663 down regulated; Figure 11g) of Myc target genes whose identities overlapped with genes induced by MycER^{T2} in adult liver, lung and kidney. To determine the extent that the transcriptional changes in *TetO-HRas; Myh6-tTA; R26*^{*CMER*/+} and *TetO-HRas; Myh6-tTA; R26*^{+/+} hearts revert to a neonatal (P1) proliferative state we compared the transcriptional profile to cardiomyocytes isolated from surgically infarcted hearts at different stages of development. Our unbiased approach revealed that the P1 neonatal regeneration signature genes were markedly enriched and correlated most closely with *TetO-HRas; Myh6-tTA; R26*^{*CMER*/+} hearts but not with cardiomyocytes isolated from adult infarcted hearts or with control *TetO-HRas; Myh6-tTA; R26*^{+/+} (no Myc) hearts (Figure 11h). Collectively, these data illustrate that Myc transcription can be driven in adult cardiomyoctes by co-expression of the transcriptional co-factor P-TEFb thus enabling adult heart tissue to re-enter the cell cycle.

### DISCUSSION

Myc is widely acknowledged to play a pivotal coordinating role in the transcriptional control of somatic cell proliferation and tissue regeneration and its activity is deregulated and typically elevated in the majority of aggressive cancers (Amati *et al.,* 1993; Eilers *et al*., 1991; Roussel *et al.,* 1991). Past studies have largely focused on identifying Myc transcriptional programmes that are common across cell types. However, Myc exhibits great cell type variability, both with respect to how efficiently it can drive cell proliferation and the phenotypes its activities elicit in different tissues (Kortlever *et al.,* 2017; Pelengaris *et al.,* 2002, 1999; Shchors *et al.,* 2006). In addition, previous studies have been limited by an inevitable focus on the longer-term outcomes of Myc activation, which makes it very difficult to parse the direct impact of Myc from the indirect pleiotropic consequences of the primary programmes that Myc engages across different tissues. To contrast and compare the direct, initial impact of Myc activation at oncogenic levels on different tissues, we developed a unique mouse model whereby the Myc encoding sequence is inserted downstream of the endogenous *Rosa26* promoter, which is expressed at comparable levels across all tested tissues. This model is enhanced in several ways: first, by the option of a variant, augmented *Rosa26* promoter incorporating additional CAG (*CMV* and *β-actin* promoter elements) that increases the expression level of Myc to supraphysiological levels frequent in many cancers, thereby establishing an ascending allelic series (*R26*^{*MER*/+}, *R26*^{*MER*/*MER*}, *R26*^{*CMER*/+}, *R26*^{*CMER*/*MER*}, *R26*^{*CMER*/*CMER*}) of increasing Myc levels with which to ascertain the relative oncogenic roles of Myc deregulation versus over-expression. Unlike most tissue-specific promoters used to drive transgenic oncogenes, neither *Rosa26* nor *RosaCAG* promoters are repressed by Myc activation, obviating a major confounding issue in most conventional transgenic models. Second, replacing Myc with the well-validated, reversibly switchable 4-OHT-dependent MycER^{T2} variant allows the analysis of the immediate and direct impact of Myc on its target genome through rapid and synchronous MycER^{T2} activation by 4-OHT. Third, inclusion of tissue-specific regulation via a Cre recombinase-excisable transcriptional STOP element provides the option for tissue restricted MycER^{T2} expression.

In our current studies, we have used the egg-specific ZP3 promoter to engage ubiquitous expression of MycER^{T2} in *R26^{CMER}* mice and used this to compare the direct impacts of oncogenic Myc activity in different adult mouse tissues. Using proliferation as the phenotypic signature of Myc output, we identified three general classes of response to activation of oncogenic levels of Myc: tissues that proliferated in response to MycER^{T2} activation; tissues that did not; and tissues that exhibited innately high endogenous Myc and proliferative indices. These innate tissue responses were maintained even when higher levels of Myc were expressed over an extended period of time. Organs with inherently high levels of cell turnover supplied from a dedicated stem cell compartment, such as the intestine and hematopoietic system, exhibit constitutively high endogenous Myc levels and activation of ectopic MycER^{T2} only modestly augments their already high proliferation ability. Tissues such as the liver, lung and pancreas are normally quiescent, have low endogenous levels of Myc, but capable of facultative regeneration in response to injury (Baddour *et al.,* 2012). Activation of ectopic MycER^{T2} in such tissues induces a profound proliferative response. Finally, adult tissues such as the brain and heart have low endogenous levels of Myc, have little or no regenerative potential, regenerate poorly or not at all after injury and generally form scar tissue, and show no proliferative response to MycER^{T2} activation. The correlation between proliferative response to ectopic Myc in these tissues (Figure 1b and c) and the innate regenerative potential of each tissue (reviewed in Kotton and Morrisey, 2014) is striking and suggests an underlying mechanistic connection.

Myc shares both its obligate dimerization partner Max and its canonical E-box DNA binding specificity with the Mxd family of transcriptional repressor proteins and it has been suggested that Myc and Mxd compete for Max and thereby operate within a mutually antagonistic network (Ayer *et al.,* 1993; Kretzner *et al.,* 1992). However, we found no correlation across multiple tissues between levels of *Mxd* expression and proliferative response to Myc - indeed, Mxd proteins are more highly expressed in some Myc-responsive tissues cells (pancreas, liver, and lung). Furthermore, our data indicating high levels of Mxd expression in proliferating tissues such as spleen and thymus shows that, as previously suggested (Baudino and Cleveland, 2001), Mxd proteins can co-exist intracellularly with Myc without necessarily antagonising Myc action. The different patterns of chromatin binding that Max-Mxd and Myc-Max dimers exhibit, possibly arising from distinct residues in the Mxd versus Myc DNA-binding basic regions, also suggest that the two classes of transcription factor operate largely independently of each other (O'Hagan *et al.,* 2000; Solomon *et al.,* 1993). These data confirm that, as previously suggested (Baudino and Cleveland, 2001), Mxd proteins in various organs do not significantly antagonise Myc activity.

ChlP-seq analysis 4 hours post MycER^{T2} activation in exemplar Myc-responsive versus non-responsive tissues (respectively, liver and heart) showed a dramatic increase in gene occupancy in both tissues. Hence, tissue non-responsiveness to Myc is not due to failure of ectopic Myc to access its chromatin targets. Indeed, we found striking similarities between the numbers of sites to which Myc is bound across the different tissues analysed, underscoring that the phenotypic outcome effected by Myc is not solely dictated at the level of recruitment to chromatin.

The significant overlap in Myc-bound promoter regions across both the liver and heart affirms the widely held notion that Myc binds a set of target genes common to multiple cell types and implicated in governing core processes required for cell growth and cell cycle progression. However, Myc also binds a set of promoter elements specific to each tissue, indicating that the Myc-dependent cistrome is cell type-specific. We also note that this tissue-specific Myc binding repertoire is characterised by the presence of chromatin marks indicative of open chromatin, in keeping with the findings of others. Interestingly as early as 4-hours post Myc activation, we clearly see a significant number of genes to be transcriptionally repressed. These repressed genes principally articulate differentiated cell-type functions and show very little overlap between tissue types. Moreover, around a third (34%) of the repressed genes appear to be indirect targets since Myc is not detected at their promoter elements (Figure 3b). Suppression of differentiation-specific genes by oncogenic Myc has been widely observed irrespective of cell or tissue type and is an example of the robust inverse generic relationship between cell proliferation and differentiation (Ruijtenberg and van den Heuvel, 2016).

A simple hypothetical explanation for the pervasive suppression of differentiation by Myc is that certain components of transcriptional machinery, such as P-TEFb or even total levels of RBP1, available for loading onto promoters are limiting, in which case repression of differentiation-specific genes is simply a consequence of their redeployment to Myc target genes, a mechanism recently proposed in 3T3 fibroblasts (De Pretis *et al.,* 2017). Analogous scarcities in transcriptional machinery could also explain why some tissues fail to respond transcriptionally to Myc. Myc has been shown to modify both RNA Polll loading and its phosphorylation by P-TEFb (De Pretis *et al.,* 2017; Eberhardy and Farnham, 2002; Jonkers and Lis, 2015; Kanazawa *et al.,* 2003; Rahl *et al.,* 2010; Shao and Zeitlinger, 2017). Prompted by the recent observation that MYC overexpression is the key molecular determinant dictating sensitivity to CDK9 inhibition in hepatocellular carcinoma (Huang *et al.,* 2014), we showed that responsiveness of individual tissues to Myc correlates with the levels of expression of the basal transcription factors P-TEFb and RNA Polll, the pause factors, DSIF and NELF, and, inversely, with the expression of the P-TEFb repression complex.

Adult mammalian heart is a prototypical, terminally-differentiated, Myc non-responsive tissue in which Cyclin T1 is known to be a limiting transcriptional regulator whose chronic ectopic overexpression elicits promiscuous RNA polymerase activity and cardiac hypertrophy. While P1 neonatal heart retains proliferative potential, this rapidly declines after birth and is largely lost by P7. This decline in neonatal cardiac proliferative potential correlates with down-regulation of multiple genes involved in cell cycle including endogenous Myc, which is high at P1 but significantly reduced by P5 (Figure 9a). Although changes in microRNAs, modulation of the Hippo pathway, and wholesale metabolic switching from glycolysis to oxidative phosphorylation have all been implicated, the mechanism underlying post-natal cardiac maturation and loss of regenerative potential remains unknown.

Interestingly, both the neonatal mouse heart and the zebrafish larval heart express high levels of P-TEFb. Whereas, the zebrafish adult heart retains 70% of the larval levels of P-TEFb and the ability to regenerate throughout development, P-TEFb levels in the adult mouse heart drop to 15%. Little is known regarding the regulation of P-TEFb during mammalian cardiac development. Levels of Cyclin T1 are regulated at the transcriptional and post-transcriptional level, with mitogens and cytokine signaling known to increase Cyclin T1 protein stability. We and others have shown that the level of Cyclin T1 is the key factor regulating the level and activity of P-TEFb within cardiomyocytes. As shown in Figure 11, oncogenic Ras signaling increases Cyclin T1 in the heart, leading to cardiac hypertrophy. It is tempting to speculate that the noted oncogenic cooperation between Ras and Myc may, in part, rely on a Ras-dependent increase in P-TEFb, promoting Myc-driven transcription.

Re-establishing regenerative proliferation in the adult heart has proven very difficult. Inhibition of Hippo signalling, enforced expression of both G1 (CDK4 and Ccnd1) and G2/M (CDK1 and Ccnb1) factors, and hypoxia all induce modest regeneration but at levels well below those seen in P1 neonatal heart. While the adult heart is refractory to Myc induced proliferation, enforced ectopic Myc expression extended the window of neonatal cardiomyocyte proliferation, up to around P15, a time-point that correlates with high endogenous cardiac P-TEFb expression. The large increase in cardiomyocyte number and Aurora B localization to centrally located mid-bodies suggests that cardiomyocytes isolated from juvenile mice can progress through the cell cycle and many can complete cytokinesis. Remarkably, we show that an increase in the level of P-TEFb activity in the adult heart is sufficient to support ectopic Myc dependent transcription, proliferation and cytokinesis of cardiomyocytes *in vivo,* resulting in increased heart size and cardiomyocyte number within a very short timeframe. Importantly this transient wave of proliferation induced in a smaller fraction of total cardiomyocytes than in juvenile mice is compatible with long-term survival. It will be of great interest to determine if this genetic combination will prove beneficial in models of cardiac injury.

Despite the significant changes in regenerative capacity and responsiveness to Myc that attend cardiac development from embryonic stem cells through mesoderm to precursor cardiomyocyte to cardiomyocyte, the chromatin status at promoters for genes involved in basic cellular function, including cell cycle regulation, show very little variation (Heintzman *et al.,* 2009; Wamstad *et al.,* 2012). Similarly, the Myc binding sites of the adult liver (proliferative) and the adult heart (non-proliferative) exhibit significant overlap, indicating that even after its maturation, adult, terminally-differentiated heart retains an open chromatin architecture at sites required to drive transcriptional control of proliferation. Hence, if Myc could be induced by mitogenic stimulus in the heart, cardiac epigenome architecture does not preclude heart regenerative capacity: rather, it is the inability of Myc to drive transcriptional output from regenerative target genes (due to lack of the P-TEFb/RNA Pol II complex) that thwarts cardiomyocyte proliferation.

Overall, our data indicate that tissue regenerative capacity is tightly linked to the capacity of that tissue to respond to Myc and that tissue Myc responsiveness is governed principally by availability of key components of the core transcriptional machinery, which Myc co-opts to drive its regenerative biological output. However, in addition to its core target genes, which are common across tissues, a significant number of its targets are expressed in a tissue-specific manner. By contrast, tissue-specific accessibility of target genes to Myc appears to be dictated principally by hard-wired, tissue-specific epigenome organization. Our current study focuses in the main on liver and heart as, respectively, prototypical examples of regenerative and Myc responsive versus non-regenerative and Myc-unresponsive tissues.

### Example II

The adult mouse brain displays many of the same non-regenerative features as the adult mouse heart, such as increased cell size, a switch from glycolysis to oxidative phosphorylation and increased mitochondrial mass (Agathocleous *et al.,* 2012; Zheng *et* al., 2016). Likewise, the nephron cells of the kidney have a huge metabolic demand, second only to the heart. We determined the level of Myc and Ccnt1/Cdk9 proteins throughout the mouse post-natal development. Similar to the heart, the level of all 3 of these proteins drop with age to adulthood, indicating P-TEFb levels may be limiting Myc driven transcription in adulthood (Figure 12a). MycER was activated in 10 day old mice with a single injection of tamoxifen, immunohistochemical staining showed some increased positivity of p-H3 in MycER expressing brain and kidney tissues (Figure 12b). Therefore, enforced ectopic Myc expression can extended the window of neonatal proliferation, up to around P15, a time-point that correlates with high endogenous cardiac P-TEFb expression, in both the brain and kidney.

### REFERENCES

Agah, R. et al., 1997. Gene recombination in postmitotic cells: Targeted expression of Cre recombinase provokes cardiac-restricted, site-specific rearrangement in adult ventricular muscle in vivo. J. Clin. Invest.
Agathocleous, M. et al. Metabolic differentiation in the embryonic retina. Nat. Cell Biol. 14, (2012).
Akalin, A. et al., 2015. Genomation: A toolkit to summarize, annotate and visualize genomic intervals. Bioinformatics 31, 1127-1129.
Amati, B. et al., 1992. Transcriptional activation by the human c-Myc oncoprotein in yeast requires interaction with Max. Nature 359, 423-426.
Amati, B. et al., 1993. The c-Myc protein induces cell cycle progression and apoptosis through dimerization with Max. EMBO J. 12, 5083-5087.
Anders, S. et al., 2015. HTSeq-A Python framework to work with high-throughput sequencing data. Bioinformatics 31, 166-169.
Ayer, D.E. et al., 1993. Mad: A heterodimeric partner for Max that antagonizes Myc transcriptional activity. Cell 72, 211-222.
Baddour, J.A. et al., 2012. Organ repair and regeneration: An overview. Birth Defects Res. Part C - Embryo Today Rev.
Bailey, T.L. et al., 2012. Inferring direct DNA binding from ChIP-seq. Nucleic Acids Res. 40, 1-10.
Barboric, M. et al., 2005. Interplay between 7SK snRNA and oppositely charged regions in HEXIM1 direct the inhibition. EMBO J. 24, 4291-4303.
Baudino, T.A. et al., 2001. The Max Network Gone Mad. Mol. Cell. Biol. 21, 691-702. Bianchi, V. et al., 2016. Integrated Systems for NGS Data Management and Analysis: Open Issues and Available Solutions. Front. Genet. 7, 1-8.
Blackwood, E.M, et al.,1991. Max: A helix-loop-helix zipper protein that forms a sequence-specific DNA-binding complex with Myc. Science (80-. ). 251, 1211-1217.
Blackwood, E.M. et al., 1992. Myc and Max associate in vivo. Genes Dev. 6, 71-80.
Blecher-Gonen, R. et al.,2013. High-throughput chromatin immunoprecipitation for genome-wide mapping of in vivo protein-DNA interactions and epigenomic states. Nat. Protoc. 8, 539-554.
Bolger, A.M. et al., 2014. Trimmomatic: A flexible trimmer for Illumina sequence data. Bioinformatics 30, 2114-2120.
Bouchard, C. et al., 2001. Regulation of cyclin D2 gene expression by the Myc / Max / Mad network : Myc-dependent TRRAP recruitment and histone acetylation at the cyclin D2 promoter. Genes Dev. 15, 2042-2047.
Chen, E.Y. et al., 2013. Enrichr: Interactive and collaborative HTML5 gene list enrichment analysis tool. BMC Bioinformatics 14.
Dang, C. V, 2013. MYC, metabolism, cell growth, and tumorigenesis. Cold Spring Harb. Perspect. Med. 3, a014217.
De Pretis, S. et al. Gene expression INSPEcT : a computational tool to infer mRNA synthesis , processing and degradation dynamics from RNA- and 4sU-seq time course experiments. Bioinformatics 31, 2829-2835 (2015).
De Pretis, S. et al., 2017. Integrative analysis of RNA polymerase II and transcriptional dynamics upon MYC activation. Genome Res. 10, 1658-1664.
De Vries, W.N. et al., 2000. Expression of Cre Recombinase in Mouse Oocytes: A Means to Study Maternal Effect Genes. Genesis 26, 110-112.
Diolaiti, D. et al., 2015. Functional interactions among members of the MAX and MLX transcriptional network during oncogenesis. Biochim. Biophys. Acta - Gene Regul. Mech. 1849, 484-500.
Dobin, A. et al., 2013. STAR: Ultrafast universal RNA-seq aligner. Bioinformatics 29, 15-21.
Dunham, I.,et al. An integrated encyclopedia of DNA elements in the human genome. Nature 489, 57-74.
Eberhardy, S.R. et al., 2002. Myc recruits P-TEFb to mediate the final step in the transcriptional activation of the cad promoter. J. Biol. Chem. 277, 40156-40162.
Eilers, M. et al., 1991. The MYC protein activates transcription of the alpha-prothymosin gene. EMBO J. 10, 133-141.
Evan, G.I. et al., 1992. Induction of apoptosis in fibroblasts by c-myc protein. Cell 69, 119-128.
Furlan, M. et al. Genome-wide dynamics of RNA synthesis , processing and degradation without RNA metabolic labeling. bioRxiv Prepr. 1-26 (2019).
Gamper, I. et al., 2017. Determination of the physiological and pathological roles of E2F3 in adult tissues. Sci. Rep. 7.
Gentleman, R. et al., 2004. Bioconductor: open software development for computational biology and bioinformatics. Genome Biol. 5, R80.
Gilsbach, R. et al., 2014. Dynamic DNA methylation orchestrates cardiomyocyte development, maturation and disease. Nat. Commun. 5, 5288.
He, N. et al., 2008. Article A La-Related Protein Modulates 7SK snRNP Integrity to Suppress P-TEFb-Dependent Transcriptional Elongation and Tumorigenesis. Mol. Cell 29, 588-599.
Heintzman, N.D. et al., 2009. Histone modifications at human enhancers reflect global cell-type-specific gene expression. Nature 459, 108-112.
Huang, C.H. et al., 2014. CDK9-mediated transcription elongation is required for MYC addiction in hepatocellular carcinoma. Genes Dev. 28, 1800-1814.
Huang, D.W. et al., 2009. Systematic and integrative analysis of large gene lists using DAVID bioinformatics resources. Nat. Protoc. 4, 44-57.
Jonkers, I., Lis, J.T., 2015. Getting up to speed with transcription elongation by RNA polymerase II. Nat. Rev. Mol. Cell Biol.
Kanazawa, S, et al., 2003. c-Myc recruits P-TEFb for transcription, cellular proliferation and apoptosis. Oncogene 22, 5707-5711.
Kishore, K. et al., 2015. methylPipe and compEpiTools: A suite of R packages for the integrative analysis of epigenomics data. BMC Bioinformatics 16, 1-11.
Kondrat, J et al. Synthesis of modified mRNA for myocardial delivery; Cardiac Gene Therapy, Vol. 152. p 127-137; 2017.
Kortlever, R.M. et al., 2017. Myc Cooperates with Ras by Programming Inflammation and Immune Suppression. Cell 171, 1301-1315.e14.
Kotton, D.N., Morrisey, E.E., 2014. Lung regeneration: Mechanisms, applications and emerging stem cell populations. Nat. Med.
Kress, T.R. et al., 2016. Identification of MYC-dependent transcriptional programs in oncogene-addicted liver tumors. Cancer Res. 76, 3463-3472.
Kress, T.R. et al., 2015. MYC: Connecting selective transcriptional control to global RNA production. Nat. Rev. Cancer.
Kretzner, L. et al., 1992. Transcriptional activities of the Myc and Max proteins in mammalian cells. Curr. Top. Microbiol. Immunol. 182, 435-443.
Leach, J.P, et al., 2017. Hippo pathway deficiency reverses systolic heart failure after infarction. Nature 550, 260-264.
Li, B., Dewey, C.N., 2011. RSEM: Accurate transcript quantification from RNA-Seq data with or without a reference genome. BMC Bioinformatics 12.
Li, F. et al.,1996. Rapid transition of cardiac myocytes from hyperplasia to hypertrophy during postnatal development. J. Mol. Cell. Cardiol. 28, 1737-1746.
Li, H., 2013. Aligning sequence reads, clone sequences and assembly contigs with BWA-MEM 00, 1-3.
Love, M.I. et al.,2014. Moderated estimation of fold change and dispersion for RNA-seq data with DESeq2. Genome Biol. 15, 1-21.
Martin, M., 2011. Cutadapt removes adapter sequences from high-throughput sequencing reads. EMBnet.journal 17, 10.
McMahon, S.B. et al., 1998. The novel ATM-related protein TRRAP is an essential cofactor for the c- Myc and E2F oncoproteins. Cell 94, 363-374.
Mohamed, T., et al., 2018. Regulation of Cell Cycle to Stimulate Adult Cardiomyocyte Proliferation and Cardiac Regeneration. Cell 173, 104-116.
Murphy, D.J., et al., 2008. Distinct Thresholds Govern Myc's Biological Output In Vivo. Cancer Cell 14, 447-457.
Muzumdar, M.D., et al., 2007. A global double-fluorescent cre reporter mouse. Genesis 45, 593-605.
Nakada, Y., et al., 2017. Hypoxia induces heart regeneration in adult mice. Nature 541, 222-227.
O'Hagan, R.C., et al., 2000. Gene-target recognition among members of the Myc superfamily and implications for oncogenesis. Nat. Genet. 24, 113-119.
Oliveros, J.C., 2007. Venny. An interactive tool for comparing lists with Venn's diagrams. Pelengaris, S., et al., 2002. Suppression of Myc-induced apoptosis in β cells exposes multiple oncogenic properties of Myc and triggers carcinogenic progression. Cell 109, 321-334.
Pelengaris, S., et al., 1999. Reversible activation of c-Myc in skin: Induction of a complex neoplastic phenotype by a single oncogenic lesion. Mol. Cell 3, 565-577.
Peterlin, B.M., Price, D.H., 2006. Controlling the Elongation Phase of Transcription with P-TEFb. Mol. Cell 23, 297-305.
Porrello, E.R., et al., 2011. Transient Regenerative Potential of the Neonatal Mouse Heart. Science (80-. ). 331, 1078-1080.
Porrello, E.R., Olson, E.N., 2014. A neonatal blueprint for cardiac regeneration. Stem Cell Res. 13, 556-570.
Rahl, P.B., et al., 2010. C-Myc regulates transcriptional pause release. Cell 141, 432-445.
Robinson, M.D., et al., 2009. edgeR: A Bioconductor package for differential expression analysis of digital gene expression data. Bioinformatics 26, 139-140.
Robinson, M.D., Oshlack, A., 2010. A scaling normalization method for differential expression analysis of RNA-seq data. Genome Biol. 11.
Roussel, M.F., et al., 1991. Myc rescue of a mutant CSF-1 receptor impaired in mitogenic signalling. Lett. To Nat. 353, 361-363.
Ruijtenberg, S., van den Heuvel, S., 2016. Coordinating cell proliferation and differentiation: Antagonism between cell cycle regulators and cell type-specific gene expression. Cell Cycle 15, 196-212.
Sabò, A., et al., 2014. Selective transcriptional regulation by Myc in cellular growth control and lymphomagenesis. Nature.
Sano, M., et al., 2002. Activation and function of cyclin T-Cdk9 (positive transcription elongation factor-b) in cardiac muscle-cell hypertrophy. Nat. Med. 8, 1310-1317.
Shao, W., Zeitlinger, J., 2017. Paused RNA polymerase II inhibits new transcriptional initiation. Nat. Genet.
Shchors, K., et al., 2006. The Myc-dependent angiogenic switch in tumors is mediated by interleukin 1β. Genes Dev. 20, 2527-2538.
Sodir, N.M., et al., 2011. Endogenous Myc maintains the tumor microenvironment. Genes Dev. 25, 907-916.
Solomon, D.L.C., et al., 1993. Distinct DNA binding preferences for the c-Myc/Max and Max/Max dimers. Nucleic Acids Res. 21, 5372-5376.
Walz, S., et al., 2014. Activation and repression by oncogenic MYC shape tumour-specific gene expression profiles. Nature 511, 483-487.
Wamstad, J.A., et al., 2012. Dynamic and coordinated epigenetic regulation of developmental transitions in the cardiac lineage. Cell 151, 206-220.
Wei, B.-R., et al., 2011. TRE-RasG12V 2011 Comp Med. Comp. Med. 61, 109-118.
Wilson, C.H., et al., 2014. The kinetics of ER fusion protein activation in vivo. Oncogene 33, 4877-4880.
Yik, J.H.N., et al., 2003. Inhibition of P-TEFb ( CDK9 / Cyclin T ) Kinase and RNA Polymerase II Transcription by the Coordinated Actions of HEXIM1 and 7SK snRNA 12, 971-982.
Zeller, K.I., et al., 2006. Global mapping of c-Myc binding sites and target gene networks in human B cells. Proc. Natl. Acad. Sci. U. S. A. 103, 17834-9.
Zhang, Y., et al., 2008. Model-based analysis of ChIP-Seq (MACS). Genome Biol. 9, R137.
Zhou, Q., et al., 2012. RNA Polymerase II Elongation Control. Annu. Rev. Biochem 81, 119-43.
Zhou, Q., Yik, J.H.N., 2006. The Yin and Yang of P-TEFb Regulation: Implications for Human Immunodeficiency Virus Gene Expression and Global Control of Cell Growth and Differentiation. Microbiol. Mol. Biol. Rev.
Zheng, X. et al. Metabolic reprogramming during neuronal differentiation from aerobic glycolysis to neuronal oxidative phosphorylation. Elife 1-25 (2016). doi:10.7554/eLife.13374

### SEQUENCE LISTING

SEQ ID NO: 1: MycER^{T2} amino acid sequence 
Met P L N V S F T N R N Y D L D Y D S V Q P Y F Y C D E E E N F Y Q Q Q Q S E
L Q P P A P S E D I W K K F E L L P T P P L S P S R R S G L C S P S Y V A V T P F S
L R G D N D G G G G S F S T A D Q L E Met V T E L L G G D Met V N Q S F I C D P
D D E T F I K N I I I Q D C Met W S G F S A A A K L V S E K L A S Y Q A A R K D S G
S P N P A R G H S V C S T S S L Y L Q D L S A A A S E C I D P S V V F P Y P L N D
S S S P K S C A S Q D S S A F S P S S D S L L S S T E S S P Q G S P E P L V L H E
E T P P T T S S D S E E E Q E D E E E I D V V S V E K R Q A P G K R S E S G S P S
A G G H S K P P H S P L V L K R C H V S T H Q H N Y A A P P S T R K D Y P A A K
R V K L D S V R V L R Q I S N N R K C T S P R S S D T E E N V K R R T H N V L E R
Q R R N E L K R S F F A L R D Q I P E L E N N E K A P K V V I L K K A T A Y I L S V
Q A E E Q K L I S E E D L L R K R R E Q L K H K L E Q L R D P G S A G D Met R A A
N L W P S P L Met I K R S K K N S L A L S L T A D Q Met V S A L L D A E P P I L Y S
E Y D P T R P F S E A S Met Met G L L T N L A D R E L V H Met I N W A K R V P G F
V D L T L H D Q V H L L E C A W L E I L Met I G L V W R S Met E H P V K L L F A P N
L L L D R N Q G K C V E G Met V E I F D Met L L A T S S R F R Met Met N L Q G E E
F V C L K S I I L L N S G V Y T F L S S T L K S L E E K D H I H R V L D K I T D T L I H
L Met A K A G L T L Q Q H Q R L A Q L L L I L S H I R H Met S N K G Met E H L Y
S Met K C K N V V P L Y D L L L E A A D A H R L H A P T S R G G A S V E E T D Q S
H L A T A G S T S S H S L Q K Y Y I T G E A E G F P A T V Stop Q
SEQ ID NO: 2: MycER^{T2} nucleic acid sequence 
ATGCCCCTCAACGTTAGCTTCACCAACAGGAACTATGACCTCGACTACGACTCGG
TGCAGCCGTATTTCTACTGCGACGAGGAGGAGAACTTCTACCAGCAGCAGCAGCA
GAGCGAGCTGCAGCCCCCGGCGCCCAGCGAGGATATCTGGAAGAAATTCGAGCT
GCTGCCCACCCCGCCCCTGTCCCCTAGCCGCCGCTCCGGGCTCTGCTCGCCCTC
CTACGTTGCGGTCACACCCTTCTCCCTTCGGGGAGACAACGACGGCGGTGGCGG
GAGCTTCTCCACGGCCGACCAGCTGGAGATGGTGACCGAGCTGCTGGGAGGAG
ACATGGTGAACCAGAGTTTCATCTGCGACCCGGACGACGAGACCTTCATCAAAAA
CATCATCATCCAGGACTGTATGTGGAGCGGCTTCTCGGCCGCCGCCAAGCTCGT
CTCAGAGAAGCTGGCCTCCTACCAGGCTGCGCGCAAAGACAGCGGCAGCCCGAA
CCCCGCCCGCGGCCACAGCGTCTGCTCCACCTCCAGCTTGTACCTGCAGGATCT
GAGCGCCGCCGCCTCAGAGTGCATCGACCCCTCGGTGGTCTTCCCCTACCCTCT
 
CAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCC
CGAGCCCCTGGTGCTCCATGAGGAGACACCGCCCACCACCAGCAGCGACTCTGA
GGAGGAACAAGAAGATGAGGAAGAAATCGATGTTGTTTCTGTGGAAAAGAGGCAG
GCTCCTGGCAAAAGGTCAGAGTCTGGATCACCTTCTGCTGGAGGCCACAGCAAA
CCTCCTCACAGCCCACTGGTCCTCAAGAGGTGCCACGTCTCCACACATCAGCACA
ACTACGCAGCGCCTCCCTCCACTCGGAAGGACTATCCTGCTGCCAAGAGGGTCA
AGTTGGACAGTGTCAGAGTCCTGAGACAGATCAGCAACAACCGAAAATGCACCAG
CCCCAGGTCCTCGGACACCGAGGAGAATGTCAAGAGGCGAACACACAACGTCTT
GGAGCGCCAGAGGAGGAACGAGCTAAAACGGAGCTTTTTTGCCCTGCGTGACCA
GATCCCGGAGTTGGAAAACAATGAAAAGGCCCCCAAGGTAGTTATCCTTAAAAAA
GCCACAGCATACATCCTGTCCGTCCAAGCAGAGGAGCAAAAGCTCATTTCTGAAG
AGGACTTGTTGCGGAAACGACGAGAACAGTTGAAACACAAACTTGAACAGCTACG
GGATCCGGGGTCTGCTGGAGACATGAGAGCTGCCAACCTTTGGCCAAGCCCGCT
CATGATCAAACGCTCTAAGAAGAACAGCCTGGCCTTGTCCCTGACGGCCGACCAG
ATGGTCAGTGCCTTGTTGGATGCTGAGCCCCCCATACTCTATTCCGAGTATGATC
CTACCAGACCCTTCAGTGAAGCTTCGATGATGGGCTTACTGACCAACCTGGCAGA
CAGGGAGCTGGTTCACATGATCAACTGGGCGAAGAGGGTGCCAGGCTTTGTGGA
TTTGACCCTCCATGATCAGGTCCACCTTCTAGAATGTGCCTGGCTAGAGATCCTG
ATGATTGGTCTCGTCTGGCGCTCCATGGAGCACCCAGTGAAGCTACTGTTTGCTC
CTAACTTGCTCTTGGACAGGAACCAGGGAAAATGTGTAGAGGGCATGGTGGAGAT
CTTCGACATGCTGCTGGCTACATCATCTCGGTTCCGCATGATGAATCTGCAGGGA
GAGGAGTTTGTGTGCCTCAAATCTATTATTTTGCTTAATTCTGGAGTGTACACATTT
CTGTCCAGCACCCTGAAGTCTCTGGAAGAGAAGGACCATATCCACCGAGTCCTGG
ACAAGATCACAGACACTTTGATCCACCTGATGGCCAAGGCAGGCCTGACCCTGCA
GCAGCAGCACCAGCGGCTGGCCCAGCTCCTCCTCATCCTCTCCCACATCAGGCA
CATGAGTAACAAAGGCATGGAGCATCTGTACAGCATGAAGTGCAAGAACGTGGTG
CCCCTCTATGACCTGCTGCTGGAGGCGGCGGACGCCCACCGCCTACATGCGCCC
ACTAGCCGTGGAGGGGCATCCGTGGAGGAGACGGACCAAAGCCACTTGGCCACT
GCGGGCTCTACTTCATCGCATTCCTTGCAAAAGTATTACATCACGGGGGAGGCAG
AGGGTTTCCCTGCCACAGTCTGACAATT
SEQ ID NO: 3: CAG promoter 
CGGCCGCCCACCCTCCCCTTCCTCTGGGGGAGTCGTTTTACCCGCCGCCGGCCG
GGCCTCGTCGTCTGATTGGCTCTCGGGGCCCAGAAAACTGGCCCTTGCCATTGG
 
CTCGTGTTCGTGCAAGTTGAGTCCATCCGCCGGCCAGCGGGGGCGGC::G:A:G:G
AGGCGCTCCCAGGTTCCGGCCCTCCCCTCGGCCCCGCGCCGCAGAGTCTGGCC
GCGCGCCCCTGCGCAACGTGGCAGGAAGCGCGCGCTGGGGGCGGGGACGGGC
AGTAGGGCTGAGCGGCTGCGGGGCGGGTGCAAGCACGTTTCCGACTTGAGTTGC
CTCAAGAGGGGCGTGCTGAGCCAGACCTCCATCGCGCACTCCGGGGAGTGGAG
GGAAGGAGCGAGGGCTCAGTTGGGCTGTTTTGGAGGCAGGAAGCACTTGCTCTC
CCAAAGTCGCTCTGAGTTGTTATCAGTAAGGGAGCTGCAGTGGAGTAGGCGGGG
AGAAGGCCGCACCCTTCTCCGGAGGGGGGAGGGGAGTGTTGCAATACCTTTCTG
GGAGTTCTCTGCTGCCTCCTGGCTTCTGAGGACCGCCCTGGGCCTGGGAGAATC
CCTT:CCCCCTCTT:CCCTCGTGATCTGCAACTCCAGTCTTT:CTAGGTAACCGATAT
CCCTGCAGGTTTTCGACATTGATTATTGACTAGTTATTAATAGTAATCAATTACGGG
GTCATTAGTTCATAGCCCATATATGGAGTTCCGCGTTACATAACTTACGGTAAATG
GCCCGCCTGGCTGACCGCCCAACGACCCCCGCCCATTGACGTCAATAATGACGT
ATGTTCCCATAGTAACGCCAATAGGGACTTTCCATTGACGTCAATGGGTGGAGTAT
TTACGGTAAACTGCCCACTTGGCAGTACATCAAGTGTATCATATGCCAAGTACGCC
CCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTAC
CAT:GGTCGAGGTGAGCCCCACGTTCTGCTTCACTCTCCCCATCTCCCCCCCCTC
CCCACCCCCAATTTTGTATTTATTTATTTTTTAATTATTTTGTGCAGCGATGGGGGC
GGGGGGGGGGGGGGCGCGCGCCAGGCGGGGCGGGGCGGGGCGAGGGGCGG
GGCGGGGCGAGGCGGAGAGGTGCGGCGGCAGCCAATCAGAGCGGCGCGCTCC
GAAAGTTTCCTTTTATGGCGAGGCGGCGGCGGCGGCGGCCCTATAAAAAGCGAA
GCGCGCGGCGGGCGGGAGTCGCTGCGTTGCCTTCGCCCCGTGCCCCGCTCCGC
GCCGCCTCGCGCCGCCCGCCCCGGCTCTGACTGACCGCGTTACTCCCACAGGT
GAGCGGGCGGGACGGCCCTTCTCCTCCGGGCTGTAATTAGCGCTTGGTTTAATG
ACGGCTCGTTTCTTTTCTGTGGCTGCGTGAAAGCCTTAAAGGGCTCCGGGAGGG
CCCTTTGTGCGGGGGGGAGCGGCTCGGGGGGTGCGTGCGTGTGTGTGTGCGTG
GGGAGCGCCGCGTGCGGCCCGCGCTGCCCGGCGGCTGTGAGCGCTGCGGGCG
CGGCGCGGGGCTTTGTGCGCTCCGCGTGTGCGCGAGGGGAGCGCGGCCGGGG
GCGGTGCCCCGCGGTGCGGGGGGGCTGCGAGGGGAACAAAGGCTGCGTGCGG
GGTGTGTGCGTGGGGGGGTGAGCAGGGGGTGTGGGCGCGGCGGTCGGGCTGT
AACCCCCCCCTGCACCCCCCTCCCCGAGTTGCTGAGCACGGCCCGGCTTCGGGT
GCGGGGCTCCGTGCGGGGCGTGGCGCGGGGCTCGCCGTGCCGGGCGGGGGGT
GGCGGCAGGTGGGGGTGCCGGGCGGGGCGGGGCCGCCTCGGGCCGGGGAGG
GCTCGGGGGAGGGGCGCGGCGGCCCCGGAGCGCCGGCGGCTGTCGAGGCGC
 
GGCGAGCCGCAGCCATTGCCTTTTATGGTAATCGTGCGAGAGGGCGCAGGGACT
TCCTTTGTCCCAAATCTGGCGGAGCCGAAATCTGGGAGGCGCCGCCGCACCCCC
TCTAGCGGGCGCGGGCGAAGCGGTGCGGCGCCGGCAGGAAGGAAATGGGCGG
GGAGGGCCTTCGTGCGTCGCCGCGCCGCCGTCCCCTTCTCCATCTCCAGCCTCG
GGGCTGCCGCAGGGGGACGGCTGCCTTCGGGGGGGACGGGGCAGGGCGGGGT
TCGGCTTCTGGCGTGTGACCGGCGGCTCTAGAAGCGTTGGGGTGAGTACTCCCT
CTCAAAAGCGGGCATGACTTCTGCGCTAAGATTGTCAGTTTCCAAAAACGAGGAG
GATTTGATATTCACCTGGCCCGCGGTGATGCCTTTGAGGGTGGCCGCGTCCATCT
GGTCAGAAAAGACAATCTTTTTGTTGTCAAGCTTGAGGTGTGGCAGGCTTGAGAT
CTGGCCATACACTTGAGTGACATTGACATCCACTTTGCCTTTCTCTCCACAGGTGT
CCACTCCCAGGGCGGCCTCCGGAGCGATCGCCGGTCCGCCTAGGCTAGCCGGC
CGCGTCGACTTAAT
SEQ ID NO: 4 : Troponin T promoter 
TCTCTCGGGGTGGGGGATAAAAGCAGTCTGGGCTTTCACATGACAGCATCT
GGGGCTGCGGCAGAGGGTCGGGTCCGAAGCGCTGCCTTATCAGCGTCCCCA
GCCCTGGGAGGTGACAGCTGGCTGGCTTGTGTCAGCCCCTCGGGCACTCAC
GTATCTCCATCCGACGGGTTTAAAATAGCAAAACTCTGAGGCCACACAATA
GCTTGGGCTTATATGGGCTCCTGTGGGGGAAGGGGGAGCACGGAGGGGGCC
GGGGCCGCTGCTGCCAAAATAGCAGCTCACAAGTGTTGCATTCCTCTCTGGG
CGCCGGGCACATTCCTGCTGGCTCTGCCCGCCCCGGGGTGGGCGCCGGGGG
GACCTTAAAGCCTCTGCCCCCCAAGGAGCCCTTCCCAGACAGCCGCCGGCA
CCCACCGCTCCGTGGGACCT
SEQ ID NO: 5: Cyclin T1 amino acid sequence 
MEGERKNNNKRWYFTREQLENSPSRRFGVDPDKELSYRQQAANLLQDMGQRLNVSQLTINTAIVYMHRFY
MIQSFTQFPGNSVAPAALFLAAKVEEQPKKLEHVIKVAHTCLHPQESLPDTRSEAYLQQVQDLVILESII
LQTLGFELTIDHPHTHVVKCTQLVRASKDLAQTSYFMATNSLHLTTFSLQYTPPVVACVCIHLACKWSNW
EIPVSTDGKHWWEYVDATVTLELLDELTHEFLQILEKTPNRLKRIWNWRACEAAKKTKADDRGTDEKTSE
QTILNMISQSSSDTTIAGLMSMSTSTTSAVPSLPVSEESSSNLTSVEMLPGKRWLSSQPSFKLEPTQGHR
TSENLALTGVDHSLPQDGSNAFISQKQNSKSVPSAKVSLKEYRAKHAEELAAQKRQLENMEANVKSQYAY
AAQNLLSHHDSHSSVILKMPIEGSENPERPFLEKADKTALKMRIPVAGGDKAASSKPEEIKMRIKVHAAA
DKHNSVEDSVTKSREHKEKHKTHPSNHHHHHNHHSHKHSHSQLPVGTGNKRPGDPKHSSQTSNLAHKTYS
LSSSFSSSSSTRKRGPSEETGGAVFDHPAKIAKSTKSSSLNFSFPSLPTMGQMPGHSSDTSGLSFSQPSC
KTRVPHSKLDKGPTGANGHNTTQTIDYQDTVNMLHSLLSAQGVQPTQPTAFEFVRPYSDYLNPRSGGISS
RSGNTDKPRPPPLPSEPPPPLPPLPK
SEQ ID NO: 6: Cyclin T1 nucleic acid sequence 
ATGGAGGGAGAGAGGAAGAACAACAACAAACGGTGGTATTTCACTCGAGAACAGCTGGAAAATAGCCCAT
CCCGTCGTTTTGGCGTGGACCCAGATAAAGAACTTTCTTATCGCCAGCAGGCGGCCAATCTGCTTCAGGA
CATGGGGCAGCGTCTTAACGTCTCACAATTGACTATCAACACTGCTATAGTATACATGCATCGATTCTAC
ATGATTCAGTCCTTCACACAGTTCCCTGGAAATTCTGTGGCTCCAGCAGCCTTGTTTCTAGCAGCTAAAG
TGGAGGAGCAGCCCAAAAAATTGGAACATGTCATCAAGGTAGCACATACTTGTCTCCATCCTCAGGAATC
CCTTCCTGATACTAGAAGTGAGGCTTATTTGCAACAAGTTCAAGATCTGGTCATTTTAGAAAGCATAATT
TTGCAGACTTTAGGCTTTGAACTAACAATTGATCACCCACATACTCATGTAGTAAAGTGCACTCAACTTG
TTCGAGCAAGCAAGGACTTAGCACAGACTTCTTACTTCATGGCAACCAACAGCCTGCATTTGACCACATT
TAGCCTGCAGTACACACCTCCTGTGGTGGCCTGTGTCTGCATTCACCTGGCTTGCAAGTGGTCCAATTGG
GAGATCCCAGTCTCAACTGACGGGAAGCACTGGTGGGAGTATGTTGACGCCACTGTGACCTTGGAACTTT
TAGATGAACTGACACATGAGTTTCTACAGATTTTGGAGAAAACTCCCAACAGGCTCAAACGCATTTGGAA
TTGGAGGGCATGCGAGGCTGCCAAGAAAACAAAAGCAGATGACCGAGGAACAGATGAAAAGACTTCAGAG
CAGACAATCCTCAATATGATTTCCCAGAGCTCTTCAGACACAACCATTGCAGGTTTAATGAGCATGTCAA
CTTCTACCACAAGTGCAGTGCCTTCCCTGCCAGTCTCCGAAGAGTCATCCAGCAACTTAACCAGTGTGGA
GATGTTGCCGGGCAAGCGTTGGCTGTCCTCCCAACCTTCTTTCAAACTAGAACCTACTCAGGGTCATCGG
ACTAGTGAGAATTTAGCACTTACAGGAGTTGATCATTCCTTACCACAGGATGGTTCAAATGCATTTATTT
CCCAGAAGCAGAATAGTAAGAGTGTGCCATCAGCTAAAGTGTCACTGAAAGAATACCGCGCGAAGCATGC
AGAAGAATTGGCTGCCCAGAAGAGGCAACTGGAGAACATGGAAGCCAATGTGAAGTCACAATATGCATAT
GCTGCCCAGAATCTCCTTTCTCATCATGATAGCCATTCTTCAGTCATTCTAAAAATGCCCATAGAGGGTT
CAGAAAACCCCGAGCGGCCTTTTCTGGAAAAGGCTGACAAAACAGCTCTCAAAATGAGAATCCCAGTGGC
AGGTGGAGATAAAGCTGCGTCTTCAAAACCAGAGGAGATAAAAATGCGCATAAAAGTCCATGCTGCAGCT
GATAAGCACAATTCTGTAGAGGACAGTGTTACAAAGAGCCGAGAGCACAAAGAAAAGCACAAGACTCACC
CATCTAATCATCATCATCATCATAATCACCACTCACACAAGCACTCTCATTCCCAACTTCCAGTTGGTAC
TGGGAACAAACGTCCTGGTGATCCAAAACATAGTAGCCAGACAAGCAACTTAGCACATAAAACCTATAGC
TTGTCTAGTTCTTTTTCCTCTTCCAGTTCTACTCGTAAAAGGGGACCCTCTGAAGAGACTGGAGGGGCTG
TGTTTGATCATCCAGCCAAGATTGCCAAGAGTACTAAATCCTCTTCCCTAAATTTCTCCTTCCCTTCACT
TCCTACAATGGGTCAGATGCCTGGGCATAGCTCAGACACAAGTGGCCTTTCCTTTTCACAGCCCAGCTGT
AAAACTCGTGTCCCTCATTCGAAACTGGATAAAGGGCCCACTGGGGCCAATGGTCACAACACGACCCAGA
CAATAGACTATCAAGACACTGTGAATATGCTTCACTCCCTGCTCAGTGCCCAGGGTGTTCAGCCCACTCA
GCCCACTGCATTTGAATTTGTTCGTCCTTATAGTGACTATCTGAATCCTCGGTCTGGTGGAATCTCCTCG
AGATCTGGCAATACAGACAAACCCCGGCCACCACCTCTGCCATCAGAACCTCCTCCACCACTTCCACCCC
TTCCTAAGTAA
SEQ ID NO: 7: CDK9 amino acid sequence 
MAKQYDSVECPFCDEVSKYEKLAKIGQGTFGEVFKARHRKTGQKVALKKVLMENEKEGFPITALREIKIL
QLLKHENVVNLIEICRTKASPYNRCKGSIYLVFDFCEHDLAGLLSNVLVKFTLSEIKRVMQMLLNGLYYI
HRNKILHRDMKAANVLITRDGVLKLADFGLARAFSLAKNSQPNRYTNRVVTLWYRPPELLLGERDYGPPI
DLWGAGCIMAEMWTRSPIMQGNTEQHQLALISQLCGSITPEVWPNVDNYELYEKLELVKGQKRKVKDRLK
 
AYVRDPYALDLIDKLLVLDPAQRIDSDDALNHDFFWSDPMPSDLKGMLSTHLTSMFEYLAPPRRKGSQIT
QQSTNQSRNPATTNQTEFERVF
SEQ ID NO: 8: CDK9 nucleic acid sequence 
ATGGCAAAGCAGTACGACTCGGTGGAGTGCCCTTTTTGTGATGAAGTTTCCAAATACGAGAAGCTCGCCA
AGATCGGCCAAGGCACCTTCGGGGAGGTGTTCAAGGCCAGGCACCGCAAGACCGGCCAGAAGGTGGCTCT
GAAGAAGGTGCTGATGGAAAACGAGAAGGAGGGGTTCCCCATTACAGCCTTGCGGGAGATCAAGATCCTT
CAGCTTCTAAAACACGAGAATGTGGTCAACTTGATTGAGATTTGTCGAACCAAAGCTTCCCCCTATAACC
GCTGCAAGGGTAGTATATACCTGGTGTTCGACTTCTGCGAGCATGACCTTGCTGGGCTGTTGAGCAATGT
TTTGGTCAAGTTCACGCTGTCTGAGATCAAGAGGGTGATGCAGATGCTGCTTAACGGCCTCTACTACATC
CACAGAAACAAGATCCTGCATAGGGACATGAAGGCTGCTAATGTGCTTATCACTCGTGATGGGGTCCTGA
AGCTGGCAGACTTTGGGCTGGCCCGGGCCTTCAGCCTGGCCAAGAACAGCCAGCCCAACCGCTACACCAA
CCGTGTGGTGACACTCTGGTACCGGCCCCCGGAGCTGTTGCTCGGGGAGCGGGACTACGGCCCCCCCATT
GACCTGTGGGGTGCTGGGTGCATCATGGCAGAGATGTGGACCCGCAGCCCCATCATGCAGGGCAACACGG
AGCAGCACCAACTCGCCCTCATCAGTCAGCTCTGCGGCTCCATCACCCCTGAGGTGTGGCCAAACGTGGA
CAACTATGAGCTGTACGAAAAGCTGGAGCTGGTCAAGGGCCAGAAGCGGAAGGTGAAGGACAGGCTGAAG
GCCTATGTGCGTGACCCATACGCACTGGACCTCATCGACAAGCTGCTGGTGCTGGACCCTGCCCAGCGCA
TCGACAGCGATGACGCCCTCAACCACGACTTCTTCTGGTCCGACCCCATGCCCTCCGACCTCAAGGGCAT
GCTCTCCACCCACCTGACGTCCATGTTCGAGTACTTGGCACCACCGCGCCGGAAGGGCAGCCAGATCACC
CAGCAGTCCACCAACCAGAGTCGCAATCCCGCCACCACCAACCAGACGGAGTTTGAGCGCGTCTTCTGA
SEQ ID NO: 9: MycER^{™} nucleic acid sequence 
ATGCCCCTCAACGTTAGCTTCACCAACAGGAACTATGACCTCGACTACGACTCGG
TGCAGCCGTATTTCTACTGCGACGAGGAGGAGAACTTCTACCAGCAGCAGCAGCA
GAGCGAGCTGCAGCCCCCGGCGCCCAGCGAGGATATCTGGAAGAAATTCGAGCT
GCTGCCCACCCCGCCCCTGTCCCCTAGCCGCCGCTCCGGGCTCTGCTCGCCCTC
CTACGTTGCGGTCACACCCTTCTCCCTTCGGGGAGACAACGACGGCGGTGGCGG
GAGCTTCTCCACGGCCGACCAGCTGGAGATGGTGACCGAGCTGCTGGGAGGAG
ACATGGTGAACCAGAGTTTCATCTGCGACCCGGACGACGAGACCTTCATCAAAAA
CATCATCATCCAGGACTGTATGTGGAGCGGCTTCTCGGCCGCCGCCAAGCTCGT
CTCAGAGAAGCTGGCCTCCTACCAGGCTGCGCGCAAAGACAGCGGCAGCCCGAA
CCCCGCCCGCGGCCACAGCGTCTGCTCCACCTCCAGCTTGTACCTGCAGGATCT
GAGCGCCGCCGCCTCAGAGTGCATCGACCCCTCGGTGGTCTTCCCCTACCCTCT
CAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCC
CGAGCCCCTGGTGCTCCATGAGGAGACACCGCCCACCACCAGCAGCGACTCTGA
GGAGGAACAAGAAGATGAGGAAGAAATCGATGTTGTTTCTGTGGAAAAGAGGCAG
 
GCTCCTGGCAAAAGGTCAGAGTCTGGATCACCTTCTGCTGGAGGCCACAGCAAA
CCTCCTCACAGCCCACTGGTCCTCAAGAGGTGCCACGTCTCCACACATCAGCACA
ACTACGCAGCGCCTCCCTCCACTCGGAAGGACTATCCTGCTGCCAAGAGGGTCA
AGTTGGACAGTGTCAGAGTCCTGAGACAGATCAGCAACAACCGAAAATGCACCAG
CCCCAGGTCCTCGGACACCGAGGAGAATGTCAAGAGGCGAACACACAACGTCTT
GGAGCGCCAGAGGAGGAACGAGCTAAAACGGAGCTTTTTTGCCCTGCGTGACCA
GATCCCGGAGTTGGAAAACAATGAAAAGGCCCCCAAGGTAGTTATCCTTAAAAAA
GCCACAGCATACATCCTGTCCGTCCAAGCAGAGGAGCAAAAGCTCATTTCTGAAG
AGGACTTGTTGCGGAAACGACGAGAACAGTTGAAACACAAACTTGAACAGCTACG
GGATCCACGAAATGAAATGGGTGCTTCAGGAGACATGAGGGCTGCCAACCTTTG
GCCAAGCCCTCTTGTGATTAAGCACACTAAGAAGAATAGCCCTGCCTTGTCCTTG
ACAGCTGACCAGATGGTCAGTGCCTTGTTGGATGCTGAACCGCCCATGATCTATT
CTGAATATGATCCTTCTAGACCCTTCAGTGAAGCCTCAATGATGGGCTTATTGACC
AACCTAGCAGATAGGGAGCTGGTTCATATGATCAACTGGGCAAAGAGAGTGCCAG
GCTTTGGGGACTTGAATCTCCATGATCAGGTCCACCTTCTCGAGTGTGCCTGGCT
GGAGATTCTGATGATTGGTCTCGTCTGGCGCTCCATGGAACACCCGGGGAAGCT
CCTGTTTGCTCCTAACTTGCTCCTGGACAGGAATCAAGGTAAATGTGTGGAAGGC
ATGGTGGAGATCTTTGACATGTTGCTTGCTACGTCAAGTCGGTTCCGCATGATGA
ACCTGCAGGGTGAAGAGTTTGTGTGCCTCAAATCCATCATTTTGCTTAATTCCGGA
GTGTACACGTTTCTGTCCAGCACCTTGAAGTCTCTGGAAGAGAAGGACCACATCC
ACCGTGTCCTGGACAAGATCACAGACACTTTGATCCACCTGATGGCCAAAGCTGG
CCTGACTCTGCAGCAGCAGCATCGCCGCCTAGCTCAGCTCCTTCTCATTCTTTCC
CATATCCGGCACATGAGTAACAAACGCATGGAGCATCTCTACAACATGAAATGCA
AGAACGTGGTACCCCTCTATGACCTGCTCCTGGAGATGTTGGATGCCCACCGCCT
TCATGCCCCAGCCAGTCGCATGGGAGTGCCCCCAGAGGAGCCCAGCCAGACCCA
GCTGGCCACCACCAGCTCCACTTCAGCACATTCCTTACAAACCTACTACATACCC
CCGGAAGCAGAGGGCTTCCCCAACACGATCTGAGAGCTCCTACTAGTGAATTC
SEQ ID NO: 10: MycER^{™} amino acid sequence 
Met P L N V S F T N R N Y D L D Y D S V Q P Y F Y C D E E E N F Y Q Q Q Q S E
L Q P P A P S E D I W K K F E L L P T P P L S P S R R S G L C S P S Y V A V T P F S
L R G D N D G G G G S F S T A D Q L E Met V T E L L G G D Met V N Q S F I C D P
D D E T F I K N I I I Q D C Met W S G F S A A A K L V S E K L A S Y Q A A R K D S G
S P N P A R G H S V C S T S S L Y L Q D L S A A A S E C I D P S V V F P Y P L N D
S S S P K S C A S Q D S S A F S P S S D S L L S S T E S S P Q G S P E P L V L H E
 
E T P P T T S S D S E E E Q E D E E E I D V V S V E K R Q A P G K R S E S G S P S
A G G H S K P P H S P L V L K R C H V S T H Q H N Y A A P P S T R K D Y P A A K
R V K L D S V R V L R Q I S N N R K C T S P R S S D T E E N V K R R T H N V L E R
Q R R N E L K R S F F A L R D Q I P E L E N N E K A P K V V I L K K A T A Y I L S V
Q A E E Q K L I S E E D L L R K R R E Q L K H K L E Q L R D P R N E Met G A S G D
Met R A A N L W P S P L V I K H T K K N S P A L S L T A D Q Met V S A L L D A E P
P Met I Y S E Y D P S R P F S E A S Met Met G L L T N L A D R E L V H Met I N W A
K R V P G F G D L N L H D Q V H L L E C A W L E I L Met I G L V W R S Met E H P G
K L L F A P N L L L D R N Q G K C V E G Met V E I F D Met L L A T S S R F R Met Met
N L Q G E E F V C L K S I I L L N S G V Y T F L S S T L K S L E E K D H I H R V L D K
I T D T L I H L Met A K A G L T L Q Q Q H R R L A Q L L L I L S H I R H Met S N K R
Met E H L Y N Met K C K N V V P L Y D L L L E Met L D A H R L H A P A S R Met G V
P P E E P S Q T Q L A T T S S T S A H S L Q T Y Y I P P E A E G F P N T I Stop E L
L L V N
SEQ ID NO: 11: c-myc amino acid sequence 
MDFFRVVENQQPPATMPLNVSFTNRNYDLDYDSVQPYFYCDEEENFYQQQQQSELQPPAPSEDIWKKFEL
LPTPPLSPSRRSGLCSPSYVAVTPFSLRGDNDGGGGSFSTADQLEMVTELLGGDMVNQSFICDPDDETFI
KNIIIQDCMWSGFSAAAKLVSEKLASYQAARKDSGSPNPARGHSVCSTSSLYLQDLSAAASECIDPSVVF
PYPLNDSSSPKSCASQDSSAFSPSSDSLLSSTESSPQGSPEPLVLHEETPPTTSSDSEEEQEDEEEIDVV
SVEKRQAPGKRSESGSPSAGGHSKPPHSPLVLKRCHVSTHQHNYAAPPSTRKDYPAAKRVKLDSVRVLRQ
ISNNRKCTSPRSSDTEENVKRRTHNVLERQRRNELKRSFFALRDQIPELENNEKAPKVVILKKATAYILS
VQAEEQKLISEEDLLRKRREQLKHKLEQLRNSCA
SEQ ID NO: 12: c-myc nucleic acid sequence 
CTGGATTTTTTTCGGGTAGTGGAAAACCAGCAGCCTCCCGCGACGATGCCCCTCAACGTTAGCTTCACCA
ACAGGAACTATGACCTCGACTACGACTCGGTGCAGCCGTATTTCTACTGCGACGAGGAGGAGAACTTCTA
CCAGCAGCAGCAGCAGAGCGAGCTGCAGCCCCCGGCGCCCAGCGAGGATATCTGGAAGAAATTCGAGCTG
CTGCCCACCCCGCCCCTGTCCCCTAGCCGCCGCTCCGGGCTCTGCTCGCCCTCCTACGTTGCGGTCACAC
CCTTCTCCCTTCGGGGAGACAACGACGGCGGTGGCGGGAGCTTCTCCACGGCCGACCAGCTGGAGATGGT
GACCGAGCTGCTGGGAGGAGACATGGTGAACCAGAGTTTCATCTGCGACCCGGACGACGAGACCTTCATC
AAAAACATCATCATCCAGGACTGTATGTGGAGCGGCTTCTCGGCCGCCGCCAAGCTCGTCTCAGAGAAGC
TGGCCTCCTACCAGGCTGCGCGCAAAGACAGCGGCAGCCCGAACCCCGCCCGCGGCCACAGCGTCTGCTC
CACCTCCAGCTTGTACCTGCAGGATCTGAGCGCCGCCGCCTCAGAGTGCATCGACCCCTCGGTGGTCTTC
CCCTACCCTCTCAACGACAGCAGCTCGCCCAAGTCCTGCGCCTCGCAAGACTCCAGCGCCTTCTCTCCGT
CCTCGGATTCTCTGCTCTCCTCGACGGAGTCCTCCCCGCAGGGCAGCCCCGAGCCCCTGGTGCTCCATGA
GGAGACACCGCCCACCACCAGCAGCGACTCTGAGGAGGAACAAGAAGATGAGGAAGAAATCGATGTTGTT
 
TCTGTGGAAAAGAGGCAGGCTCCTGGCAAAAGGTCAGAGTCTGGATCACCTTCTGCTGGAGGCCACAGCA
AACCTCCTCACAGCCCACTGGTCCTCAAGAGGTGCCACGTCTCCACACATCAGCACAACTACGCAGCGCC
TCCCTCCACTCGGAAGGACTATCCTGCTGCCAAGAGGGTCAAGTTGGACAGTGTCAGAGTCCTGAGACAG
ATCAGCAACAACCGAAAATGCACCAGCCCCAGGTCCTCGGACACCGAGGAGAATGTCAAGAGGCGAACAC
ACAACGTCTTGGAGCGCCAGAGGAGGAACGAGCTAAAACGGAGCTTTTTTGCCCTGCGTGACCAGATCCC
GGAGTTGGAAAACAATGAAAAGGCCCCCAAGGTAGTTATCCTTAAAAAAGCCACAGCATACATCCTGTCC
GTCCAAGCAGAGGAGCAAAAGCTCATTTCTGAAGAGGACTTGTTGCGGAAACGACGAGAACAGTTGAAAC
ACAAACTTGAACAGCTACGGAACTCTTGTGCGTAA
SEQ ID NO: 13: Cyclin T1 RNA sequence 
AUGGAGGGAGAGAGGAAGAACAACAACAAACGGUGGUAUUUCACUCGAGAACAGCUGGAAAAUAGCCCAU
CCCGUCGUUUUGGCGUGGACCCAGAUAAAGAACUUUCUUAUCGCCAGCAGGCGGCCAAUCUGCUUCAGGA
CAUGGGGCAGCGUCUUAACGUCUCACAAUUGACUAUCAACACUGCUAUAGUAUACAUGCAUCGAUUCUAC
AUGAUUCAGUCCUUCACACAGUUCCCUGGAAAUUCUGUGGCUCCAGCAGCCUUGUUUCUAGCAGCUAAAG
UGGAGGAGCAGCCCAAAAAAUUGGAACAUGUCAUCAAGGUAGCACAUACUUGUCUCCAUCCUCAGGAAUC
CCUUCCUGAUACUAGAAGUGAGGCUUAUUUGCAACAAGUUCAAGAUCUGGUCAUUUUAGAAAGCAUAAUU
UUGCAGACUUUAGGCUUUGAACUAACAAUUGAUCACCCACAUACUCAUGUAGUAAAGUGCACUCAACUUG
UUCGAGCAAGCAAGGACUUAGCACAGACUUCUUACUUCAUGGCAACCAACAGCCUGCAUUUGACCACAUU
UAGCCUGCAGUACACACCUCCUGUGGUGGCCUGUGUCUGCAUUCACCUGGCUUGCAAGUGGUCCAAUUGG
GAGAUCCCAGUCUCAACUGACGGGAAGCACUGGUGGGAGUAUGUUGACGCCACUGUGACCUUGGAACUUU
UAGAUGAACUGACACAUGAGUUUCUACAGAUUUUGGAGAAAACUCCCAACAGGCUCAAACGCAUUUGGAA
UUGGAGGGCAUGCGAGGCUGCCAAGAAAACAAAAGCAGAUGACCGAGGAACAGAUGAAAAGACUUCAGAG
CAGACAAUCCUCAAUAUGAUUUCCCAGAGCUCUUCAGACACAACCAUUGCAGGUUUAAUGAGCAUGUCAA
CUUCUACCACAAGUGCAGUGCCUUCCCUGCCAGUCUCCGAAGAGUCAUCCAGCAACUUAACCAGUGUGGA
GAUGUUGCCGGGCAAGCGUUGGCUGUCCUCCCAACCUUCUUUCAAACUAGAACCUACUCAGGGUCAUCGG
ACUAGUGAGAAUUUAGCACUUACAGGAGUUGAUCAUUCCUUACCACAGGAUGGUUCAAAUGCAUUUAUUU
CCCAGAAGCAGAAUAGUAAGAGUGUGCCAUCAGCUAAAGUGUCACUGAAAGAAUACCGCGCGAAGCAUGC
AGAAGAAUUGGCUGCCCAGAAGAGGCAACUGGAGAACAUGGAAGCCAAUGUGAAGUCACAAUAUGCAUAU
GCUGCCCAGAAUCUCCUUUCUCAUCAUGAUAGCCAUUCUUCAGUCAUUCUAAAAAUGCCCAUAGAGGGUU
CAGAAAACCCCGAGCGGCCUUUUCUGGAAAAGGCUGACAAAACAGCUCUCAAAAUGAGAAUCCCAGUGGC
AGGUGGAGAUAAAGCUGCGUCUUCAAAACCAGAGGAGAUAAAAAUGCGCAUAAAAGUCCAUGCUGCAGCU
GAUAAGCACAAUUCUGUAGAGGACAGUGUUACAAAGAGCCGAGAGCACAAAGAAAAGCACAAGACUCACC
CAUCUAAUCAUCAUCAUCAUCAUAAUCACCACUCACACAAGCACUCUCAUUCCCAACUUCCAGUUGGUAC
UGGGAACAAACGUCCUGGUGAUCCAAAACAUAGUAGCCAGACAAGCAACUUAGCACAUAAAACCUAUAGC
UUGUCUAGUUCUUUUUCCUCUUCCAGUUCUACUCGUAAAAGGGGACCCUCUGAAGAGACUGGAGGGGCUG
UGUUUGAUCAUCCAGCCAAGAUUGCCAAGAGUACUAAAUCCUCUUCCCUAAAUUUCUCCUUCCCUUCACU
UCCUACAAUGGGUCAGAUGCCUGGGCAUAGCUCAGACACAAGUGGCCUUUCCUUUUCACAGCCCAGCUGU
 
AAAACUCGUGUCCCUCAUUCGAAACUGGAUAAAGGGCCCACUGGGGCCAAUGGUCACAACACGACCCAGA
CAAUAGACUAUCAAGACACUGUGAAUAUGCUUCACUCCCUGCUCAGUGCCCAGGGUGUUCAGCCCACUCA
GCCCACUGCAUUUGAAUUUGUUCGUCCUUAUAGUGACUAUCUGAAUCCUCGGUCUGGUGGAAUCUCCUCG
AGAUCUGGCAAUACAGACAAACCCCGGCCACCACCUCUGCCAUCAGAACCUCCUCCACCACUUCCACCCC
UUCCUAAGUAA
SEQ ID NO: 14: CDK9 RNA sequence 
AUGGCAAAGCAGUACGACUCGGUGGAGUGCCCUUUUUGUGAUGAAGUUUCCAAAUACGAGAAGCUCGCCA
AGAUCGGCCAAGGCACCUUCGGGGAGGUGUUCAAGGCCAGGCACCGCAAGACCGGCCAGAAGGUGGCUCU
GAAGAAGGUGCUGAUGGAAAACGAGAAGGAGGGGUUCCCCAUUACAGCCUUGCGGGAGAUCAAGAUCCUU
CAGCUUCUAAAACACGAGAAUGUGGUCAACUUGAUUGAGAUUUGUCGAACCAAAGCUUCCCCCUAUAACC
GCUGCAAGGGUAGUAUAUACCUGGUGUUCGACUUCUGCGAGCAUGACCUUGCUGGGCUGUUGAGCAAUGU
UUUGGUCAAGUUCACGCUGUCUGAGAUCAAGAGGGUGAUGCAGAUGCUGCUUAACGGCCUCUACUACAUC
CACAGAAACAAGAUCCUGCAUAGGGACAUGAAGGCUGCUAAUGUGCUUAUCACUCGUGAUGGGGUCCUGA
AGCUGGCAGACUUUGGGCUGGCCCGGGCCUUCAGCCUGGCCAAGAACAGCCAGCCCAACCGCUACACCAA
CCGUGUGGUGACACUCUGGUACCGGCCCCCGGAGCUGUUGCUCGGGGAGCGGGACUACGGCCCCCCCAUU
GACCUGUGGGGUGCUGGGUGCAUCAUGGCAGAGAUGUGGACCCGCAGCCCCAUCAUGCAGGGCAACACGG
AGCAGCACCAACUCGCCCUCAUCAGUCAGCUCUGCGGCUCCAUCACCCCUGAGGUGUGGCCAAACGUGGA
CAACUAUGAGCUGUACGAAAAGCUGGAGCUGGUCAAGGGCCAGAAGCGGAAGGUGAAGGACAGGCUGAAG
GCCUAUGUGCGUGACCCAUACGCACUGGACCUCAUCGACAAGCUGCUGGUGCUGGACCCUGCCCAGCGCA
UCGACAGCGAUGACGCCCUCAACCACGACUUCUUCUGGUCCGACCCCAUGCCCUCCGACCUCAAGGGCAU
GCUCUCCACCCACCUGACGUCCAUGUUCGAGUACUUGGCACCACCGCGCCGGAAGGGCAGCCAGAUCACC
CAGCAGUCCACCAACCAGAGUCGCAAUCCCGCCACCACCAACCAGACGGAGUUUGAGCGCGUCUUCUGA
SEQ ID NO: 15: c-myc RNA sequence 
CUGGAUUUUUUUCGGGUAGUGGAAAACCAGCAGCCUCCCGCGACGAUGCCCCUCAACGUUAGCUUCACCA
ACAGGAACUAUGACCUCGACUACGACUCGGUGCAGCCGUAUUUCUACUGCGACGAGGAGGAGAACUUCUA
CCAGCAGCAGCAGCAGAGCGAGCUGCAGCCCCCGGCGCCCAGCGAGGAUAUCUGGAAGAAAUUCGAGCUG
CUGCCCACCCCGCCCCUGUCCCCUAGCCGCCGCUCCGGGCUCUGCUCGCCCUCCUACGUUGCGGUCACAC
CCUUCUCCCUUCGGGGAGACAACGACGGCGGUGGCGGGAGCUUCUCCACGGCCGACCAGCUGGAGAUGGU
GACCGAGCUGCUGGGAGGAGACAUGGUGAACCAGAGUUUCAUCUGCGACCCGGACGACGAGACCUUCAUC
AAAAACAUCAUCAUCCAGGACUGUAUGUGGAGCGGCUUCUCGGCCGCCGCCAAGCUCGUCUCAGAGAAGC
UGGCCUCCUACCAGGCUGCGCGCAAAGACAGCGGCAGCCCGAACCCCGCCCGCGGCCACAGCGUCUGCUC
CACCUCCAGCUUGUACCUGCAGGAUCUGAGCGCCGCCGCCUCAGAGUGCAUCGACCCCUCGGUGGUCUUC
CCCUACCCUCUCAACGACAGCAGCUCGCCCAAGUCCUGCGCCUCGCAAGACUCCAGCGCCUUCUCUCCGU
CCUCGGAUUCUCUGCUCUCCUCGACGGAGUCCUCCCCGCAGGGCAGCCCCGAGCCCCUGGUGCUCCAUGA
GGAGACACCGCCCACCACCAGCAGCGACUCUGAGGAGGAACAAGAAGAUGAGGAAGAAAUCGAUGUUGUU
 
UCUGUGGAAAAGAGGCAGGCUCCUGGCAAAAGGUCAGAGUCUGGAUCACCUUCUGCUGGAGGCCACAGCA
AACCUCCUCACAGCCCACUGGUCCUCAAGAGGUGCCACGUCUCCACACAUCAGCACAACUACGCAGCGCC
UCCCUCCACUCGGAAGGACUAUCCUGCUGCCAAGAGGGUCAAGUUGGACAGUGUCAGAGUCCUGAGACAG
AUCAGCAACAACCGAAAAUGCACCAGCCCCAGGUCCUCGGACACCGAGGAGAAUGUCAAGAGGCGAACAC
ACAACGUCUUGGAGCGCCAGAGGAGGAACGAGCUAAAACGGAGCUUUUUUGCCCUGCGUGACCAGAUCCC
GGAGUUGGAAAACAAUGAAAAGGCCCCCAAGGUAGUUAUCCUUAAAAAAGCCACAGCAUACAUCCUGUCC
GUCCAAGCAGAGGAGCAAAAGCUCAUUUCUGAAGAGGACUUGUUGCGGAAACGACGAGAACAGUUGAAAC
ACAAACUUGAACAGCUACGGAACUCUUGUGCGUAA
SEQ ID NO: 16: Cyclin T1 - pseudouridine* (Denoted by P) 
APGGAGGGAGAGAGGAAGAACAACAACAAACGGPGGPAPPPCACPCGAGAACAGCPGGAAAAPAGCCCAP
CCCGPCGPPPPGGCGPGGACCCAGAPAAAGAACPPPCPPAPCGCCAGCAGGCGGCCAAPCPGCPPCAGGA
CAPGGGGCAGCGPCPPAACGPCPCACAAPPGACPAPCAACACPGCPAPAGPAPACAPGCAPCGAPPCPAC
APGAPPCAGPCCPPCACACAGPPCCCPGGAAAPPCPGPGGCPCCAGCAGCCPPGPPPCPAGCAGCPAAAG
PGGAGGAGCAGCCCAAAAAAPPGGAACAPGPCAPCAAGGPAGCACAPACPPGPCPCCAPCCPCAGGAAPC
CCPPCCPGAPACPAGAAGPGAGGCPPAPPPGCAACAAGPPCAAGAPCPGGPCAPPPAGAAAGCAPAAPP
PPGCAGACPPPAGGCPPPGAACPAACAAPPGAPCACCCACAPACPCAPGPAGPAAAGPGCACPCAACPPG
PPCGAGCAAGCAAGGACPPAGCACAGACPPCPPACPPCAPGGCAACCAACAGCCPGCAPPPGACCACAPP
PAGCCPGCAGPACACACCPCCPGPGGPGGCCPGPGPCPGCAPPCACCPGGCPPGCAAGPGGPCCAAPPGG
GAGAPCCCAGPCPCAACPGACGGGAAGCACPGGPGGGAGPAPGPPGACGCCACPGPGACCPPGGAACPPP
PAGAPGAACPGACACAPGAGPPPCPACAGAPPPGGAGAAAACPCCCAACAGGCPCAAACGCAPPPGGAA
PPGGAGGGCAPGCGAGGCPGCCAAGAAAACAAAAGCAGAPGACCGAGGAACAGAPGAAAAGACPPCAGAG
CAGACAAPCCPCAAPAPGAPPPCCCAGAGCPCPPCAGACACAACCAPPGCAGGPPAAPGAGCAPGPCAA
CPPCPACCACAAGPGCAGPGCCPPCCCPGCCAGPCPCCGAAGAGPCAPCCAGCAACPPAACCAGPGPGGA
GAPGPPGCCGGGCAAGCGPPGGCPGPCCPCCCAACCPPCPPPCAAACPAGAACCPACPCAGGGPCAPCGG
ACPAGPGAGAAPPPAGCACPPACAGGAGPPGAPCAPPCCPPACCACAGGAPGGPPCAAAPGCAPPPAPPP
CCCAGAAGCAGAAPAGPAAGAGPGPGCCAPCAGCPAAAGPGPCACPGAAAGAAPACCGCGCGAAGCAPGC
AGAAGAAPPGGCPGCCCAGAAGAGGCAACPGGAGAACAPGGAAGCCAAPGPGAAGPCACAAPAPGCAPAP
GCPGCCCAGAAPCPCCPPPCPCAPCAPGAPAGCCAPPCPPCAGPCAPPCPAAAAAPGCCCAPAGAGGGPP
CAGAAAACCCCGAGCGGCCPPPPCPGGAAAAGGCPGACAAAACAGCPCPCAAAAPGAGAAPCCCAGPGGC
AGGPGGAGAPAAAGCPGCGPCPPCAAAACCAGAGGAGAPAAAAAPGCGCAPAAAAGPCCAPGCPGCAGCP
GAPAAGCACAAPPCPGPAGAGGACAGPGPPACAAAGAGCCGAGAGCACAAAGAAAAGCACAAGACPCACC
CAPCPAAPCAPCAPCAPCAPCAPAAPCACCACPCACACAAGCACPCPCAPPCCCAACPPCCAGPPGGPAC
PGGGAACAAACGPCCPGGPGAPCCAAAACAPAGPAGCCAGACAAGCAACPPAGCACAPAAAACCPAPAGC
PPGPCPAGPPCPPPPPCCPCPPCCAGPPCPACPCGPAAAAGGGGACCCPCPGAAGAGACPGGAGGGGCPG
PGPPPGAPCAPCCAGCCAAGAPPGCCAAGAGPACPAAAPCCPCPPCCCPAAAPPPCPCCPPCCCPPCACP
PCCPACAAPGGGPCAGAPGCCPGGGCAPAGCPCAGACACAAGPGGCCPPPCCPPPPCACAGCCCAGCPGP
 
AAAACPCGPGPCCCPCAPPCGAAACPGGAPAAAGGCCCACPGGGGCCAAPGGPCACAACACGACCCAGA
CAAPAGACPAPCAAGACACPGPGAAPAPGCPPCACPCCCPGCPCAGPGCCCAGGGPGPPCAGCCCACPCA
GCCCACPGCAPPPGAAPPPGPPCGPCCPPAPAGPGACPAPCPGAAPCCPCGGPCPGGPGGAAPCPCCPCG
AGAPCPGGCAAPACAGACAAACCCCGGCCACCACCPCPGCCAPCAGAACCPCCPCCACCACPPCCACCCC
PPCCPAAGPAA
SEQ ID NO: 17: CDK9 - pseudouridine* (Denoted by P) 
APGGCAAAGCAGPACGACPCGGPGGAGPGCCCPPPPGPGAPGAAGPPPCCAAAPACGAGAAGCPCGCCA
AGAPCGGCCAAGGCACCPPCGGGGAGGPGPPCAAGGCCAGGCACCGCAAGACCGGCCAGAAGGPGGCPCP
GAAGAAGGPGCPGAPGGAAAACGAGAAGGAGGGGPPCCCCAPPACAGCCPPGCGGGAGAPCAAGAPCCPP
CAGCPPCPAAAACACGAGAAPGPGGPCAACPPGAPPGAGAPPPGPCGAACCAAAGCPPCCCCPAPAACC
GCPGCAAGGGPAGPAPAPACCPGGPGPPCGACPPCPGCGAGCAPGACCPPGCPGGGCPGPPGAGCAAPGP
PPPGGPCAAGPPCACGCPGPCPGAGAPCAAGAGGGPGAPGCAGAPGCPGCPPAACGGCCPCPACPACAPC
CACAGAAACAAGAPCCPGCAPAGGGACAPGAAGGCPGCPAAPGPGCPPAPCACPCGPGAPGGGGPCCPGA
AGCPGGCAGACPPPGGGCPGGCCCGGGCCPPCAGCCPGGCCAAGAACAGCCAGCCCAACCGCPACACCAA
CCGPGPGGPGACACPCPGGPACCGGCCCCCGGAGCPGPPGCPCGGGGAGCGGACPACGGCCCCCCAPP
GACCPGPGGGGPGCPGGGPGCAPCAPGGCAGAGAPGPGGACCCGCAGCCCCAPCAPGCAGGGCAACACGG
AGCAGCACCAACPCGCCCPCAPCAGPCAGCPCPGCGGCPCCAPCACCCCPGAGGPGPGGCCAAACGPGGA
CAACPAPGAGCPGPACGAAAAGCPGGAGCPGGPCAAGGGCCAGAAGCGGAAGGPGAAGGACAGGCPGAAG
GCCPAPGPGCGPGACCCAPACGCACPGGACCPCAPCGACAAGCPGCPGGPGCPGGACCCPGCCCAGCGCA
PCGACAGCGAPGACGCCCPCAACCACGACPPCPPCPGGPCCGACCCCAPGCCCPCCGACCPCAAGGGCAP
GCPCPCCACCCACCPGACGPCCAPGPPCGAGPACPPGGCACCACCGCGCCGGAAGGGCAGCCAGAPCACC
CAGCAGPCCACCAACCAGAGPCGCAAPCCCGCCACCACCAACCAGACGGAGPPPGAGCGCGPCPPCPGA
SEQ ID NO: 18: cyclin K amino acid sequence 
MKENKENSSPSVTSANLDHTKPCWYWDKKDLAHTPSQLEGLDPATEARYRREGARFIFDVGTRLGLHYDT
LATGIIYFHRFYMFHSFKQFPRYVTGACCLFLAGKVEETPKKCKDIIKTARSLLNDVQFGQFGDDPKEEV
MVLERILLQTIKFDLQVEHPYQFLLKYAKQLKGDKNKIQKLVQMAWTFVNDSLCTTLSLQWEPEIIAVAV
MYLAGRLCKFEIQEWTSKPMYRRWWEQFVQDVPVDVLEDICHQILDLYSQGKQQMPHHTPHQLQQPPSLQ
PTPQVPQVQQSQPSQSSEPSQPQQKDPQQPAQQQQPAQQPKKPSPQPSSPRQVKRAVVVSPKEENKAAEP
PPPKIPKIETTHPPLPPAHPPPDRKPPLAAALGEAEPPGPVDATDLPKVQIPPPAHPAPVHQPPPLPHRP
PPPPPSSYMTGMSTTSSYMSGEGYQSLQSMMKTEGPSYGALPPAYGPPAHLPYHPHVYPPNPPPPPVPPP
PASFPPPAIPPPTPGYPPPPPTYNPNFPPPPPRLPPTHAVPPHPPPGLGLPPASYPPPAVPPGGQPPVPP
PIPPPGMPPVGGLGRAAWMR
SEQ ID NO: 19: cyclin K nucleotide sequence 
ATGAAGGAGAATAAAGAAAATTCAAGCCCTTCAGTAACTTCAGCAAACCTGGACCACACAAAGCCATGTT
GGTACTGGGATAAGAAAGACTTGGCTCATACACCCTCACAACTTGAAGGACTTGATCCAGCCACCGAGGC
CCGGTACCGCCGAGAGGGCGCTCGGTTCATCTTTGATGTGGGCACACGTTTGGGGCTACACTATGATACC
 
CTGGCAACTGGAATAATTTATTTTCATCGCTTCTATATGTTTCATTCCTTCAAGCAATTCCCAAGATATG
TGACAGGAGCCTGTTGCCTCTTTCTGGCTGGGAAAGTAGAAGAAACACCAAAAAAATGTAAAGATATCAT
CAAAACAGCTCGTAGTTTATTAAATGATGTACAATTTGGCCAGTTTGGAGATGACCCAAAGGAGGAAGTA
ATGGTTCTGGAGAGAATCTTACTGCAGACCATCAAGTTTGATTTACAGGTAGAACATCCATACCAGTTCC
TACTAAAATATGCAAAGCAACTCAAAGGTGATAAAAACAAAATTCAAAAGTTGGTTCAAATGGCATGGAC
ATTTGTAAATGACAGTCTCTGCACCACCTTGTCACTGCAGTGGGAACCAGAGATCATAGCAGTAGCAGTG
ATGTATCTCGCAGGACGTTTGTGCAAATTTGAAATACAAGAATGGACCTCCAAACCCATGTATAGGAGAT
GGTGGGAGCAGTTTGTTCAAGATGTCCCGGTCGACGTTTTGGAAGACATCTGCCACCAAATCCTGGATCT
TTACTCACAAGGAAAACAACAGATGCCTCATCACACCCCCCATCAGCTGCAACAGCCCCCATCTCTTCAG
CCTACACCACAAGTGCCGCAAGTACAGCAGTCACAGCCGTCTCAAAGCTCCGAACCATCCCAGCCCCAGC
AGAAGGACCCCCAGCAACCAGCCCAGCAGCAGCAGCCAGCCCAACAGCCCAAGAAACCCTCTCCGCAGCC
CAGTTCTCCCCGACAGGTTAAGCGAGCCGTGGTTGTTTCTCCCAAAGAAGAGAACAAAGCAGCAGAACCA
CCACCACCTAAAATCCCCAAAATTGAGACCACTCATCCACCGTTGCCTCCAGCCCACCCACCTCCAGACC
GGAAGCCTCCCCTCGCTGCTGCCTTAGGTGAGGCTGAGCCGCCGGGCCCTGTGGATGCCACTGACCTCCC
CAAAGTCCAGATTCCCCCTCCGGCCCACCCGGCCCCTGTGCACCAGCCACCGCCGCTGCCACACCGGCCC
CCGCCCCCACCCCCCTCCAGCTACATGACCGGGATGTCCACCACCAGCTCCTACATGTCTGGAGAGGGCT
ACCAGAGCCTGCAGTCCATGATGAAGACCGAGGGACCCTCCTACGGTGCCCTGCCCCCCGCCTACGGCCC
ACCTGCACACCTGCCCTACCACCCCCATGTCTACCCGCCCAACCCGCCCCCGCCACCTGTGCCTCCTCCC
CCAGCCTCCTTCCCCCCACCTGCCATCCCACCCCCTACTCCTGGCTACCCCCCACCCCCACCCACCTACA
ACCCCAACTTCCCACCCCCACCCCCACGCCTCCCGCCTACCCACGCAGTCCCCCCTCATCCTCCTCCAGG
GTTGGGCCTGCCGCCAGCCAGCTACCCACCTCCTGCCGTCCCCCCTGGAGGACAGCCTCCTGTGCCCCCG
CCCATTCCCCCACCCGGCATGCCTCCAGTTGGGGGGCTGGGGCGGGCAGCCTGGATGAGATAA
SEQ ID NO: 20: cyclin T2 amino acid sequence 
MASGRGASSRWFFTREQLENTPSRRCGVEADKELSCRQQAANLIQEMGQRLNVSQLTINTAIVYMHRFYM
HHSFTKFNKNIISSTALFLAAKVEEQARKLEHVIKVAHACLHPLEPLLDTKCDAYLQQTQELVILETIML
QTLGFEITIEHPHTDVVKCTQLVRASKDLAQTSYFMATNSLHLTTFCLQYKPTVIACVCIHLACKWSNWE
IPVSTDGKHWWEYVDPTVTLELLDELTHEFLQILEKTPNRLKKIRNWRANQAARKPKVDGQVSETPLLGS
SLVQNSILVDSVTGVPTNPSFQKPSTSAFPAPVPLNSGNISVQDSHTSDNLSMLATGMPSTSYGLSSHQE
WPQHQDSARTEQLYSQKQETSLSGSQYNINFQQGPSISLHSGLHHRPDKISDHSSVKQEYTHKAGSSKHH
GPISTTPGIIPQKMSLDKYREKRKLETLDLDVRDHYIAAQVEQQHKQGQSQAASSSSVTSPIKMKIPIAN
TEKYMADKKEKSGSLKLRIPIPPTDKSASKEELKMKIKVSSSERHSSSDEGSGKSKHSSPHISRDHKEKH
KEHPSSRHHTSSHKHSHSHSGSSSGGSKHSADGIPPTVLRSPVGLSSDGISSSSSSSRKRLHVNDASHNH
HSKMSKSSKSSGSSSSSSSSSVKQYISSHNSVFNHPLPPPPPVTYQVGYGHLSTLVKLDKKPVETNGPDAN
HEYSTSSQHMDYKDTFDMLDSLLSAQGMNM
SEQ ID NO: 21: cyclin T2 nucleotide sequence 
ATGGCGTCGGGCCGTGGAGCTTCTTCTCGCTGGTTCTTTACTCGGGAACAGCTGGAGAACACGCCGAGCC
GCCGCTGCGGAGTGGAGGCGGATAAAGAGCTCTCGTGCCGCCAGCAGGCGGCCAACCTCATCCAGGAGAT
 
GGGACAGCGTCTCAATGTCTCTCAGCTTACAATAAACACTGCGATTGTTTATATGCACAGGTTTTATATG
CACCATTCTTTCACCAAATTCAACAAAAATATAATATCGTCTACTGCATTATTTTTGGCTGCAAAAGTGG
AAGAACAGGCTCGAAAACTTGAACATGTTATCAAAGTAGCACATGCTTGTCTTCATCCTCTAGAGCCACT
GCTGGATACTAAATGTGATGCTTACCTTCAACAGACTCAAGAACTGGTTATACTTGAAACCATAATGCTA
CAAACTCTAGGTTTTGAGATCACCATTGAACACCCACACACAGATGTGGTGAAATGTACCCAGTTAGTAA
GAGCAAGCAAGGATTTGGCACAGACATCCTATTTCATGGCTACCAACAGTCTGCATCTTACAACCTTCTG
TCTTCAGTACAAACCAACAGTGATAGCATGTGTATGCATTCATTTGGCTTGCAAATGGTCCAATTGGGAG
ATCCCTGTATCAACTGATGGAAAGCATTGGTGGGAATATGTGGATCCTACAGTTACTCTAGAATTATTAG
ATGAGCTAACACATGAGTTTCTACAAATATTGGAGAAAACGCCTAATAGGTTGAAGAAGATTCGAAACTG
GAGGGCTAATCAGGCAGCTAGGAAACCAAAAGTAGATGGACAGGTATCAGAGACACCACTTCTTGGTTCA
TCTTTGGTCCAGAATTCCATTTTAGTAGATAGTGTCACTGGTGTGCCTACAAACCCAAGTTTTCAGAAAC
CATCTACATCAGCATTCCCTGCGCCAGTACCTCTAAATTCAGGAAATATTTCTGTTCAAGACAGCCATAC
ATCTGATAATTTGTCAATGCTAGCAACAGGAATGCCAAGTACTTCATACGGTTTATCATCACACCAGGAA
TGGCCTCAACATCAAGACTCAGCAAGGACAGAACAGCTATATTCACAGAAACAGGAGACATCTTTGTCTG
GTAGCCAGTACAACATCAACTTCCAGCAGGGACCTTCTATATCACTGCATTCAGGATTACATCACAGACC
TGACAAAATTTCAGATCATTCTTCTGTTAAGCAAGAATATACTCATAAAGCAGGGAGCAGTAAACACCAT
GGGCCAATTTCCACTACTCCAGGAATAATTCCTCAGAAAATGTCTTTAGATAAATATAGAGAAAAGCGTA
AACTAGAAACTCTTGATCTCGATGTAAGGGATCATTATATAGCTGCCCAGGTAGAACAGCAGCACAAACA
AGGGCAGTCACAGGCAGCCAGCAGCAGTTCTGTTACTTCTCCCATTAAAATGAAAATACCTATCGCAAAT
ACTGAAAAATACATGGCAGACAAAAAGGAAAAGAGTGGGTCACTGAAATTACGGATTCCAATACCACCCA
CTGATAAAAGCGCCAGTAAAGAAGAACTGAAAATGAAAATAAAAGTTTCTTCTTCAGAAAGACACAGCTC
TTCTGATGAAGGCAGTGGGAAGAGCAAACATTCAAGCCCACATATTAGCAGAGACCATAAGGAGAAGCAC
AAGGAGCATCCTTCAAGCCGCCACCACACCAGCAGCCACAAGCATTCCCACTCGCATAGTGGCAGCAGCA
GCGGTGGCAGTAAACACAGTGCCGACGGAATACCACCCACTGTTCTGAGGAGTCCTGTTGGCCTGAGCAG
TGATGGCATTTCCTCTAGCTCCAGCTCTTCAAGGAAGAGGCTGCATGTCAATGATGCATCTCACAACCAC
CACTCCAAAATGAGCAAAAGTTCCAAAAGTTCAGGTAGTTCATCTAGTTCTTCCTCCTCTGTTAAGCAGT
ATATATCCTCTCACAACTCTGTTTTTAACCATCCCTTACCCCCTCCTCCCCCTGTCACATACCAGGTGGG
CTACGGACATCTCAGCACCCTCGTGAAACTGGACAAGAAGCCAGTGGAGACCAACGGTCCTGATGCCAAT
CACGAGTACAGTACAAGCAGCCAGCATATGGACTACAAAGACACATTCGACATGCTGGACTCACTGTTAA
GTGCCCAAGGAATGAACATGTAA
SEQ ID NO: 22: I-myc nucleotide sequence 
ATGTGCGTGTGTGCTGGCTGCCGGGCTGCCCCGAGCCGGCGGGGAGCCGGTCCGCTCCAGGTGGCGGGCG
GCTGGAGCGAGGGAGCGGACATGGACTACGACTCGTACCAGCACTATTTCTACGACTATGACTGCGGGGA
GGATTTCTACCGCTCCACGGCGCCCAGCGAGGACATCTGGAAGAAATTCGAGCTGGTGCCATCGCCCCCC
ACGTCGCCGCCCTGGGGCTTGGGTCCCGGCGCAGGGGACCCGGCCCCCGGGATTGGTCCCCCGGAGCCGT
GGCCCGGAGGGTGCACCGGAGACGAAGCGGAATCCCGGGGCCACTCGAAAGGCTGGGGCAGGAACTACGC
CTCCATCATACGCCGTGACTGCATGTGGAGCGGCTTCTCGGCCCGGGAACGGCTGGAGAGAGCTGTGAGC
GACCGGCTCGCTCCTGGCGCGCCCCGGGGGAACCCGCCCAAGGCGTCCGCCGCCCCGGACTGCACTCCCA
 
GCCTCGAAGCCGGCAACCCGGCGCCCGCCGCCCCCTGTCCGCTGGGCGAACCCAAGACCCAGGCCTGCTC
CGGGTCCGAGAGCCCAAGCGACTCGGAGAATGAAGAAATTGATGTTGTGACAGTAGAGAAGAGGCAGTCT
CTGGGTATTCGGAAGCCGGTCACCATCACGGTGCGAGCAGACCCCCTGGATCCCTGCATGAAGCATTTCC
ACATCTCCATCCATCAGCAACAGCACAACTATGCTGCCCGTTTTCCTCCAGAAAGCTGCTCCCAAGAAGA
GGCTTCAGAGAGGGGTCCCCAAGAAGAGGTTCTGGAGAGAGATGCTGCAGGGGAAAAGGAAGATGAGGAG
GATGAAGAGATTGTGAGTCCCCCACCTGTAGAAAGTGAGGCTGCCCAGTCCTGCCACCCCAAACCTGTCA
GTTCTGATACTGAGGATGTGACCAAGAGGAAGAATCACAACTTCCTGGAGCGCAAGAGGCGGAATGACCT
GCGTTCGCGATTCTTGGCGCTGAGGGACCAGGTGCCCACCCTGGCCAGCTGCTCCAAGGCCCCCAAAGTA
GTGATCCTAAGCAAGGCCTTGGAATACTTGCAAGCCCTGGTGGGGGCTGAGAAGAGGATGGCTACAGAGA
AAAGACAGCTCCGATGCCGGCAGCAGCAGTTGCAGAAAAGAATTGCATACCTCACTGGCTACTAA
SEQ ID NO: 23: I-myc amino acid sequence 
MCVCAGCRAAPSRRGAGPLQVAGGWSEGADMDYDSYQHYFYDYDCGEDFYRSTAPSEDIWKKFELVPSPP
TSPPWGLGPGAGDPAPGIGPPEPWPGGCTGDEAESRGHSKGWGRNYASIIRRDCMWSGFSARERLERAVS
DRLAPGAPRGNPPKASAAPDCTPSLEAGNPAPAAPCPLGEPKTQACSGSESPSDSENEEIDVVTVEKRQS
LGIRKPVTITVRADPLDPCMKHFHISIHQQQHNYAARFPPESCSQEEASERGPQEEVLERDAAGEKEDEE
DEEIVSPPPVESEAAQSCHPKPVSSDTEDVTKRKNHNFLERKRRNDLRSRFLALRDQVPTLASCSKAPKV
VILSKALEYLQALVGAEKRMATEKRQLRCRQQQLQKRIAYLTGY
SEQ ID NO 24: n-myc nucleotide sequence 
ATGCCGAGCTGCTCCACGTCCACCATGCCGGGCATGATCTGCAAGAACCCAGACCTCGAGTTTGACTCGC
TACAGCCCTGCTTCTACCCGGACGAAGATGACTTCTACTTCGGCGGCCCCGACTCGACCCCCCCGGGGGA
GGACATCTGGAAGAAGTTTGAGCTGCTGCCCACGCCCCCGCTGTCGCCCAGCCGTGGCTTCGCGGAGCAC
AGCTCCGAGCCCCCGAGCTGGGTCACGGAGATGCTGCTTGAGAACGAGCTGTGGGGCAGCCCGGCCGAGG
AGGACGCGTTCGGCCTGGGGGGACTGGGTGGCCTCACCCCCAACCCGGTCATCCTCCAGGACTGCATGTG
GAGCGGCTTCTCCGCCCGCGAGAAGCTGGAGCGCGCCGTGAGCGAGAAGCTGCAGCACGGCCGCGGGCCG
CCAACCGCCGGTTCCACCGCCCAGTCCCCGGGAGCCGGCGCCGCCAGCCCTGCGGGTCGCGGGCACGGCG
GGGCTGCGGGAGCCGGCCGCGCCGGGGCCGCCCTGCCCGCCGAGCTCGCCCACCCGGCCGCCGAGTGCGT
GGATCCCGCCGTGGTCTTCCCCTTTCCCGTGAACAAGCGCGAGCCAGCGCCCGTGCCCGCAGCCCCGGCC
AGTGCCCCGGCGGCGGGCCCTGCGGTCGCCTCGGGGGCGGGTATTGCCGCCCCAGCCGGGGCCCCGGGGG
TCGCCCCTCCGCGCCCAGGCGGCCGCCAGACCAGCGGCGGCGACCACAAGGCCCTCAGTACCTCCGGAGA
GGACACCCTGAGCGATTCAGATGATGAAGATGATGAAGAGGAAGATGAAGAGGAAGAAATCGACGTGGTC
ACTGTGGAGAAGCGGCGTTCCTCCTCCAACACCAAGGCTGTCACCACATTCACCATCACTGTGCGTCCCA
AGAACGCAGCCCTGGGTCCCGGGAGGGCTCAGTCCAGCGAGCTGATCCTCAAACGATGCCTTCCCATCCA
CCAGCAGCACAACTATGCCGCCCCCTCTCCCTACGTGGAGAGTGAGGATGCACCCCCACAGAAGAAGATA
AAGAGCGAGGCGTCCCCACGTCCGCTCAAGAGTGTCATCCCCCCAAAGGCTAAGAGCTTGAGCCCCCGAA
ACTCTGACTCGGAGGACAGTGAGCGTCGCAGAAACCACAACATCCTGGAGCGCCAGCGCCGCAACGACCT
TCGGTCCAGCTTTCTCACGCTCAGGGACCACGTGCCGGAGTTGGTAAAGAATGAGAAGGCCGCCAAGGTG
 
GTCATTTTGAAAAAGGCCACTGAGTATGTCCACTCCCTCCAGGCCGAGGAGCACCAGCTTTTGCTGGAAA
AGGAAAAATTGCAGGCAAGACAGCAGCAGTTGCTAAAGAAAATTGAACACGCTCGGACTTGCTAG
SEQ ID NO: 25: n-myc amino acid sequence
SEQ ID NO 26: P2A amino acid sequence
   ATNFSLLKQAGDVEENPGP
SEQ ID NO 27: E2A amino acid sequence
   QCTNYALLKLAGDV ESNPGP
SEQ ID NO 28: F2A amino acid sequence
   VKQTLNFDLLKLAGDVESNPGP
SEQ ID NO 29: T2A amino acid sequence
   EGRGSLLTCGDVEENPGP
SEQ ID NO: 30 - guide sequence targeting Larp7
   ATCCGGAGGAAAAAACCTC
SEQ ID NO: 31 - guide sequence targeting Larp7
   AGTCTACTGGAGATCCAAA
SEQ ID NO: 32 - guide sequence targeting Larp7
   AAGAAAGGCCGAATGAAAA
SEQ ID NO: 33 - guide sequence targeting Larp7
   CTTACCTGAAGTCAGAACA
SEQ ID NO: 34 - guide sequence targeting Larp7
   ACTTTTGATCGGGGAGCTA
SEQ ID NO: 35 - Larp7 amino acid sequence 
MIPNIEGMETESGNQEKVMEEESTEKKKEVEKKKRSRVKQVLADIAKQVDFWFGDANLHKDRFLREQIEK
SRDGYVDISLLVSFNKMKKLTTDGKLIARALRSSAVVELDLEGTRIRRKKPLGERPKDEDERTVYVELLP
KNVNHSWIERVFGKCGNVVYISIPHYKSTGDPKGFAFVEFETKEQAAKAIEFLNNPPEEAPRKPGIFPKT
VKNKPIPALRVVEEKKKKKKKKGRMKKEDNIQAKEENMDTSNTSISKMKRSRPTSEGSDIESTEPQKQCS
KKKKKRDRVEASSLPEVRTGKRKRSSSEDAESLAPRSKVKKIIQKDIIKEASEASKENRDIEISTEEEKD
TGDLKDSSLLKTKRKHKKKHKERHKMGEEVIPLRVLSKSEWMDLKKEYLALQKASMASLKKTISQIKSES
 
EMETDSGVPQNTGMKNEKTANREECRTQEKVNATGPQFVSGVIVKIISTEPLPGRKQVRDTLAAISEVLY
VDLLEGDTECHARFKTPEDAQAVINAYTEINKKHCWKLEILSGDHEQRYWQKILVDRQAKLNQPREKKRG
TEKLITKAEKIRLAKTQQASKHIRFSEYD
SEQ ID NO: 36 - Larp7 nucleotide sequence 
ATGATCCCTAACATAGAAGGAATGGAAACTGAAAGTGGAAATCAGGAAAAGGTAATGGAAGAAAGCA
CTGAAAAGAAAAAAGAAGTTGAAAAAAAGAAACGGTCACGAGTTAAACAGGTGCTTGCAGATATTGCTAA
GCAAGTGGACTTCTGGTTTGGGGATGCAAATCTTCACAAGGATAGATTTCTTCGAGAACAGATAGAAAAA
TCTAGAGATGGATATGTTGATATATCACTACTTGTGTCTTTTAACAAAATGAAAAAATTGACTACTGATG
GGAAGTTAATTGCCAGAGCATTGAGAAGTTCAGCTGTTGTAGAGCTTGATTTGGAAGGCACCAGAATCCG
GAGGAAAAAACCTCTGGGGGAAAGACCAAAGGATGAGGATGAACGCACAGTGTATGTGGAGTTACTTCCC
AAAAATGTTAATCACAGCTGGATTGAAAGAGTATTTGGGAAATGTGGCAATGTTGTTTATATAAGTATAC
CACATTATAAGTCTACTGGAGATCCAAAGGGATTTGCGTTTGTGGAATTTGAAACAAAAGAACAAGCAGC
AAAAGCAATTGAGTTTCTTAACAACCCACCAGAAGAAGCACCAAGAAAACCTGGCATATTTCCTAAAACA
GTGAAAAATAAGCCCATTCCAGCCTTAAGAGTTGTGGAAGAGAAGAAAAAGAAAAAGAAGAAGAAAGGCC
GAATGAAAAAGGAAGACAATATCCAAGCCAAAGAAGAAAACATGGACACAAGCAACACCAGCATCAGTAA
AATGAAAAGATCCAGACCCACATCTGAGGGCTCTGACATTGAGTCCACTGAACCCCAAAAGCAGTGCTCA
AAGAAAAAGAAAAAACGGGACAGAGTTGAAGCATCTAGCTTACCTGAAGTCAGAACAGGGAAGAGGAAGA
GAAGCAGCTCTGAAGATGCAGAATCCCTAGCTCCCCGATCAAAAGTAAAGAAAATTATTCAGAAAGACAT
CATTAAGGAAGCATCAGAAGCTTCCAAGGAAAATAGAGATATAGAAATCTCTACTGAAGAGGAAAAGGAT
ACTGGAGATCTAAAAGATAGCTCTCTCTTGAAAACAAAAAGGAAACATAAGAAAAAACATAAAGAGAGAC
ATAAAATGGGAGAAGAAGTTATACCATTAAGAGTGCTATCAAAGAGCGAATGGATGGATTTGAAAAAAGA
GTATTTAGCGCTACAAAAAGCTAGCATGGCTTCTTTAAAAAAAACAATATCCCAAATAAAATCAGAGTCA
GAAATGGAAACAGACAGTGGAGTACCTCAAAACACTGGAATGAAAAATGAAAAAACAGCCAACAGGGAAG
AGTGTCGCACCCAGGAGAAAGTTAATGCAACAGGACCACAGTTCGTGAGTGGAGTGATTGTGAAGATCAT
TAGCACAGAGCCTCTACCTGGCAGGAAACAAGTCCGGGATACTTTGGCAGCAATCTCAGAAGTTCTTTAT
GTTGATTTGCTAGAAGGGGATACAGAATGCCATGCTAGATTTAAAACTCCTGAGGATGCTCAAGCAGTAA
TAAATGCCTATACAGAAATTAACAAGAAACACTGCTGGAAACTCGAGATCCTTTCTGGTGATCACGAACA
AAGGTATTGGCAGAAGATTTTGGTTGATAGACAGGCAAAACTTAATCAGCCTCGGGAAAAGAAAAGAGGC
ACTGAAAAGTTAATCACCAAAGCTGAAAAGATTAGACTGGCAAAGACTCAACAAGCGAGTAAACATATAA
GATTTTCTGAATATGATTGA
SEQ ID NO: 37: c-Myc - pseudouridine* (Denoted by P) 
CPGGAPPPPPPCGGGPAGPGGAAAACCAGCAGCCPCCCGCGACGAPGCCCCPCAACGPPAGCPPCACCA
ACAGGAACPAPGACCPCGACPACGACPCGGPGCAGCCGPAPPPCPACPGCGACGAGGAGGAGAACPPCPA
CCAGCAGCAGCAGCAGAGCGAGCPGCAGCCCCGGCGCCCAGCGAGGAPAPCPGGAAGAAAPPCGAGCPG
CPGCCCACCCCGCCCCPGPCCCCPAGCCGCCGCPCCGGGCPCPGCPCGCCCPCCPACGPPGCGGPCACAC
CCPPCPCCCPPCGGGGAGACAACGACGGCGGPGGCGGGAGCPPCPCCACGGCCGACCAGCPGGAGAPGGP
GACCGAGCPGCPGGGAGGAGACAPGGPGAACCAGAGPPPCAPCPGCGACCCGGACGACGAGACCPPCAPC
 
AAAAACAPCAPCAPCCAGGACPGPAPGPGGAGCGGCPPCPCGGCCGCCGCCAAGCPCGPCPCAGAGAAGC
PGGCCPCCPACCAGGCPGCGCGCAAAGACAGCGGCAGCCCGAACCCCGCCCGCGGCCACAGCGPCPGCPC
CACCPCCAGCPPGPACCPGCAGGAPCPGAGCGCCGCCGCCPCAGAGPGCAPCGACCCCPCGGPGGPCPPC
CCCPACCCPCPCAACGACAGCAGCPCGCCCAAGPCCPGCGCCPCGCAAGACPCCAGCGCCPPCPCPCCGP
CCPCGGAPPCPCPGCPCPCCPCGACGGAGPCCPCCCCGCAGGGCAGCCCCGAGCCCCPGGPGCPCCAPGA
GGAGACACCGCCCACCACCAGCAGCGACPCPGAGGAGGAACAAGAAGAPGAGGAAGAAAPCGAPGPPGPP
PCPGPGGAAAAGAGGCAGGCPCCPGGCAAAAGGPCAGAGPCPGGAPCACCPPCPGCPGGAGGCCACAGCA
AACCPCCPCACAGCCCACPGGPCCPCAAGAGGPGCCACGPCPCCACACAPCAGCACAACPACGCAGCGCC
PCCCPCCACPCGGAAGGACPAPCCPGCPGCCAAGAGGGPCAAGPPGGACAGPGPCAGAGPCCPGAGACAG
APCAGCAACAACCGAAAAPGCACCAGCCCCAGGPCCPCGGACACCGAGGAGAAPGPCAAGAGGCGAACAC
ACAACGPCPPGGAGCGCCAGAGGAGGAACGAGCPAAAACGGAGCPPPPPGCCCPGCGPGACCAGAPCCC
GGAGPPGGAAAACAAPGAAAGGCCCCCAAGGPAGPPAPCCPPAAAAAGCCACAGCAPACAPCCPGPCC
GPCCAAGCAGAGGAGCAAAAGCPCAPPPCPGAAGAGGACPPGPPGCGGAAACGACGAGAACAGPPGAAAC
ACAAACPPGAACAGCPACGGAACPCPPGPGCGPAA
SEQ ID NO: 38: 5'UTR sequence
   GGGAAATAAGAGAGAAAAGAAGAGTAAGAAGAAATATAAGAGCCACC

## Claims

1. A nucleic acid molecule comprising a nucleic acid sequence encoding a myc transcription factor selected from c-myc, I-myc or n-myc and a cyclin T1 protein for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

2. The nucleic acid molecule for use according to claim 1, wherein the nucleic acid molecule is a ribonucleotide molecule, preferably a mRNA molecule, and wherein the mRNA molecule comprises at least one modification selected from a cap modification, a tail modification, a nucleoside modification and an untranslated region (UTR) modification.

3. The nucleic acid molecule for use according to claim 2, wherein the mRNA molecule encodes a myc transcription factor as defined in SEQ ID NO: 11 or a functional variant thereof, wherein the functional variant retains the transcription factor activity of the full non-variant sequence and has at least 75% overall sequence identity to SEQ ID NO: 11, and a cyclin T1 protein as defined in SEQ ID NO: 5 or a functional variant thereof, wherein the functional variant retains the biological function of the full non-variant sequence and has at least 75% overall sequence identity to SEQ ID NO: 5.

4. The nucleic acid molecule for use according to claim 1, wherein the nucleic acid molecule is a nucleic acid construct or vector.

5. The nucleic acid molecule for use according to claim 4, wherein the nucleic acid sequence encodes an inducible myc transcription factor as defined in SEQ ID NO: 1 or 10 or a functional variant thereof, wherein the functional variant retains the transcription factor activity of the full non-variant sequence and has at least 75% overall sequence identity to SEQ ID NO: 1 or 10, operably linked to a first regulatory sequence; and a nucleic acid sequence encoding a cyclin T1 as defined in SEQ ID NO: 5 or a functional variant thereof, wherein the functional variant retains the biological function of the full non-variant sequence and has at least 75% overall sequence identity to SEQ ID NO: 5, operably linked to the first regulatory sequence or a second regulatory sequence.

6. The nucleic acid molecule for use according to claim 5, wherein the regulatory sequence is a promoter, preferably the CAG promoter.

7. The nucleic acid molecule for use according to any of claims 4 to 6, wherein the vector is a viral vector, preferably an adeno-associated viral vector.

8. A composition comprising the nucleic acid molecule of any of claims 1 to 7 and a pharmaceutically acceptable carrier for use as a medicament.

9. A composition comprising a first modified mRNA molecule and at least a second modified mRNA molecule, wherein the first modified mRNA molecule encodes a myc transcription factor selected from c-myc, I-myc or n-myc and the second modified mRNA molecule encodes a cyclin T1 protein, wherein the modification is selected from at least one of a cap modification, a tail modification, a nucleoside modification and a untranslated region (UTR) modification for use as a medicament, and wherein the first modified mRNA molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

10. A composition comprising a first vector and at least a second vector, wherein the first vector comprises a nucleic acid sequence encoding an inducible myc transcription factor selected from c-myc, n-myc or I-myc operably linked to a regulatory sequence and the second vector comprises a nucleic acid sequence encoding a cyclin T1 protein operably linked to a regulatory sequence for use as a medicament, wherein the nucleic acid molecule encodes a myc transcription factor as defined in SEQ ID NO: 11, 23 or 25 or a functional variant thereof, wherein the functional variant retains the transcription factor activity of the full non-variant sequence and has at least 75% overall sequence identity to SEQ ID NO: 11, 23 or 25.

11. The nucleic acid molecule of any of claims 1 to 7 or the composition of any of claims 8 to 10 for use in the treatment of a condition **characterised by** cell loss, wherein the condition is selected from myocardial infarction or reduced ejection fraction of the heart.

12. A method of increasing at least one of cell proliferation, mitosis and cytokinesis in a cell *in vitro,* the method comprising introducing the nucleic acid molecule of any of claims 1 to 7 or the composition of any of claims 8 to 10 to the cell, wherein the cell is a heart cell.

13. A method of increasing organ size *in vitro,* the method comprising introducing the nucleic acid molecule of any of claims 1 to 7 or a composition of any of claims 8 to 10 to the organ, wherein the organ is a heart.

14. A host cell comprising the nucleic acid molecule of any of claims 1 to 7 or the composition of claim 8, wherein the host cell is a heart cell.

15. A nanoparticle comprising a nucleic acid molecule of any of claims 1 to 7.

## Patentansprüche

1. Nukleinsäuremolekül, umfassend eine Nukleinsäuresequenz, die für einen Myc-Transkriptionsfaktor, der aus c-Myc, I-Myc oder n-Myc ausgewählt ist, und ein Cyclin-T1-Protein zur Verwendung als Medikament kodiert, wobei das Nukleinsäuremolekül für einen Myc-Transkriptionsfaktor, wie in SEQ ID NO. 11, 23 oder 25 definiert, oder für eine funktionelle Variante davon kodiert, wobei die funktionelle Variante die Transkriptionsfaktoraktivität der vollständigen nicht-varianten Sequenz beibehält und eine Gesamtsequenzidentität von mindestens 75 % zu SEQ ID NO. 11, 23 oder 25 aufweist.

2. Nukleinsäuremolekül zur Verwendung nach Anspruch 1, wobei das Nukleinsäuremolekül ein Ribonukleotidmolekül ist, vorzugsweise ein mRNA-Molekül, und wobei das mRNA-Molekül mindestens eine Modifikation umfasst, die aus einer Cap-Modifikation, einer Tail-Modifikation, einer Nukleosid-Modifikation und einer Modifikation der untranslatierten Region (UTR) ausgewählt ist.

3. Nukleinsäuremolekül zur Verwendung nach Anspruch 2, wobei das mRNA-Molekül für einen Myc-Transkriptionsfaktor, wie in SEQ ID NO. 11 definiert, oder eine funktionelle Variante davon kodiert, wobei die funktionelle Variante die Transkriptionsfaktoraktivität der vollständigen nicht-varianten Sequenz beibehält und eine Gesamtsequenzidentität von mindestens 75 % zu SEQ ID NO. 11 aufweist, und für ein Cyclin-T-Protein, wie in SEQ ID NO. 5 definiert, oder eine funktionelle Variante davon kodiert, wobei die funktionelle Variante die biologische Funktion der vollständigen nicht-varianten Sequenz beibehält und eine Gesamtsequenzidentität von mindestens 75 % zu SEQ ID NO. 5 aufweist.

4. Nukleinsäuremolekül zur Verwendung nach Anspruch 1, wobei das Nukleinsäuremolekül ein Nukleinsäurekonstrukt oder Vektor ist.

5. Nukleinsäuremolekül zur Verwendung nach Anspruch 4, wobei die Nukleinsäuresequenz für einen induzierbaren Myc-Transkriptionsfaktor, wie in SEQ ID NO. 1 oder 10 definiert, oder eine funktionelle Variante davon kodiert, wobei die funktionelle Variante die Transkriptionsfaktoraktivität der vollständigen, nicht-varianten Sequenz beibehält und eine Gesamtsequenzidentität von mindestens 75 % zu SEQ ID NO. 1 oder 10 aufweist, die funktionsfähig mit einer ersten regulatorischen Sequenz verknüpft ist; und eine Nukleinsäuresequenz für ein Cyclin T1, wie in SEQ ID NO. 5 definiert, oder eine funktionelle Variante davon kodiert, wobei die funktionelle Variante die biologische Funktion der vollständigen, nicht-varianten Sequenz beibehält und eine Gesamtsequenzidentität von mindestens 75 % zu SEQ ID NO. 5 aufweist, die funktionsfähig mit der ersten oder einer zweiten regulatorischen Sequenz verknüpft ist.

6. Nukleinsäuremolekül zur Verwendung nach Anspruch 5, wobei die regulatorische Sequenz ein Promotor ist, vorzugsweise der CAG-Promotor.

7. Nukleinsäuremolekül zur Verwendung nach Ansprüchen 4 bis 6, wobei der Vektor ein viraler Vektor, vorzugsweise ein adeno-assoziierter viraler Vektor, ist.

8. Zusammensetzung, umfassend das Nukleinsäuremolekül nach einem der Ansprüche 1 bis 7 und einen pharmazeutisch verträglichen Träger zur Verwendung als Medikament.

9. Zusammensetzung, umfassend ein erstes modifiziertes mRNA-Molekül und mindestens ein zweites modifiziertes mRNA-Molekül, wobei das erste modifizierte mRNA-Molekül für einen Myc-Transkriptionsfaktor, der aus c-Myc, L-Myc oder N-Myc ausgewählt ist, kodiert und das zweite modifizierte mRNA-Molekül für ein Cyclin-T1-Protein kodiert, wobei die Modifikation ausgewählt ist aus mindestens einer von Cap-Modifikation, Tail-Modifikation, Nukleosid-Modifikation und Modifikation der untranslatierten Region (UTR) zur Verwendung als Medikament, und wobei das erste modifizierte mRNA-Molekül für einen Myc-Transkriptionsfaktor, wie in SEQ ID NO. 11, 23 oder 25 definiert, oder eine funktionelle Variante davon kodiert, wobei die funktionelle Variante die Transkriptionsfaktoraktivität der vollständigen, nicht-varianten Sequenz beibehält und eine Gesamtsequenzidentität von mindestens 75 % zu SEQ ID NO. 11, 23 oder 25 aufweist.

10. Zusammensetzung, umfassend einen ersten Vektor und mindestens einen zweiten Vektor, wobei der erste Vektor eine Nukleinsäuresequenz umfasst, die für einen induzierbaren Myc-Transkriptionsfaktor, der aus c-Myc, n-Myc oder I-Myc ausgewählt ist, kodiert und mit einer regulatorischen Sequenz verknüpft ist, und der zweite Vektor eine Nukleinsäuresequenz umfasst, die für ein Cyclin-T1-Protein kodiert, das funktionsfähig mit einer regulatorischen Sequenz zur Verwendung als Medikament verknüpft ist, wobei das Nukleinsäuremolekül für einen Myc-Transkriptionsfaktor, wie in SEQ ID NO. 11, 23 oder 25 definiert, oder eine funktionelle Variante davon kodiert, wobei die funktionelle Variante die Transkriptionsfaktoraktivität der vollständigen, nicht-varianten Sequenz beibehält und eine Gesamtsequenzidentität von mindestens 75 % zu SEQ ID NO. 11, 23 oder 25 aufweist.

11. Nukleinsäuremolekül nach einem der Ansprüche 1 bis 7 oder Zusammensetzung nach einem der Ansprüche 8 bis 10 zur Verwendung bei der Behandlung einer Erkrankung, die durch Zellverlust gekennzeichnet ist, wobei die Erkrankung aus Myokardinfarkt oder verminderter Ejektionsfraktion des Herzens ausgewählt ist.

12. Verfahren zur Vergrößerung mindestens einer von Zellproliferation, Mitose oder Zytokinese in einer Zelle *in vitro,* wobei das Verfahren das Einbringen des Nukleinsäuremoleküls nach einem der Ansprüche 1 bis 7 oder der Zusammensetzung nach einem der Ansprüche 8 bis 10 in die Zelle umfasst, wobei die Zelle eine Herzzelle ist.

13. Verfahren zur Vergrößerung einer Organgröße *in vitro,* wobei das Verfahren das Einbringen des Nukleinsäuremoleküls nach einem der Ansprüche 1 bis 7 oder einer Zusammensetzung nach einem der Ansprüche 8 bis 10 in das Organ umfasst, wobei das Organ ein Herz ist.

14. Wirtszelle, umfassend das Nukleinsäuremolekül nach einem der Ansprüche 1 bis 7 oder die Zusammensetzung nach Anspruch 8, wobei die Wirtszelle eine Herzzelle ist.

15. Nanopartikel, umfassend ein Nukleinsäuremolekül nach einem der Ansprüche 1 bis 7.

## Revendications

1. Molécule d'acide nucléique comprenant une séquence d'acide nucléique codant un facteur de transcription myc sélectionné parmi c-myc, l-myc ou n-myc et une protéine cycline T1 pour une utilisation en tant que médicament, dans laquelle la molécule d'acide nucléique code un facteur de transcription myc tel que défini dans la SEQ ID NO : 11, 23 ou 25 ou une variante fonctionnelle de celui-ci, dans laquelle la variante fonctionnelle conserve l'activité de facteur de transcription de la séquence complète non variante et présente une identité de séquence globale d'au moins 75 % avec la SEQ ID NO : 11, 23 ou 25.

2. Molécule d'acide nucléique à utiliser selon la revendication 1, dans laquelle la molécule d'acide nucléique est une molécule de ribonucléotide, de préférence une molécule d'ARNm, et dans laquelle la molécule d'ARNm comprend au moins une modification choisie parmi une modification de coiffe, une modification de queue, une modification de nucléoside et une modification de région non traduite (UTR).

3. Molécule d'acide nucléique à utiliser selon la revendication 2, dans laquelle la molécule d'ARNm code un facteur de transcription myc tel que défini dans la SEQ ID NO : 11 ou une variante fonctionnelle de celui-ci, dans laquelle la variante fonctionnelle conserve l'activité de facteur de transcription de la séquence complète non variante et présente une identité de séquence globale d'au moins 75 % avec la SEQ ID NO : 11, et une protéine cycline T1 telle que définie dans la SEQ ID NO : 5 ou une variante fonctionnelle de celle-ci, dans laquelle la variante fonctionnelle conserve la fonction biologique de la séquence complète non variante et présente une identité de séquence globale d'au moins 75 % avec la SEQ ID NO : 5.

4. Molécule d'acide nucléique à utiliser selon la revendication 1, dans laquelle la molécule d'acide nucléique est un produit de recombinaison ou un vecteur d'acide nucléique.

5. Molécule d'acide nucléique à utiliser selon la revendication 4, dans laquelle la séquence d'acide nucléique code pour un facteur de transcription myc inductible tel que défini dans la SEQ ID N° 1 ou 10, ou une variante fonctionnelle de celui-ci, dans laquelle la variante fonctionnelle conserve l'activité de facteur de transcription de la séquence complète non variante et présente une identité de séquence globale d'au moins 75 % avec la SEQ ID N° 1 ou 10, liée de manière fonctionnelle à une première séquence régulatrice ; et une séquence d'acide nucléique codant pour une cycline T1 telle que définie dans la séquence d'identification n° 5, ou une variante fonctionnelle de celle-ci, dans laquelle la variante fonctionnelle conserve la fonction biologique de la séquence complète non variante et présente une identité de séquence globale d'au moins 75 % avec la SEQ ID N° 5, liée de manière fonctionnelle à la première séquence régulatrice ou à une seconde séquence régulatrice.

6. Molécule d'acide nucléique à utiliser selon la revendication 5, dans laquelle la séquence régulatrice est un promoteur, de préférence le promoteur CAG.

7. Molécule d'acide nucléique à utiliser selon les revendications 4 à 6, dans laquelle le vecteur est un vecteur viral, de préférence un vecteur viral adéno-associé.

8. Composition comprenant la molécule d'acide nucléique selon l'une quelconque des revendications 1 à 7 et un support pharmaceutiquement acceptable pour une utilisation en tant que médicament.

9. Composition comprenant une première molécule d'ARNm modifiée et au moins une seconde molécule d'ARNm modifiée, dans laquelle la première molécule d'ARNm modifiée code pour un facteur de transcription myc choisi parmi c-myc, l-myc ou n-myc et la seconde molécule d'ARNm modifiée code pour une protéine cycline T1, dans laquelle la modification est choisie parmi au moins une modification de coiffe, une modification de queue, une modification de nucléoside et une modification de région non traduite (UTR) pour une utilisation en tant que médicament, et la première molécule d'ARNm modifiée code pour un facteur de transcription myc tel que défini dans les SEQ ID NO : 11, 23 ou 25 ou une variante fonctionnelle de celui-ci, dans laquelle la variante fonctionnelle conserve l'activité de facteur de transcription de la séquence complète non variante et présente une identité de séquence globale d'au moins 75 % avec les SEQ ID NO : 11, 23 ou 25.

10. Composition comprenant un premier vecteur et au moins un second vecteur, dans laquelle le premier vecteur comprend une séquence d'acide nucléique codant pour un facteur de transcription myc inductible choisi parmi c-myc, n-myc ou l-myc, lié de manière fonctionnelle à une séquence régulatrice, et le second vecteur comprenant une séquence d'acide nucléique codant pour une protéine cycline T1, liée de manière fonctionnelle à une séquence régulatrice, pour une utilisation en tant que médicament, dans laquelle la molécule d'acide nucléique code pour un facteur de transcription myc tel que défini dans les SEQ ID NO : 11, 23 ou 25, ou pour une variante fonctionnelle de celui-ci, dans laquelle la variante fonctionnelle conserve l'activité de facteur de transcription de la séquence complète non variante et présente une identité de séquence globale d'au moins 75 % avec les SEQ ID NO : 11, 23 ou 25.

11. Molécule d'acide nucléique selon l'une quelconque des revendications 1 à 7 ou composition selon l'une quelconque des revendications 8 à 10 à utiliser dans le traitement d'une affection **caractérisée par** une perte cellulaire, dans laquelle l'affection est choisie parmi l'infarctus du myocarde ou la fraction d'éjection réduite du cœur.

12. Procédé d'augmentation d'au moins un des processus suivants : prolifération cellulaire, mitose et cytokinèse dans une cellule *in vitro,* le procédé comprenant l'introduction dans la cellule de la molécule d'acide nucléique selon l'une quelconque des revendications 1 à 7 ou de la composition selon l'une quelconque des revendications 8 à 10, dans lequel la cellule est une cellule cardiaque.

13. Procédé d'augmentation de la taille d'un organe *in vitro,* le procédé comprenant l'introduction dans l'organe de la molécule d'acide nucléique selon l'une quelconque des revendications 1 à 7 ou d'une composition selon l'une quelconque des revendications 8 à 10, dans lequel l'organe est un cœur.

14. Cellule hôte comprenant la molécule d'acide nucléique selon l'une quelconque des revendications 1 à 7 ou la composition selon la revendication 8, dans laquelle la cellule hôte est une cellule cardiaque.

15. Nanoparticule comprenant une molécule d'acide nucléique selon l'une quelconque des revendications 1 à 7.
